# EUROPEAN PATENT APPLICATION

(11) **EP 4 840 882 A1**
(43) Date of publication of application: **18.11.2026**
(21) Application number: 25175949.4
(22) Date of filing: 13.05.2025
(51) Int. Cl.: C07K 14/725

(54) **TCRS RECOGNIZING MAGE-A4 AND USES THEREOF**

(71) Applicant: Genovie AB, 151 36 Södertälje (SE)
(72) Inventor: JARVIS, Reagan Micheal, 151 36 Södertälje (SE); PASE, Luke Benjamin, 151 36 Södertälje (SE); HILL, Ryan Edward, 151 36 Södertälje (SE); SALTER, Hugh Alexander, 151 36 Södertälje (SE)
(74) Representative: Aera A/S

(57) **Abstract**

The invention relates to a selection of verified targets for T-cell receptors (TCR) against mutated human melanoma-associated antigen A4 (MAGE-A4) peptide, as well as to several new T-cell receptors (TCRs), which are isolated and/or expressed in an engineered cell, and which specifically recognize a human MAGE-A4 peptide, wherein the peptide is a MAGE-A4 peptide, which consists of GVYDGREHTV (SEQ ID NO: 1) presented in a HLA-A*02 complex, and wherein the individual TCR comprises a paired TCR alpha- and TCR beta-chain.

The invention further relates to engineered cells and vectors expressing said TCRs and medical uses of the same for treating cancer patients.

## Description

### Technical field

The present invention relates to the field of treating cancer patients who express MAGE-A4. Herein disclosed is a selection of verified targets for T cell receptors (TCR) against melanoma-associated antigen A4 (MAGE-A4) peptides, as well as novel T cell receptors (TCR) which are isolated and/or expressed in an engineered cell, and which specifically recognize the MAGE A4 peptide, GVYDGREHTV (SEQ ID NO: 1) presented in HLA-A*02:01.

### Background

Adoptive cell transfer (ACT) therapies using engineered T cells (TCR-Ts) or chimeric antigen receptor (CAR)-T cells, have gained much traction in recent years. The use of CAR-T in the treatment of B cell malignancies has demonstrated to be very effective (Weber et al 2020), however this efficacy is not replicable in the treatment of solid tumours. TCR-T-based immunotherapy holds much promise for patients with difficult-to-treat cancers where there are currently no alternative treatment options. A great advantage of TCR-T-based therapy relates to T cells being able to recognise a much larger number of antigenic targets and from all cellular compartments than chimeric antigen receptors. The TCR-antigen-HLA interaction is the core component of the targeting mechanism that allows T cells to kill cancer cells. TCRs also have lower epitope density requirement for activation compared to chimeric antigen receptors. An important consideration when designing TCR-T-based therapy is the Human Leucocyte Antigen (HLA) restriction, as any given TCR can only be used to treat patients with the corresponding HLA genetic background to avoid tissue rejection. This restriction needs to be taken into consideration when developing a therapeutic TCR and when using such a TCR in engineered T cells.

There remains an unmet need in the art for obtaining specific and safe TCRs for difficult-to-treat cancers.

### The T cell Receptor (TCR)

The TCR is the component of the T cell responsible for interacting with and sensing the targets of T cell adaptive immunity. In general terms, the TCR is comprised of a heterodimeric protein complex presented on the cell surface. Each of the two TCR chains are composed of two extracellular domains, being the variable (V)-region and the constant (C)-region, both of the immunoglobulin superfamily (IgSF) domain, forming antiparallel β-sheets. These are anchored in the cell membrane by a type-I transmembrane domain, which adjoins a short cytoplasmic tail. The quality of the T cells to adapt and detect diverse molecular constituents arises from variations in the TCR chains that are generated during T cell genesis. This variation is generated by somatic recombination in a similar manner to antibody genesis in B cells.

### TCR chain diversity

The T cell pool consists of several functionally and phenotypically heterogeneous subpopulations. However, T cells may be broadly classified as αβ or γδ according to the somatically rearranged TCR isoform they express at their surface. There exist two TCR chain pair isoforms; TCR alpha (TRA) and TCR beta (TRB) pairs; and TCR gamma (TRG) and TCR delta (TRD) pairs. T cells expressing TRA:TRB pairs are referred to as αβ T cells, while T cells expressing TRG:TRD pairs are referred to as γδ T cells.

TCRs of both αβ and γδ forms are responsible for the recognition of diverse ligands, or 'antigens', and each T cell generates αβ or γδ receptor chains *de novo* during T cell maturation. These *de novo* TCR chain pairs achieve diversity of recognition through generation of receptor sequence diversity in a process called somatic V(D)J recombination after which each T cell expresses copies of a single distinctly rearranged TCR. At the TRA and TRG loci, a number of discrete variable (V) and functional (J) gene segments are available for recombination and juxtaposed to constant (C) gene segments, thus referred to as VJ recombination. Recombination at the TRB and TRD loci additionally includes a diversity (D) gene segment and is referred to as VDJ recombination.

Each recombined TCR possesses potential for unique ligand specificity, determined by the structure of the ligand-binding site formed by the α and β chains in the case of αβ T cells or γ and δ chains in the case of γδ T cells. The structural diversity of TCRs is largely confined to three short hairpin loops on each chain, called complementarity-determining regions (CDR), and termed CDR1, CDR2 and CDR3 respectively. Three CDRs are contributed from each chain of the receptor chain pair, and collectively these six CDR loops sit at the membrane-distal end of the TCR extracellular domain to form the antigen-HLA binding sites.

Sequence diversity in each TCR chain is achieved in two modes. First, the random selection of gene segments for recombination provides basal sequence diversity. For example, TRB recombination occurs between 47 unique V, 2 unique D and 13 unique J germline gene segments. In general, the V gene segment contributes both the CDR1 and CDR2 loops and are thus germline encoded. The second mode to generate sequence diversity occurs within the hypervariable CDR3 loops, which are generated by random deletion of template nucleotides and addition of non-template nucleotides, at the junctions between recombining V, (D) and J gene segments.

### Human Leukocyte Antigen (HLA) class I and II

HLAs are complexes on cell surface, encoded by HLA family of genes. They can also be refered to as Major Histocompatibility Complexes (MHC), for simplicity both the gene and MHC will collectively be referred to herein as HLAs.There are two forms of classical HLA complexes: HLA class I (HLAI) and HLA class II (HLAII). There are three classical HLAI genes: *HLA-A, HLA-B, HLA-C.* These genes encode a membrane-spanning α-chain, which associates with an invariant beta-2-microglobulin (b2M) chain. The HLAI α-chain is composed of three domains with an immunoglobulin fold: α1, α2 and α3. The α3 domain is membrane-proximal and largely invariant, while the α1 and α2 domains together form the polymorphic membrane-distal antigen-binding cleft. There are six classical HLAII genes: *HLA-DPA1, HLA-DPB1, HLA- DQA1, HLA-DQB1, HLA-DRA,* and *HLA-DRB1.* These genes encode paired DP, DQ and DR heterodimeric HLA complexes comprising a α-chain and a β-chain. Each chain has two major structural domains with an immunoglobulin fold, where the α2 and β2 domain comprise membrane-proximal and largely invariant modules similar to that of the HLAI α3 domain. The HLAII α2 and β2 domains together form the membrane-distal antigen-binding cleft and are regions of high polymorphism.

The antigen-binding cleft of HLAI and HLAII comprises two anti-parallel α-helices on a platform of eight anti-parallel β-sheets. In this cleft the peptide antigen is bound and presented in an extended conformation. The peptide-contacting residues in HLAI and HLAII are the location of most of the sequence polymorphism, which constitutes the molecular basis of the diverse peptide repertoires presented by different HLA alleles. The peptide makes extensive contacts with the antigen-binding cleft and as a result each HLA allele imposes distinct sequence constraints and preferences on the presented peptides. A given peptide will thus only bind a limited number of HLAs, and reciprocally each allele only accommodates a particular fraction of the peptide collection from a given protein. The set of HLAI and HLAII alleles that is present in each individual is called the HLA genotype, each of the two chromosomes containing a linear haplotype that will, in the absence of recombination, be transmitted intact to progeny cells. The polymorphism of HLAI and HLAII genes and the co-dominant expression of inherited alleles drives very large diversity of HLA haplotypes across the human population, which when coupled to the enormous sequence diversity of αβ TCR, presents high obstacles to standardisation of analysis of these HLA-antigen-TCR interactions.

### αβ TCR engagement of HLAI and HLAII

The αβ TCRs recognize peptides as part of a mixed peptide-HLA (pHLA) binding interface formed by residues of both the HLA and the peptide antigen (altered self). HLAI complexes are presented on the surface of nearly all nucleated cells and are generally considered to present peptides derived from endogenous proteins. T cells can thus interrogate the endogenous cellular proteome of an HLAI-presenting cell by sampling pHLAI complexes of an interacting cell. Engagement of HLAI requires the expression of the TCR co-receptor CD8 by the interacting T cell, thus HLAI sampling is restricted to CD8⁺ αβ T cells. In contrast, the surface presentation of HLAII complexes is largely restricted to professional APCs and are generally considered to present peptides derived from proteins exogenous to the presenting cell. An interacting T cell can therefore interrogate the proteome of the extracellular microenvironment in which the presenting cell resides. The engagement of HLAII requires the expression of the TCR co-receptor CD4 by the interacting T cell, and thus HLAII sampling is restricted to CD4⁺ αβ T cells.

### TCR:CD3 Complex

Mature αβ and γδ TCR chain pairs are presented on the cell surface in a complex with accessory CD3 subunits, denoted ε, γ, δ and ζ. These subunits associate with αβ or γδ TCRs as three dimers (εγ, εδ, ζζ). This TCR:CD3 complex (TCRsp) forms the unit for initiation of cellular signalling responses upon engagement of an αβ or γδ TCR with HLA-restricted antigen. The CD3 accessories associated as a TCR:CD3 complex contribute signalling motifs called immunoreceptor tyrosine-based activation motifs (ITAMs).

CD3ε, CD3γ and CD3δ each contribute a single ITAM while the CD3ζ homodimer contains 3 ITAMs. The three CD3 dimers (εγ, εδ, ζζ) that assemble with the TCR thus contribute 10 ITAMs. Upon TCR ligation with antigen, phosphorylation of the tandem tyrosine residues creates paired docking sites for proteins that contain Src homology 2 (SH2) domains, such as the critical ζ-chain-associated protein of 70 kDa (ZAP- 70). Recruitment of such proteins initiate the formation of TCR:CD3 signalling complexes that are ultimately responsible for T cell activation and differentiation.

### αβ T cells

αβ T cells are generally more abundant in humans than their γδ T cell counterparts. A majority of αβ T cells interact with peptide antigens that are presented by complexes on the cell surface. Peptide-HLA (pHLA)-recognising T cells were the first to be described and are by far the best characterised. More rare forms of αβ T cells have also been described. Mucosal-associated invariant T (MAIT) cells appear to have a relatively limited α and β chain diversity and recognise bacterial metabolites rather than protein fragments. The invariant natural killer T-cells (iNK T cells) and germline-encoded mycolyl-reactive T cells (GEM T cells) are restricted to recognition of glycolipids that are cross-presented by non-HLA molecules. iNK T cells are largely considered to interact with CD1d-presented glycolipids, whereas GEM T cells interact with CD1b-presented glycolipids. Additional forms of T cells are thought to interact with glycolipids in the context of CD1a and CD1c, however, such cells are yet to be characterised in significant detail.

### Conventional αβ T cells

The key feature of most αβ T cells is the recognition of peptide antigens in the context of HLA molecules. These are often referred to as 'conventional' αβ T cells. Within an individual, self-HLA molecules present peptides from self and foreign proteins to T cells, providing the essential basis for adaptive immunity against malignancies and foreign pathogens, adaptive tolerance towards commensal organisms, foodstuffs, and self. The HLA locus that encodes HLA proteins is the most gene-dense and polymorphic region of the human genome, and there are in excess of 12,000 alleles described in humans. The high degree of polymorphism in the HLA locus ensures a diversity of peptide antigen presentation between individuals, which is important for immunity at the population level.

### MAGE-A4 gene family

MAGE family of proteins are classified as cancer-testis antigens and are highly expressed in a variety of tumour types according to the Cancer Genome Atlas (TCGA) Research network (https://www.cancer.gov/tcga). MAGE-A4 is (over)expressed in solid cancers including synovial sarcoma (SS), myxoid/round cell liposarcoma (MRCLS), 19-35% non-small-cell lung cancer (NSCLC), head and neck squamous cell carcinoma (HNSCC) and 47% in epithelial ovarian cancers, urothelial, melanoma and 22% gastroesophageal cancers and 11% breast cancers (Caballero & Chen 2009), (Daudi et al 2014), (Sharma et al. 2006), (Ishihara et al 2020), (Iura et al. 2017), (Tajima et al 2003), (Gure et al Clin Cancer Res 2005), (Kim et al 2012), (Otte et al 2001), (Li et al 2005). Expression in normal tissue is restricted to immune privileged sites where it is thought to be involved in regulating key cellular mechanisms such as apoptosis and cell cycle control, although its exact function remains partially understood. MAGE-A4 is intracellularly processed and the resulting peptide fragments are presented on via Human Leucocyte Antigens (HLAs).

TCR-T-based immunotherapy targeted towards MAGE-A4 holds much promise for patients with difficult to treat cancers where there are currently no alternative treatment options such as but not limited to, synovial sarcoma (SS), myxoid/round cell liposarcoma (MRCLS), non-small-cell lung cancer (NSCLC), head and neck squamous cell carcinoma (HNSCC) and ovarian, urothelial, melanoma and gastroesophageal cancers. The involvement of MAGE-A4 in cancer progression and its potential role in immune system evasion underscore the therapeutic interest in targeting MAGE-A4 in oncology. Still, there is a need for validating optimal targets and isolating TCRs that recognize these suitable targets with high affinity and specificity without displaying cytotoxicity towards e.g., suboptimal targets.

### Summary of the invention

In general, the identification of TCRs that have a particularly suitable profile for therapeutic use is not straightforward and typically has a high attrition rate. The current invention thus for the first time discloses several verified suitable MAGE-A4 targets as well as TCRs that have been stringently de-risked and are therefore suitable therapeutic candidates for use in cancer therapy.

The current invention relates to several new T cell receptors (TCR), which are isolated and/or expressed in an engineered cell, and which specifically recognize a MAGE-A4 peptide, wherein the peptide is GVYDGREHTV (SEQ ID NO: 1) presented in HLA-A*02:01, and wherein the individual TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

In one embodiment, as exemplified in the experimental section, the TCR according to the current invention is a TCR, which, when expressed in an engineered cell, does not have detectable cross-reactivity towards any somatically expressed HLA-presented peptide other than a MAGE-A4 peptide, which consists of GVYDGREHTV (SEQ ID NO: 1) and which is presented in HLA-A*02:01 in a cross-reactivity assay.

In aspects, TCRs according to the invention have a higher sensitivity towards SEQ ID NO:1 when presented in a HLA-A*02:01 complex than does a known TCR. Such sensitivity is determined in a cell-cell contact assay wherein peptide-pulsed eAPCs are contacted with TCR-expressing eTPCs, wherein the eTPCs present the TCR to be assessed and wherein the eTPCs have been engineered to contain a Response Element used to assess the sensitivity in combination with CD3 downregulation. Such an assay is described in Example 3 in more detail.

In aspects, the sensitivity is at least 1.10 fold, such as at least 1.15 fold, such as at least 1.2 fold, such as at least 1.25 fold, such as at least 1.3 fold, such as at least 1.35 fold, such as at least 1.4 fold, such as at least 1.45 fold, such as at least 1.5 fold, such as at least 1.6 fold, such as at least 1.65 fold higher than that of a known TCR.

A TCR disclosed herein typically comprises:
a. a TCR alpha chain CDR3 sequence with at least 75%, such as at least 80% identity to a TCR alpha chain CDR3 sequence selected from the group consisting of SEQ ID NO: 46, SEQ ID NO: 48, and SEQ ID NO: 50, and
b. a TCR beta chain CDR3 sequence with at least 75%, such as at least 80% identity to a TCR beta chain CDR3 sequence selected from the group consisting of SEQ ID NO: 47, SEQ ID NO: 49, and SEQ ID NO: 51.

In embodiments, the TCR according to the current invention comprises:
a. a TCR alpha chain CDR3 sequence with a TCR alpha chain CDR3 sequence selected from the group consisting of SEQ ID NO: 46, SEQ ID NO: 48, and SEQ ID NO: 50, and
b. a TCR beta chain CDR3 sequence with a TCR beta chain CDR3 sequence selected from the group consisting of SEQ ID SEQ ID NO: 47, SEQ ID NO: 49, and SEQ ID NO: 51.

In a currently preferred embodiment of the invention the TCR alpha and beta chain variable domain scaffold regions or CDRs of the TCR comprises and/or consists of one of the sequences selected from the group consisting of:

| | SEQ ID NO | SEQ ID NO | SEQ ID NO | SEQ ID NO |
|---|---|---|---|---|
| SC1alpha | 11 | 13 | 11 | 13 |
| CDR1alpha | 32 | 34 | 32 | 34 |
| SC2alpha | 18 | 20 | 18 | 20 |
| CDR2alpha | 39 | 41 | 39 | 41 |
| SC3alpha | 25 | 27 | 25 | 27 |
| CDR3alpha | 46 | 48 | 50 | 48 |
| SC4alpha | 5 | 5 | 8 | 5 |
| SC5alpha | 54 | 54 | 54 | 54 |
| SC1beta | 12 | 14 | 15 | 14 |
| CDR1beta | 33 | 35 | 36 | 35 |
| SC2beta | 19 | 21 | 22 | 21 |
| CDR2beta | 40 | 42 | 43 | 42 |
| SC3beta | 26 | 28 | 29 | 28 |
| CDR3beta | 47 | 49 | 51 | 49 |
| SC4beta | 6 | 7 | 9 | 7 |
| SC5beta | 55 | 56 | 55 | 82 |

or sequences having at least 90% such as at least 95%, at least 97% or at least 99%, or 100% identity to said respective sequences.

In currently preferred embodiments, the TCR alpha chain variable, domain scaffold regions, and/or or CDRs comprise and/or consist of one of the sequences selected from the group consisting of SEQ ID NO: 57, SEQ ID NO: 59 and SEQ ID NO: 61, wherein the beta chain variable, domain scaffold regions, and/or or CDRs comprise and/or consist of one of the sequences selected from the group consisting of SEQ ID NO: 58, SEQ ID NO: 60, SEQ ID NO: 62 and SEQ ID NO: 83.

A TCR according to the current invention can be selected from the group comprising chimeric, humanized and human TCRs.

A TCR according to the invention can comprise constant regions of the alpha and beta chains which have been modified to include cysteine residues to allow covalent connection by di-sulphide bridges or comprise constant regions of the alpha and beta chains which allow non covalent connections between said paired alpha and beta chains. In embodiments, the alpha and/or beta chain comprise(s) a constant region of a TCR-chain of another mammal, such as, but not limited to, a mouse chimeric constant region(s). Further, the alpha and/or beta chain can have been modified to include cysteine residues to allow di-sulphide bridges.

In aspects, the TCR comprises a TCR alpha chain (TRA) and a TCR beta chain (TRB) which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein.

In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The current invention in one aspect relates to a soluble TCR and/or a conjugated soluble TCR according to the current invention. In aspects, the TCR is a bi-specific T cell engager (such as BiTE), or it is an immune mobilizing monoclonal T cell receptor.

The invention further relates to a pharmaceutical composition comprising a soluble protein consisting of all or a functional part of a TCR as disclosed and/or defined herein and at least one pharmaceutically acceptable excipient, carrier and/or stabilizer.

A pharmaceutical composition of the current invention comprises a soluble TCR, a conjugated soluble TCR, a cell expressing a TCR and/or a composition comprising a TCR, wherein the TCR is a TCR according to the current invention. Said soluble TCR can been conjugated to a radionuclide, a chemotherapeutic agent, a toxin or another therapeutically active component, and at least one pharmaceutically acceptable excipient, carrier or stabilizer.

In one aspect, the invention relates to a method of treating a disorder characterized by MAGE-A4 expression in a subject comprising administering to said subject a therapeutically effective amount of genetically engineered cells that express a TCR as disclosed and/or defined herein, or a pharmaceutical composition disclosed and/or defined herein, wherein said disorder is a solid malignancy expressing the peptide GVYDGREHTV (SEQ ID NO: 1), wherein said engineered cell is a T cell, T cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour and/or into the volume surrounding a solid tumour.

Said method can be used for treating a disorder in a subject wherein said subject carries an HLA allele selected from HLA-A*01, HLA-A*02, HLA-A*03, HLA-A*11, HLA-A*23, HLA-A*24, HLA-A*25, HLA-A*26, HLA-A*29, HLA-A*30, HLA-A*31, HLA-A*32, HLA-A*33, HLA-A*34, HLA-A*66, HLA-A*68, HLA-A*80, HLA-B*07, HLA-B*08, HLA-B*13, HLA-B*14, HLA-B*15, HLA-B*18, HLA-B*27, HLA-B*35, HLA-B*37, HLA-B*38, HLA-B*40, HLA-B*44, HLA-B*49, HLA-B*50, HLA-B*51, HLA-B*52, HLA-B*53, HLA-B*54, HLA-B*55, HLA-B*57, HLA-B*58, HLA-C*01, HLA-C*02, HLA-C*03, HLA-C*04, HLA-C*05, HLA-C*06, HLA-C*07, HLA-C*08, HLA-C*12, HLA-C*14, HLA-C*15, HLA-C*16, HLA-E*01, optionally wherein the HLA allele is selected from the group consisting of HLA-A*01-01, HLA-A*01-02, HLA-A*01-03, HLA-A*02-01, HLA-A*02:02, HLA-A*02:03, HLA-A*02:04, HLA-A*02:05, HLA-A*02:06, HLA-A*02:07, HLA-A*03:01, HLA-A*11:01, HLA-A*11:02, HLA-A*11:03, HLA-A*11:04, HLA-A*11:05, HLA-A*11:09, HLA-A*23:01, HLA-A*24:02, HLA-A*24:03, HLA-A*24:04, HLA-A*24:07, HLA-A*24:10, HLA-A*24:17, HLA-A*25:01, HLA-A*26:01, HLA-A*29:02, HLA-A*30:01, HLA-A*30:02, HLA-A*31:01, HLA-A*32:01, HLA-A*33:01, HLA-A*33:03, HLA-A*34:02, HLA-A*66:01, HLA-A*66:02, HLA-A*68:01, HLA-A*68:02, HLA-A*80:01, HLA-B*07:02, HLA-B*07:05, HLA-B*08:01, HLA-B*13:01, HLA-B*13:02, HLA-B*14:01, HLA-B*14:02, HLA-B*15:01, HLA-B*15:02, HLA-B*15:03, HLA-B*18:01, HLA-B*27:05, HLA-B*35:01, HLA-B*35:02, HLA-B*35:03, HLA-B*35:05, HLA-B*35:08, HLA-B*35:12, HLA-B*37:01, HLA-B*38:01, HLA-B*40:01, HLA-B*40:02, HLA-B*44:02, HLA-B*44:03, HLA-B*49:01, HLA-B*50:01, HLA-B*51:01, HLA-B*51:02, HLA-B*52:01, HLA-B*53:01, HLA-B*54:01, HLA-B*55:01, HLA-B*57:01, HLA-B*58:01, HLA-C*01:02, HLA-C*02:02, HLA-C*03:03, HLA-C*03:04, HLA-C*04:01, HLA-C*05:01, HLA-C*06:02, HLA-C*07:01, HLA-C*07:02, HLA-C*08:01, HLA-C*08:02, HLA-C*12:02, HLA-C*12:03, HLA-C*14:02, HLA-C*15:02, HLA-C*16:01, HLA-E*01:01, HLA-E*01:03. In in an embodiment the HLA allele is HLA-A*02, such as HLA-A*02:01.

In addition, said method can be used for treating a said subject, wherein said subject carries an alternate subform of HLA-A*02 other than HLA-A*02:03 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention, such as wherein said subject carries HLA-B*57:01.

In addition, said method can be used for treating a said subject, wherein said subject carries an alternate subform of HLA-A*02 other than HLA-A*02:03, HLA-A*02:04, HLA-A*02:05 and HLA-A*02:06 which alternate subform of HLA-A*02 is capable of specifically presenting the peptide of SEQ ID NO: 1 and which is specifically recognized by the TCR according to the current invention, such as wherein said subject carries a different HLA-A, HLA-B or HLA-C allele than HLA-A*02, that is capable of specifically presenting the peptide of SEQ ID NO: 1 and which is recognized by the TCR according to the current invention.

In addition, said method can be used for treating a said subject, wherein said subject carries an alternate subform of HLA-A*02 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention such as wherein said subject carries HLA-A*68:02 and/or HLA-B*15:01.

In one aspect, the current invention relates to a TCR disclosed, described and/or defined herein for use as a medicament. In particular, the current invention relates to a TCR disclosed, described and/or defined herein for use in the treatment of a cancer in a patient carrying HLA-A*02, comprising administering to said patient said TCR or a pharmaceutical composition comprising said TCR, wherein said cancer is a solid malignancy expressing the peptide of SEQ ID NO: 1, and wherein said TCR or a pharmaceutical composition comprising said TCR is infused systemically into said patient or injected or pumped directly into a solid tumour, into a blood vessel that feeds a tumour and/or into the volume surrounding a solid tumour in said patient.

In aspects, said treatment comprises administering to said patient an engineered cell, wherein said engineered cell is a T cell, T cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour and/or into the volume surrounding a solid tumour.

Typically, the use related to herein is for treating a disorder in a subject, wherein said subject carries a subform of HLA-A*02 capable of specifically presenting the peptide of SEQ ID NO: 1 and thus is recognized by a TCR according to the current invention.

In an aspect a TCR according to the invention is capable of specifically recognising both of SEQ ID NO: 1 and SEQ ID NO: 85 when either is presented in a HLA-A*02 complex, thereby allowing for treatment of patients wherein either or both peptides are presented in the HLA-A*02 context.

Typically, the use related to herein is for treating a disorder in a subject, wherein said subject carries an alternate subform of HLA-A*02, which alternate subform of HLA-A*02 is capable of specifically presenting the peptide of SEQ ID NO: 1 and thus is recognized by a TCR disclosed, described and/or defined herein, or wherein said subject carries a different HLA-A, HLA-B or HLA-C allele than HLA-A*02, that is capable of specifically presenting the peptide of SEQ ID NO: 1 and which is recognized by the TCR according to the current invention.

In one aspect, the invention relates to a nucleic acid encoding a TCR comprising a paired TCR alpha- and TCR beta-chain as disclosed, described and/or defined herein.

In aspects of the invention, the vector disclosed, described and/or defined herein is a non-viral vector (nucleic acid vector or a nanoparticle vector) or a viral vector wherein the vector comprises one or more nucleic acid sequences encoding a TCR comprising a paired TCR alpha- and TCR beta-chains as but are not limited to, linear or circular double stranded DNA, or linear or circular single stranded DNA, that may or may not be chemically modified DNA, linear or circular double stranded RNA, or linear or circular single stranded RNA, that may or may not be chemically modified RNA. The RNA vector may contain a 5' CAP, 5' UTR, 3' UTR and 3' poly(A) tail and Internal Ribosome Entry Site (IRES), and may contain substituted nucleotides such as pseudouridine, and may contain replication-directing elements for self-amplification and/or may be circularized. In certain aspects, a nucleic acid payload according to the current invention is encapsulated within a nanoparticle vector such as lipid nanoparticle (LNP) comprising ionizable cationic lipid, a sterol, a phospholipid, a non-functionalized PEG-lipid, and a functionalized PEG-lipid wherein the functionalized PEG-lipid may have been conjugated with a binding moiety, and wherein the LNP encapsulates a payload of mRNA. The mRNA payload may contain a linear expression construct with 5' cap, 5' UTR, 3' UTR, poly-A features and IRES, and may contain substituted nucleotides such as pseudouridine, and may contain replication-directing elements for self-amplification. The mRNA payload may also be circular. The LNP can be targeted (tLNP) to deliver payload to cell types based on expression of cell surface markers (Tombácz et al 2021; Breda et al 2023) for example LNP may be conjugated to anti-CD5, or anti-CD3, or anti-CD4, or anti - CD8 targetting molecules for targeting CD8⁺ and CD4⁺ T cells (Wada (2018); Dalloul, (2009), Lee (2021)) or CD16 or NK1.1 for targeting NK cells, EpCAM as a targeting epithelial cells targeting molecule or to another marker or a functional ligand thereof that targets cellular delivery to specific target cells. Such tLNP particles may be used both ex vivo and in vivo to direct the mRNA payload to a desired immune cell type. In aspects, the viral vector comprises a packaged nucleic acid payload within one or more surface envelope receptors that bind to a ligand on a target cell, heterologous viral envelope glycoproteins, fusion glycoproteins, T cell activation or co-stimulation molecules, cytokines or cytokine-based transduction enhancers, and/or transmembrane proteins comprising a mitogenic domain and/or cytokine-based domain exposed on the surface and/or conjugated to the surface of the viral vector. The tropism of the vector can further be retargeted by pseudotyping with a Cocal or a Nipah virus envelope protein, and the Nipah envelope protein can be engineered to bind EpCAM, CD3, CD5, CD4, or CD8 or other marker suitable for targeted cellular delivery of an encapsulated nucleic acid vector. Such viral vectors may be used both ex vivo and in vivo to direct the mRNA payload to a desired immune cell type. Several viral vector systems, modified viruses designed to deliver genetic material into cells, are suitable for such manipulations and include viral vector systems derived from retroviruses such as lentivirus (LV) and gammaretrovirus, systems which are typically used for ex vivo manipulation and manufacture. Viral systems incorporating vectors may also be used for in vivo delivery of target payloads, typically derived from adeno-associated virus (AAV).

In another aspect, the invention relates to a vector comprising a nucleic acid encoding a TCR comprising a paired TCR a - and TCR b chain as disclosed, described and/or defined herein. Said vector can be selected from the group consisting of an expression vector, a cloning vector, viral vector or a non-viral vector. In certain aspects, said vector further comprises a nucleic acid sequence encoding CD8α and/or CD8β that can be operably linked to a nucleic acid encoding a tag, such as, but not limited to, wherein the nucleic acid encoding a tag is at the 5' upstream of the nucleic acid sequence encoding TCR α and/ or TCR β and/or wherein the tag is a truncated CD34 molecule representing a cell surface enrichment tag. The isolated nucleic acid according to the current invention and the nucleic acid sequence encoding CD8α and/or CD8β can optionally be interconnected with an internal ribosome entry site or a nucleic acid sequence encoding a self-cleaving peptide, such as selected from the group consisting of P2A, E2A, F2A and T2A.

In aspects, such vectors may be used for ex vivo manufacture of cell therapy products for infusion and/or targeted delivery of vectors in vivo for TCR expression in targeted cell populations. For ex vivo manufacture and cellular manipulation, vectors may be delivered to cells maintained in culture as encapsulated LNPs, as tLNPs, by combination with agents that promote uptake of nucleic acids such as lipofectamine, by electroporation or by inclusion in a viral-genome derived backbone so as to form a viral-like particle (VLP) capable of mediating both integrative or non-integrative manipulation of target cells.

A further aspect of the invention relates to an engineered cell expressing the TCR as disclosed, described and/or defined herein and/or the isolated nucleic acid disclosed, described and/or defined herein and/or which comprises a vector disclosed, described and/or defined herein.

Optionally, the engineered cell according to the current invention comprises a chromosomal gene knock out of one or more TCR gene(s), one or more HLA gene(s), or both, such as wherein the engineered cell comprises a knockout of an HLA gene selected from the group consisting of an a-1-macroglobulin gene, a-2-macroglobulin gene, a-3-macroglobulin gene, b-1-microglobulin gene, b-2-microglobulin gene, and combinations thereof, or such as, wherein the engineered cell comprises a knockout of a TCR gene selected from a TCR alpha variable region gene, TCR beta variable region gene, TCR constant region gene, and combination thereof. An engineered cell according to the current invention can express CD8a and/or CD8b, and, optionally, the CD8a and/or CD8 b is fused to a truncated CD34 enrichment tag. The engineered cells can be enriched using the truncated CD34 enrichment tag.

Typically, the engineered cell disclosed, described and/or defined herein, is a hematopoietic progenitor cell, peripheral blood mononuclear cell (PBMC), cord blood cell, or immune cell. In an embodiment, the immune cell is a T cell, cytotoxic lymphocyte, cytotoxic lymphocyte precursor cell, cytotoxic lymphocyte progenitor cell, cytotoxic lymphocyte stem cell, CD4⁺ T cell, CD8⁺ T cell, CD4/CD8 double negative T cell, gamma delta (gd) T cell, natural killer (NK) cell, NK-T cell, dendritic cell, or a combination thereof. In one embodiment, the cell is derived from a hematopoietic stem cell (HSC) or an induced pluripotent stem cell (iPSC). The T cell can be a naive T cell, central memory T cell, effector memory T cell, or a combination thereof, or a primary T cell or a cell of a T cell line.

In a currently preferred embodiment, the engineered cell disclosed, described and/or defined herein does not express or has a lower surface expression of an endogenous TCR and is capable of producing a cytokine or a cytotoxic molecule when contacted with a target cell that presents a human MAGE-A4 peptide, wherein the peptide consists of GVYDGREHTV (SEQ ID NO: 1) presented in a HLA-A*02 context.

In embodiments, the engineered cell according to the current invention is capable of producing TNF-alpha, IL-2, and/or IFN-gamma, and/or a perforin and/or a granzyme, such as granzyme B. An engineered cell according to the current invention can be contacted with a target cell *in vitro* or ex *vivo* and is preferably capable of producing a higher level of cytokine or a cytotoxic molecule when contacted with a target cell with a heterozygous expression of MAGE-A4, such as an at least 1.05-fold higher level of cytokine or a cytotoxic molecule.

In embodiments, the engineered cell according to the current invention is capable of killing a target cell that presents a MAGE-A4 peptide, wherein the peptide is human MAGE-A4 peptide, wherein the peptide consists of GVYDGREHTV (SEQ ID NO: 1) presented in a HLA-A*02 context. The killing is determined by a killing assay and the ratio of the engineered cell and the target cell in the killing assay is from 1:16 to 1:1, wherein the target cell is a target cell pulsed with a range of between 0.1 pM and 1 mM of a MAGE-A4 peptide, wherein the peptide is a human MAGE-A4 peptide, wherein the peptide consists of GVYDGREHTV (SEQ ID NO: 1) presented in a HLA-A*02 context.

In an embodiment, the engineered cell disclosed, described and/or defined herein is capable of killing a higher number of target cells when the target cells have heterozygous expression of a MAGE-A4 peptide, wherein the peptide is a human MAGE-A4 peptide, wherein the peptide consists of GVYDGREHTV (SEQ ID NO: 1) presented in a HLA-A*02 complex. Typically, the cell killing is at least 1.05-fold higher in the target cells that present GVYDGREHTV (SEQ ID NO: 1) in HLA-A*02.

In aspects, the target cell is a cell line or a primary cell, such as selected from the group consisting of a cancer cell line, a primary cancer cell line, a transformed cell line, and an immortalized cell line, such as a HEK293-, ARH-77-, NCI-H1755-, NCI-H1703-, A375- or U266, or SAOS-2- derived cell lines. Such cell lines may be modified to stably express HLA-A*02 and/or luciferase reporters.

The current invention also relates to a population of engineered cells disclosed, described and/or defined herein as well as to a pharmaceutical composition comprising any one or more of the engineered cells or vectors of the current invention, such as wherein the vectors are selected from the group consisting of viral vectors, non-viral vectors, LNPs or any other carrier comprising the nucleic acid sequence described, disclosed and/or defined herein.

### Abbreviations and definitions

It is to be understood that the terminology used herein is for the purpose of describing particular embodiments only and is not intended to limit the scope of the present invention which will be limited only by the appended claims.

It must be noted that as used herein and in the appended claims, the singular forms "a", "an", and "the" include plural references unless the context clearly dictates otherwise.

### Abbreviations

- aAM: Analyte antigenic molecule
- aAPX: Analyte antigen-presenting complex
- APC: Antigen-presenting cell
- APX: Antigen-presenting complex
- B-cell: B lymphocytes
- β2M: Beta 2 Microglobulin
- BFP: Blue fluorescent protein
- CAR: Chimeric antigen receptor
- CAR-T: CAR T cell
- CD1b: Cluster of differentiation 1b
- CD1d: Cluster of differentiation 1d
- CD3: Cluster of differentiation 3
- CDR: Complementarity-determining regions
- CM: Cargo molecules
- CMV: Cytomegalovirus
- C-region: Constant region
- D-region: Diversity region
- DAMPS: Danger associated molecular patterns
- DC: Dendritic cells
- DNA: Deoxyribonucleic acid
- dsDNA: Double stranded DNA
- eAPC: Engineered antigen-presenting cell Engineered antigen-presenting cell expressing an analyte antigenic
- eAPC-a: molecule Engineered antigen-presenting cell that present an analyte antigen-
- eAPC-p: presenting complex Engineered antigen-presenting cell that presents an analyte antigen-presenting
- eAPC-pa: complex and analyte antigenic molecule
- eAPCS: Engineered antigen-presenting cell system

- eTPC: Engineered TCR-presenting cell
- eTPC-t: Engineered TCR-presenting cell that present full-length TCR pairs
- eTPCS: Engineered TCR-presenting cell system
- FAB: Antibody fragment antigen binding
- FACS: Fluorescence-activated cell sorting
- FL: Full length
- FRT: Flippase recognition target
- GEM T-cells: Germ line-encoded mycolyl lipid-reactive T-cells
- GFP: Green fluorescent protein
- HCMV: Human Cytomegalovirus
- HDR: Homology directed recombination
- HIV: Human immunodeficiency virus
- HLA: Human leukocyte antigen
- HLAI: HLA class I
- HLAII: HLA class II
- IgSF: Immunoglobulin superfamily
- iNK T-cells: Invariant natural killer T-cells
- IRES: Internal ribosome entry site
- ITAM: Immunoreceptor tyrosine-based activation motif
- J-donor: Joining donor
- J-region: Joining region
- LNP: Lipid Nanoparticle
- MACS: Magnetic-activated cell sorting
- MAGE: Melanoma associated antigen
- MAIT: Mucosal-associated invariant T
- MG: Minigene
- MHC: Major Histocompatability Complex
- MR1: Major histocompatibility complex class I-related gene protein
- mRNA: Messenger ribonucleic acid
- NCBP: Non-cell based particles
- NK T-cells: Natural killer T cells
- odeCDR3: Oligonucleotide duplex encoding CDR3
- ORF: Open reading frame
- PAMPS: Pathogen-associated molecular patterns
- PCR: Polymerase chain reaction
- PDAC: Pancreatic ductal adenocarcinoma
- pHLA: Peptide HLA
- pMHC: Peptide MHC
- PMSA: Prostate-specific membrane antigen
- RFP: Red fluorescent protein
- RMCE: Recombinase mediated cassette exchange
- RNA: Ribonucleic acid
- RT: Reverse Transcription
- SH2: Src homology 2
- T cells: T lymphocytes
- TAA: Tumour-associated-antigens
- TALEN: Transcription activator-like effector nucleases
- TCR: T-cell Receptor
- TCRsp: TCR surface proteins in complex with CD3
- tLNP: Targeted Lipid Nanoparticle
- TORES: TCR ORF Reconstitution and Engineering System
- TRA: TCR alpha
- TRB: TCR beta
- TRD: TCR delta
- TRG: TCRgamma
- UFD: Ubiquitin fusion domain
- V-C entry vector: Variable-Constant entry vector
- V (-region): Variable region
- ZAP-70: ζ-chain-associated protein of 70 kDa

### Definitions

**aAM:** Analyte antigenic molecule. Generally, a protein but could also be a metabolite that is expressed by a cell from their genomic DNA and/or a specific introduced genetic sequence. The AM is expressed in the cell and a fragment can then be presented on the cell surface by an APX as cargo or on its own. Either as cargo or not, the AM can then be the target of T-cell receptor bearing cells or related affinity reagents.

**Adaptive immunity:** A subsystem of the overall immune system that is composed of highly specialized, systemic cells and processes that eliminate pathogens or prevent their growth.

**Adjuvants:** A substance, which when administered prior, together or after administration of an antigen accelerates, prolongs and/or enhances the quality and/or strength of an immune response to the antigen in comparison to the administration of the antigen alone. Adjuvants can increase the magnitude and duration of the immune response induced by vaccination.

**A pair of complementary TCR chains:** Two TCR chains wherein the translated proteins are capable of forming a TCRsp on the surface of a TCR presenting cell when expressed with the CD3 components.

**Affinity:** Kinetic or equilibrium parameter of an interaction between two or more molecules or proteins

**Affinity Reagent:** Any reagent that is prepared as analyte to probe TCRsp binding and/or stimulation at the cell surface of the eTPC-t in an eTPC:A system

**Allele:** Variant form of a given gene

**Alloreactivity:** Significant activity of a TCR to an HLA other than that for which it was selected; "off-HLA" reactivity

**Amplicon:** a piece of DNA or RNA that is the source and/or product of artificial amplification using various methods including PCR

**Analyte:** an entity that is of interest to be identified and/or measured and/or queried in the combined system

**Antibody:** Affinity molecule that is expressed by specialized cells of the immune system called B-cells and that contains of two chains

**Antigen:** any molecule that may be engaged by a TCR and results in a signal being transduced within the T cell

**Analyte antigen:** collectively the eTPC:Antigen system (eTPC:A) representing any entity presenting an antigen for analytical determination

**Antibody Drug Conjugates:** (ADCs) consist of antibodies, toxic drugs, and linkers. The antibody moiety targets the antigen on the tumour, once bound the ADCs are internalised into the tumour and release the drugs in lysosomes to kill the tumour cells

**Antigen-binding cleft:** long cleft or groove that is the site at which peptide antigens bind to the MHC-I molecule

**APC:** Antigen-presenting cell. A cell capable of presenting antigen on its cell surface, generally in the context of an HLA

**aAPX:** Analyte antigen-presenting complex. A protein that is expressed and presented on the cell surface by nucleated cells from genes/ORF encoding genomic DNA and/or a specific introduced genetic sequence. The APX presents a cargo, being either a peptide or other metabolite molecules.

**Autoimmunity:** Is the system of immune responses of an organism against its own healthy cells and tissues.

**Avidity:** the sum total of the strength of binding of two molecules to one another at multiple sites, eg taking into account the valency of each interaction

**Bi-Specific T cell engager:** artificial bispecific monoclonal antibodies that direct T cell's cytotoxic activity against cancer or a target cell

**C (-region):** Constant gene segment. One of the gene segments that is used to assemble the T-cell receptor. The c-region is a distinct segment that rather than driving diversity of the TCR, defines its general function in the immune system.

**Cargo-loading machinery:** Cellular set of proteins that generate and load cargo molecules on APX from proteins or other presented molecules found in the cell.

**C cloning fragment:** Constant Cloning fragment. Also referred to as a C gene segment cloning fragment. A construct carrying a portion of a C gene segment used to construct a V-C entry vector.

**CD3:** a multi-protein complex of 6 chains, that associate with T cell receptor as three dimers (εγ, εδ, ζζ). This TCR:CD3 complex forms the unit for initiation of cellular signalling responses upon engagement of a αβ or γδ TCR with antigen. The CD3 accessories associated as a TCR:CD3 complex contribute signalling motifs called immunoreceptor tyrosine-based activation motifs (ITAMs).

**CDR:** complementarity-determining regions. Short sequences on the antigen-facing end of TCRs and antibodies that perform most of the target binding function. Each antibody and TCR contains six CDRs and they are generally the most variable part of the molecules allowing detection of a large number of diverse target molecules.

**CAR:** Chimeric Antigen Receptor. A fusion protein engineered to contain 2 or more amino acid sequences linked together in a way that does not occur naturally or does not naturally occur in a host cell, which fusion protein can function as a receptor when present on a surface of a cell.

**Cis-acting element:** regions of non-coding DNA that regulate the transcription of nearby ORFs.

**C-part:** Constant part. A small portion of Constant gene segment sequence carried by a J receiving cassette fragment, J receiving cassette and J donor vector to standardise overhang sequences for operation of the TORES to reconstitute TCR ORFs.

**CM:** Cargo molecules. Peptide or metabolite that is presented by an antigen-presenting complex for example a HLA I or HLA II. The CM can be expressed by the cell intrinsically from the genomic DNA, introduced into the culture medium or expressed from a specifically introduced genetic sequence.

**Cognate Antigen:** An antigen, often presented by an HLA, that is recognised in a particular TCR. TCR and antigen are cognate objects.

**Copy-number:** The whole number occurrence of a defined sequence encoded within the genome of a cell.

**Cross-reactivity:** cytotoxic activity of a T cell for a somatically expressed HLA-presented peptide other than the desired target HLA-presented peptide

**Cytogenetic:** The study of inheritance in relation to the structure and function of chromosomes, i.e. determine the karyotype of a cell

**Cytotoxic/Cytotoxicity:** Process in which a T cells releases factors that directly and specifically damage a target cell.

**D (-region):** Diversity gene segment. One of the gene segments that is used to assemble the T-cell receptor. Each individual has a large number of different variations of these regions making it possible for each individual to arm T cells with a very large variety of different TCR.

**Dimer:** is an oligomer consisting of two structurally similar monomers joined by bonds that can be either strong or weak, covalent or intermolecular.

**DNA:** Desoxyribonucleic acid. Chemical name of the molecule that forms genetic material encoding genes and proteins.

**eAPC/eAPC-p/eAPC-pa:** Engineered antigen-presenting cell, as described in WO 2017/083316. -p indicates that that the cell presents an antigen-presenting complex, and -pa indicates that the cell presents an antigen presented in an antigen-presented complex.

**EC50:** Half maximal effective concentration, concentration effective in producing 50% of the maximal response

**Endogenous:** Substance that originated from within a cell

**Eukaryotic conditional regulatory element:** A DNA sequence that can influence the activity of a promoter, which may be induced or repressed under defined conditions

**Eukaryotic Promoter:** A DNA sequence that encodes a RNA polymerase biniding site and response elements. The sequence of the promoter region controls the binding of the RNA polymerase and transcription factors, therefore promoters play a large role in determining where and when your gene of interest will be expressed.

**Eukaryotic terminator/Signal terminator:** A DNA sequence that are recognized by protein factors that are associated with the RNA polymerase II and which trigger the termination process of transcription. It also encodes the poly-A signal

**Engineered Cell:** A cell wherein the genome has been engineered through genetic modification modified.

**Epitope:** An epitope, also known as antigenic determinant, is the part of an antigen that is recognized by the immune system, specifically by antibodies, B cells, or T cells. For example, the epitope is the specific piece of the antigen to which an antibody binds.

**Epigenetic insulator sequence:** DNA element that disrupts the communication between a regulatory sequence, such as an enhancer or a silencer, and a promoter.

**eTPC system:** eTPCS, the system by which eTPC-t cells, or libraries thereof, are prepared for combination in the eAPC:eTPC system

**FACS/Flow Cytometry:** Fluorescence-activated cell sorting. Flow cytometry is a technique by which individual cells can be analyzed en masse for the expression of specific cell surface and/or intracellular markers. A variation of that technique, cell sorting, allows cells that carry a defined set of markers to be retrieved for further analysis.

**Family of APX:** A set of several similar genes that encode functionally related proteins, which constitute an antigen presentation complex

**Flp Recombinase:** A recombinase (Flippase, Flp) derived from the 2 µm plasmid of baker's yeast *Saccharomyces cerevisiae.*

**Fluorescent (protein) marker:** Molecule that has specific extinction and emission characteristics and which can be detected by Microscopy, FACS and related techniques.

**Germline gene segments:** (TCR) Gene segments that are naturally occurring in humans.

**Gene cis-acting elements:** are present on the same molecule of DNA as the gene they regulate whereas trans-regulatory elements can regulate genes distant from the gene from which they were transcribed. Cis-regulatory elements are often binding sites for one or more trans-acting factors.

**Genetic barcoding:** DNA barcoding is a taxonomic method that uses a short genetic marker in an organism's DNA to identify it as belonging to a particular species.

**Genomic donor vector:** matched to genomic receiver site, designed to deliver donor genetic material

**Genomic Receiver Site:** A site within the genome for targeted integration of donor genetic material encoded within a Genetic Donor Vector.

**Genomic Receiver Site Recycling:** The reversion of an occupied genomic receiver site back to the conformation wherein a new analyte (TCR) ORF can be integrated

**Haplotype:** a physical arrangement of genomic variants that tend to be inherited together.

**HLA haplotype:** a linear arrangement of HLA alleles along an individual chromosome

**Heterospecific recombinase sites:** A DNA sequence that is recognized by a recombinase enzyme to promote the crossover of two DNA molecules.

**HLA I:** Human Leukocyte Antigen class I. A gene that is expressed in humans in all nucleated cells and exported to the cell surface where it presents as cargo short fragments, peptides, of internal proteins to T-cell receptors. As such it presents fragments of potential ongoing infections along with intrinsic proteins. The HLA I can additionally present as cargo peptides that are added to the culture medium, generated from proteins expressed form introduced genetic elements or generated from proteins that are taken up by the cell. HLA class I genes are polymorphic meaning that different individuals are likely to have variation in the same gene leading to a variation in presentation. Related to HLA class II.

**HLA II:** Human Leukocyte Antigen Class II. A gene that is expressed in humans in specific cells that are coordinating and helping the adaptive immune response for example dendritic cells. Related to HLA class I. HLA class II proteins are exported to the cell surface where they present as cargo short fragments, peptides, of external proteins to T-cell receptors. As such it presents fragments of potential ongoing infections along with intrinsic proteins. The HLA II can additionally present as cargo peptides that are added to the culture medium, generated from proteins expressed form introduced genetic elements or generated from proteins that are taken up by the cell. HLA class II genes are polymorphic meaning that different individuals are likely to have variation in the same gene leading to a variation in presentation.

**HLA-A*02 allele group:** main examples of HLA-A*02 sub alleles are HLA-A*02:01, HLA-A*02:02, HLA-A*02:03, HLA-A*02:04, HLA-A*02:05, HLA-A*02:06, and HLA-A*02:07. Thus, the term "HLA-A*02" as used herein is intended to include these sub alleles unless otherwise stated.

**Homologous arms:** A stretch of DNA that has near identical sequence identity to a complement homologous arm and therefore promote the exchange of two DNA molecules by the cellular process, homology directed repair.

**ImmTACs: I**mmune **m**obilising **m**onoclonal **T**-cell receptors **A**gainst **C**ancer. These bispecifics are designed to activate T cells against cancer and virus-infected cells. An ImmTAC is specific to both MHC-peptides (pHLA) located on the surface of the tumor and CD3s located on T cells. The ImmTAC is thus able to directly activate CD3 and its corresponding pathways in activated T cells.

**Immune surveillance:** Process in which the immune system detects and becomes activated by infections, malignancies or other potentially pathogenic alterations.

**Immunotherapy:** a type of treatment that boosts the body's natural defenses to fight a disease. It uses substances made by the body or in a laboratory to improve or restore immune system function.

**Insulator:** A DNA sequence that prevents a gene from being influenced by the activation or repression of nearby genes. Insulators also prevent the spread of heterochromatin from a silenced gene to an actively transcribed gene.

**Integration:** The physical ligation of a DNA sequence into a chromosome of a cell

**Integration vector:** The product of TORES containing TCR ORFs, and matched to genomic receiver sites, containing genetic elements at the 5' and 3' ends to enable integration.

**Integration couple:** matched integration vector and genomic receiver site

**Internal ribosome entry site (IRES):** A DNA sequence that once transcribed encodes a RNA element that allows the initiation of translation in a cap-independent manner

**Isoform:** any of two or more functionally similar proteins that have a similar but not identical amino acid sequence and are either encoded by different genes or by RNA transcripts from the same gene which have had different exons removed.

**J (-region):** Joining segment. One of the gene segments that is used to assemble the T-cell receptor. Each individual has a large number of different variations of these regions making it possible for each individual to arm T cells with a very large variety of different TCR.

**J donor backbone:** Joining donor backbone. The vector backbone into which a J receiving cassette fragment is inserted to create a J receiving cassette vector.

**J donor vector:** The vector of the two-component vector system that carries the J TCR segment, and donates this segment to the V-C entry vector during reconstitution of a full-length TCR ORF.

**J receiving cassette fragment:** Joining receiving cassette fragment. A cloning fragment that carries a C-part used to construct a J receiving cassette vector.

**J receiving cassette vector:** Joining receiving cassette vector. The vector, carrying a C-part, into which a J segment part is inserted to create a J donor vector.

**J segment part:** Joining segment part. A DNA construct carring a portion of a J gene segment that is inserted into a J receiving cassette vector to generate a J donor vector.

**Karyotype:** a complete set of chromosomes

**Kozak Sequence:** Short sequence required for the efficient initiation of translation

**Lipid nanoparticle:** Small spherical particles composed of lipid. Used to deliver a payload into a target cell

**Major HLA class I:** a Family of APX that comprise of the genes HLA-A, HLA-B and HLA-C

**Manufacturing:** ex vivo manufacturing, where T cells are isolated, modified, expanded outside the body, and infused into the patient, and; in vivo manufacturing, where T cells are modified directly within the patient without ex vivo manipulation.

**Matched:** When two components encode genetic elements that direct and restrict the interaction between the complemented components

**Meganuclease recognition site:** A DNA sequence that is recognized by a endodeoxyribonuclease, commonly referred to as a meganuclease

**Metabolite:** A molecule created or altered through metabolic pathways of the cell

**MHC:** Major Histocomaptability Complex. Large locus on vertebrate DNA containing a set of closely linked polymorphic genes that code for cell surface proteins essential for the adaptive immune system.

**Mobile genetic element:** A DNA sequence that can permit the integration of DNA with the activity of transposases enzymes

**Monoclone cell line:** A defined group of cells produced from a single ancestral cell by repeated cellular replication

**mRNA splice acceptor site:** At the 5' end the DNA nucleotides are GT [GU in the pre-messenger RNA (pre-mRNA)]; at the 3' end they are AG. These nucleotides are part of the splicing sites. DONOR-SPLICE: splicing site at the beginning of an intron, intron 5' left end. ACCEPTOR-SPLICE: splicing site at the end of an intron, intron 3' right end.

**Multimer:** A protein complex consisting of multiple identical monomers. Often used in context of HLA multimer reagent.

**Native:** an entity that is naturally occurring in the cell

**Negative Selection Marker:** A selectable marker that confers negative selection of a vector and/or of host organism carrying said marker-bearing vector

**Non-cell-based Particle:** (NCBP) acts in a similar manner to an affinity reagent, inasmuch that the particle presents an analyte antigen or other entity that is to be assessed for TCRsp engagement at the surface of a eTPC-t within and eTPC:A system. However, an NCBP is considered as a larger entity that can further carry genetic or other information that is to act as an identifier, either directly or by proxy, of the presented analyte antigen or other binding entity. A typical example of an NCBP would be a bacteriophage in a phage-display scenario

**Non-coding gene:** A non protein coding DNA sequence that is transcribed into functional non-coding RNA molecules

**Nucleases:** enzymes that degrade nucleic acids

**odeCDR3:** oligonuclotide duplex encoding complementarity-determining regions. A synthetic construct carrying CDR3 genetic sequence with terminal overhangs, used in conjunction with the two-component vector system to reconstitute a full-length TCR ORF.

**Oncogenic driver mutations:** mutations found in genes that encode for signalling proteins that are critical for maintaining cellular proliferation and survival

**Origin of replication:** a particular sequence in a vector, plasmid or genome at which replication is initiated.

**ORF:** Open reading frame. Stretch of genetic material that encodes a translation frame for synthesis of a protein (polypeptide) by the ribosome

**Overhang:** A single stranded sequence at the terminus of a double stranded nucleic acid molecule. Often referred to as sticky or cohesive ends.

**Para-reactivity:** in the present context means reactivity towards an HLA that does not belong to the putative HLA but presents the relevant peptide

**PCR:** Polymerase chain reaction in which a specific target DNA molecule is exponentially amplified

**Peptide:** short string of amino acids between 6 - 30 amino acids in length

**Phenotypic analysis:** Analysis of the observable characteristics of a cell.

**Plasmid:** A genetic construct can replicate independently of the chromosomes, typically a small circular DNA strand in the cytoplasm of a bacterium or protozoan.

**pMHC:** peptide bound to a Major Histocompatibility Class I Complex (p-MHC)

**Polymorphic:** Present in different forms in individuals of the same species through the presence of different alleles of the same gene.

**Polypeptide:** Protein consisting of a stretch of peptides, forming a three-dimensional structure.

**Positive Selection Marker:** A selectable marker that confers positive selection of a vector and/or host organism carrying said marker-bearing vector

**Primer:** Short DNA sequence that allows specific recognition of a target DNA sequence for example during a PCR.

**Professional APC:** any nucleated cell capable of presenting an antigen for sampling by alpha beta and gamma delta T cells presented in HLAII.

**Promoter:** Regulatory DNA element for the controlled initiation of gene expression.

**Recombinase:** Enzymes that mediate genetic recombination.

**Reporter Element:** A genetic element that mediates a reported signal in the organism or vector bearing said element. May be used as a positive or negative selection maker.

**Restriction Enzyme Cleavage Sequence:** The genetic sequence cleaved by a restriction enzyme, which can be extrinsic or intrinsic to the recognition sequence of said restriction enzyme.

**Restriction Enzyme Recognition Sequence:** The genetic sequence recognised and engaged by a restriction enzyme.

**Selectable marker:** A DNA sequence that confers a trait suitable for artificial selection methods

**Sensitivity:** TCR sensitivity is an assessment of the activation of a TCR, when expressed by an engineered TCR-presenting cell, by a target peptide presented in the relevant HLA complex. The lower the numeric value is, the higher the sensitivity is.

**Specific cytotoxicity:** killing of cells that express MAGE-A4, by effector T cells expressing TCR specific for the MAGE-A4 peptide presented in an HLA molecule.

**Splice acceptor site:** A DNA sequence at the 3' end of the intron AM, APX CM or affinity reagent for interaction with cells with TCRsp on the surface, or TCRsp based reagents

**Splice donor site:** A DNA sequence at the 5' end of the intron

**Soluble TCR:** not membrane bound

**Somatic V(D)J recombination:** process after which each T cell expresses copies of a single distinctly rearranged TCR. Refers to recombination at the TRB and TRD loci and additionally include a diversity (D) gene segment.

**Suicide gene:** A gene that will mediate cell death within the host organism carrying said gene. May be used as a positive or negative selection marker.

**Synthetic:** an entity that is artificially generated.

**Targeted endonucleases:** nucleases that can make a double-strand break to the genome and may be used to insert material into the genome

**Targeted lipid nanoparticle:** A lipid nanoparticle targeted to deliver payload to specific cell types based on expression of surface markers. Used for both ex vivo or in vivo payload delivery

**T cell:** T lymphocyte. White blood cell that expresses a T-cell receptor on its surface. Selected by the immune system to not react with the own body but have the potential to recognize infections and malignancies as well as reject grafts from most members of the same species.

**T cell maturation:** process that allows T cells to distinguish cells that belong to the body and are healthy from those that aren't healthy or don't belong to the body at all. Takes place in the thymus

**T cell repertoire:** T cells distinct set of T cell receptors

**T cell Receptor (TCR):** Affinity molecule expressed by a subgroup of lymphocytes called T lymphocytes. In humans the TCR recognizes cargo presented by APX CM or APX AM, including fragments from virus or bacterial infections or cancerous cells. Therefore, the TCR recognition is an integral part of the adaptive immune system. The TCR consists of two chains that are paired on the cell surface. The TCR expressed on the surface of each cells is assembled at random from a large pool of varied genes (the v,d,j and c segments) and thus each individual has a pool of T cells expressing a very large and diverse repertoire of different TCRs.

**TCRsp:** analyte pair of TCR chains that are expressed as TCR surface proteins in complex with CD3

**TCR Drug Conjugates (TDC):** soluble TCR (instead of an antibody) used to target tumour-specific p-MHCs and deliver a toxic drug into tumour cells

**Terminator element:** is a section of nucleic acid sequence that marks the end of a gene or operon in genomic DNA during transcription. This sequence mediates transcriptional termination by providing signals in the newly synthesized mRNA that trigger processes which release the mRNA from the transcriptional complex. These processes include the direct interaction of the mRNA secondary structure with the complex and/or the indirect activities of recruited termination factors. Release of the transcriptional complex frees RNA polymerase and related transcriptional machinery to begin transcription of new mRNAs. The termination element is in the template strand of DNA and consists of two inverted repeats separated by half a dozen bases and followed by a run of adenines (A's).

**Thymic selection:** Immature thymocytes undergo a process of **selection,** based on the specificity of their T-cell receptors. This involves **selection** of T cells that are functional (positive **selection),** and elimination of T cells that are autoreactive (negative **selection).** The medulla of the **thymus** is the site of T Cell maturation.

**tCD34:** a truncated CD34 enrichment tag, i.e. a CD34 that is truncated so that the biological function of CD34 is reduced or removed. Cells can be enriched if they express such a truncated CD34 enrichment tag,

**Tumour associated antigens:** Tumour antigen is an antigenic substance produced in tumour cells, i.e., it triggers an immune response in the host. Tumor antigens are useful tumour markers in identifying and quantifying tumour cells with diagnostic tests and are potential candidates for use in cancer therapy.

**TRA:** TCR alpha encoding locus. One of the four different locus encoding genes that can form a VDJ recombined TCR chain. Translated TCR alpha chain proteins typically pair with translated TCR beta chain proteins to form alpha/beta TCRsp.

**TRB:** TCR beta encoding locus. One of the four different locus encoding genes that can form a VDJ recombined TCR chain. Translated TCR beta chain proteins typically pair with TCR alpha chain proteins to form alpha/beta TCRsp.

**TRD:** TCR delta encoding locus. One of the four different locus encoding genes that can form a VDJ recombined TCR chain. Translated TCR delta chain proteins typically pair with translated TCR gamma chain proteins to form gamma/delta TCRsp.

**TRG:** TCR gamma encoding locus. One of the four different locus encoding genes that can form a VDJ recombined TCR chain. Translated TCR gamma chain proteins typically pair with translate TCR delta chain proteins to form gamma/delta TCRsp.

**Two-component vector system:** a single V-C entry vector and a single J donor vector with desired sequences can be combined with a short DNA oligonucleotide duplex encoding CDR3 (odeCDR3) sequence to reconstitute a full length TCR ORF in vitro in a single-tube reaction, in a restriction enzyme and ligase dependent and PCR independent manner.

**Type** I **transmembrane domain:** single-pass molecules anchored to the lipid membrane with a stop-transfer anchor sequence and their N-terminal domain targeted to the endoplasmic reticulum lumen during synthesis (and the extracellular space, if mature forms are located on the Plasmalemma).

**Type IIS Restriction Enzyme:** restriction enzymes that recognize asymmetric DNA sequences and cleave outside of their recognition sequence.

**V (-region):** Variable region. One of the gene segments that is used to assemble the T-cell receptor. Each individual has a large number of different variations of these regions making it possible for each individual to arm T cells with a very large variety of different TCR.

**V-C entry vector:** The vector of the two-component vector system that carries the V and C TCR segments, and which receives sequences from the J donor vectors and odeCDR3 during reconstitution of a full-length TCR ORF.

**V cloning fragment:** Variable Cloning fragment. Also referred to as a V gene segment cloning fragment. A construct carrying a portion of a V gene segment used to construct a V-C entry vector.

**Vector:** A vector is a genetic construct that carries genetic information. In the present context vector usually describes plasmid DNA vectors. A vector can represent any such construct that can be propagated and selected in a host organism.

**Z-score:** The z-score is the intensity of a specific peptide as compared to the intensity of all other peptides in the sample, in other words the formula for calculating z-scores takes the raw intensity score of the peptide and subtracts the population mean, and all is divided by the population standard deviation.

### Detailed description of invention

The current invention discloses several verified suitable MAGE-A4 targets as well as TCRs recognising these targets, and which have a particularly suitable profile for therapeutic use in cancer therapy.

The present invention, for the first time, discloses a T cell receptor (TCR), which is isolated and/or expressed in an engineered cell, and which specifically recognizes a melanoma associated antigen A4 (MAGE-A4) peptide, wherein the peptide is a human melanoma associated antigen A4 (MAGE-A4), and wherein the peptide consists of GVYDGREHTV (SEQ ID NO: 1) presented in a HLA-A*02 context.

In one aspect, the invention relates to a method of treating a disorder characterized by MAGE-A4 expression in a subject, such as a human patient, comprising administering to said subject a therapeutically effective amount of genetically engineered cells that express a TCR as disclosed and/or defined herein, or a pharmaceutical composition disclosed and/or defined herein.

In an aspect a TCR according to the invention is capable of specifically recognising peptide of SEQ ID NO: 1 when the peptide is presented in a HLA-A*02 complex, thereby allowing for treatment of patients wherein the peptide is presented in the HLA-A*02 context.

### MAGE-A4

The inventors of the present application have surprisingly found novel functional TCRs that are able to bind to productively engage with a functionally presented target such as, a MAGE-A4 (SEQ ID NO: 1) peptide in the context of HLA-A*02:01 with high affinity and specificity, wherein productive engagement of TCR with said targets triggers a signalling, cytotoxic response in a T cell expressing such TCR.

In one embodiment, as demonstrated in the experimental section, the TCR according to the current invention is a TCR, which, when expressed in an engineered cell, has at the most 4% cross-reactivity towards any somatically expressed HLA-presented peptide other than a MAGE-A4 peptide, which consists of GVYDGREHTV (SEQ ID NO: 1) and which is presented in a HLA-A*02:01 complex in a cross-reactivity assay.

A TCR disclosed herein typically comprises:
a. a TCR alpha chain CDR3 sequence with at least 75% identity, such as at least 80% identity, to a TCR alpha chain CDR3 sequence selected from the group consisting of SEQ ID NO: 46, SEQ ID NO: 48 and SEQ ID NO: 50,
   and
b. a TCR beta chain CDR3 sequence with at least 75% identity, such as at least 80% identity, to a TCR beta chain CDR3 sequence selected from the group consisting of SEQ ID NO: 47, SEQ ID NO: 49 and SEQ ID NO: 51.

In embodiments, the TCR according to the current invention comprises:
a. a TCR alpha chain CDR3 sequence with a TCR alpha chain CDR3 sequence selected from the group consisting of SEQ ID NO: 46, SEQ ID NO: 48 and SEQ ID NO: 50,
   and
b. a TCR beta chain CDR3 sequence with a TCR beta chain CDR3 sequence selected from the group consisting of SEQ ID NO: 47, SEQ ID NO: 49 and SEQ ID NO: 51.

In a currently preferred embodiment of the invention, the TCR alpha chain variable, domain scaffold regions, and/or CDRs comprise and/or consist of one of the sequences selected from the group consisting of SEQ ID NO: 57, SEQ ID NO: 59 and SEQ ID NO: 61 and the beta chain variable, domain scaffold regions, and/or or CDRs comprise and/or consist of one of the sequences selected from the group consisting of SEQ ID NO: 58, SEQ ID NO: 60, SEQ ID NO: 62 and SEQ ID NO: 83

In an even more preferred embodiment of the invention, the TCR comprises an alpha chain paired with a beta chain, wherein such pairs are selected from the group of alpha:beta chain pairs consisting of SEQ ID NO:57 and SEQ ID NO 58; SEQ ID NO: 59 and SEQ ID NO 60; SEQ ID NO: 61 and SEQ ID NO: 62; SEQ ID NO: 61 and SEQ ID NO: 83.

A TCR according to the current invention can be selected from the group comprising chimeric, humanized and/or human TCRs.

A TCR according to the invention may comprise constant regions of the alpha and beta chains which have been modified to include disulphide bonds, either interchain disulfide bridge (Boulter et al 2003) which has been shown to enable high-yield production of stable, soluble protein from refolded inclusion bodies, or including additional TRA Constant domain : TRB Constant domain disulphides or stabilizing mutations on the surface of the constant domains (Robinson et al 2021). In embodiments, the alpha and/or beta TCR chain comprise(s) a constant region from another mammal, such as, but not limited to, a mouse chimeric constant region(s), wherein, the TCRs can comprise a mouse constant region comprising mouse constant region of an alpha chain and mouse constant region of beta chain, or both. Preferably, the TCR comprises both. It is appreciated by those skilled in the art that as well as replacing the constant region(s) with those from mouse, the constant domains of another mammal such as a rat, chimpanzee, gorilla, macaque or rhesus monkey may be used, which are alternative embodiments.

In aspects, the TCR comprises a TCR alpha chain a TCR beta chain which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein.

In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The alpha/beta paired TCRs of the present description may have an introduced disulfide bond between their constant domains. Preferred TCRs of this type include those which have parts of a TRA constant domain sequence and a TRBI or TRB2 constant domain sequence replaced by cysteine residues, the said cysteines forming a disulfide bond between the TRA constant domain sequence and the TRBI or TRB2 constant domain sequence of the TCR. It is appreciated by those skilled in the art that there are few amino acid residues within the TRA and TRB1/TRB2 sequence chains, respectively, that may be modified to cysteine so as to form a disulfide bond.

With or without the introduced inter-chain bond mentioned above, the alpha/beta paired TCRs of the present description may have a TRA constant domain sequence and a TRB1 or TRB2 constant domain sequences, and the TRA constant domain sequence and the TRB1 or TRB2 constant domain sequences of the TCR may be linked by the native disulphide bond.

The TCR can comprise a human/mouse chimeric TCR. In this regard, the TCR can comprise a mouse constant region of an alpha chain, a mouse constant region of a beta chain, or both mouse constant regions. Preferably, the TCR comprises both alpha and beta mouse constant regions. It is appreciated by those skilled in the art that, as well as replacing the constant region(s) with those from mouse, the constant domains of another mammal such as a rat, chimpanzee, gorilla, macaque or rhesus monkey may be used, which are alternative embodiments.

Alternatively or additionally, the inventive human/ mouse chimeric TCR can comprise any of the alpha chain CDR3s of the invention.

Alternatively or additionally, the inventive human/ mouse chimeric TCR can comprise any of the beta chain CDR3s of the invention.

### Specifically recognizing

In the context of the present specification, "specifically recognizing" means that a TCR recognises a target peptide presented by a specific HLA allele.

The current invention relates to several new T cell receptors (TCR), which are isolated and/or expressed in an engineered cell, and which specifically recognize a melanoma associated antigen A4 (MAGE-A4) peptide, which consists of GVYDGREHTV (SEQ ID NO: 1) presented in a HLA-A*02 complex, and wherein the individual TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

TCRs according to invention, when expressed in an engineered cell, do not cause specific cytotoxic activity towards cells not expressing a MAGE-A4 peptide.

TCRs according to the invention, when expressed in an engineered cell, do not specifically recognize the MAGE-A4 peptide, wherein the peptide consists of GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01, HLA-A*02:02, HLA-A*02:04, HLA-A*02:05, HLA-A*02:06, HLA-A*02:07 and HLA-B*57:01.

TCRs according to the invention, when expressed in an engineered cell, do not specifically recognize the MAGE-A4 peptide, wherein the peptide consists of GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01, HLA-A*02:02 and HLA-A*02:07.

TCRs according to the invention, when expressed in an engineered cell, do not specifically recognize the MAGE-A4 peptide, wherein the peptide consists of GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01, HLA-A*02:02, HLA-A*02:03, HLA-A*02:04, HLA-A*02:05, HLA-A*02:06, HLA-A*02:07, HLA-A*68:02 and HLA-B*15:01.

Furthermore, when expressed in an engineered cell, a TCR according to the invention does not recognise allelic variants of the human leukocyte antigen (HLA) molecule other than one or more allelic variant of the human leukocyte antigen (HLA) molecule selected from the group consisting of HLA-B*57:01, HLA-A*02:02 and HLA-B*15:01.

Disclosed herein is a TCR that when expressed in an engineered cell, does not specifically recognize the MAGE-A4 peptide, wherein the peptide consists of GVYDGREHTV (SEQ ID NO: 1) presented in an allelic variant of the human leukocyte antigen (HLA) molecule other than the HLA-A*02 and/ or HLA-A*11:04 and/or HLA-A*29:02 and/or HLA-A*30:02 and /or HLA-A*68:02 and/ or HLA-B*38:01 and/ or HLA-B*54:01 and/or HLA-C*07:01 and/ or HLA-C*12:03 and/ or HLA-C*15:02.

In one embodiment, TCRs according to the invention do not specifically recognize a MAGE-A4 peptide, wherein the peptide consists of GVYDGREHTV (SEQ ID NO: 1) presented in any of the following HLA-A*01-01, HLA-A*01-02, HLA-A*01-03, HLA-A*03:01, HLA-A*11:01, HLA-A*11:02, HLA-A*11:03, HLA-A*11:04, HLA-A*11:05, HLA-A*11:09, HLA-A*23:01, HLA-A*24:02, HLA-A*24:03, HLA-A*24:04, HLA-A*24:07, HLA-A*24:10, HLA-A*24:17, HLA-A*25:01, HLA-A*26:01, HLA-A*29:02, HLA-A*30:01, HLA-A*30:02, HLA-A*31:01, HLA-A*32:01, HLA-A*33:01, HLA-A*33:03, HLA-A*34:02, HLA-A*66:01, HLA-A*66:02, HLA-A*68:01, HLA-A*80:01, HLA-B*07:02, HLA-B*07:05, HLA-B*08:01, HLA-B*13:01, HLA-B*13:02, HLA-B*14:01, HLA-B*14:02, HLA-B*15:02, HLA-B*15:03, HLA-B*18:01, HLA-B*27:05, HLA-B*35:01, HLA-B*35:02, HLA-B*35:03, HLA-B*35:05, HLA-B*35:08, HLA-B*35:12, HLA-B*37:01, HLA-B*38:01, HLA-B*40:01, HLA-B*40:02, HLA-B*44:02, HLA-B*44:03, HLA-B*49:01, HLA-B*50:01, HLA-B*51:01, HLA-B*51:02, HLA-B*52:01, HLA-B*53:01, HLA-B*54:01, HLA-B*55:01, HLA-B*58:01, HLA-C*01:02, HLA-C*02:02, HLA-C*03:03, HLA-C*03:04, HLA-C*04:01, HLA-C*05:01, HLA-C*06:02, HLA-C*07:01, HLA-C*07:02, HLA-C*08:01, HLA-C*08:02, HLA-C*12:02, HLA-C*12:03, HLA-C*14:02, HLA-C*15:02, HLA-C*16:01, HLA-E*01:01, HLA-E*01:03

In one embodiment, as exemplified in the experimental section, the TCR according to the current invention is a TCR, which, when expressed in an engineered cell, has at the most 4% cross-reactivity towards any somatically expressed HLA-presented peptide other than a MAGE-A4 peptide, which consists of GVYDGREHTV (SEQ ID NO: 1) and/or GLYDGREHSV (SEQ ID NO: 85) and which is presented in a HLA-A*02:01 complex in a cross-reactivity assay.

### Alloreactivity "off-HLA" recognition by T cell.

Alloreactivity in the context of the present invention means an undesirable reactivity of the TCR towards a peptide-free HLA not belonging to a putative HLA for that TCR, i.e., in the case of a TCR specific for MAGE-A4, reactivity towards an HLA that does not belong to the HLA-A*02 supergroup and at the same time does not present a peptide.

### Para-reactivity "on-target-off-HLA" recognition by a T cell

Para-reactivity in the present context means reactivity towards an HLA that does not belong to the putative HLA but presents the relevant peptide, i.e. in the present context an HLA not belonging to the HLA-A*02 supergroup but presenting a MAGE-A4 peptide, wherein the peptide consists of GVYDGREHTV (SEQ ID NO: 1).

### Strong cytotoxic activity

Strong cytotoxic activity in the current context means T cell cytotoxic activity towards a target cell population resulting in an increase in specific lysis of the target cell population that is increased more than 4.5-fold when target cells are co-cultured with T cells in a 1:1 ratio compared to a 1:4 or to a 1:16 T cell to target cell ratio.

### TCRs of the current invention

A TCR disclosed herein typically comprises:
a. a TCR alpha chain CDR3 sequence with at least 80% identity to a TCR alpha chain CDR3 sequence selected from the group consisting of SEQ ID NO: 46, SEQ ID NO: 48 and SEQ ID NO: 50.
   and
b. a TCR beta chain CDR3 sequence with at least 80% identity to a TCR beta chain CDR3 sequence selected from the group consisting of SEQ ID NO: 47, SEQ ID NO: 49 and SEQ ID NO: 51.

In embodiments, the TCR according to the current invention comprises:
a. a TCR alpha chain CDR3 sequence with a TCR alpha chain CDR3 sequence selected from the group consisting of SEQ ID NO: 46, SEQ ID NO: 48 and SEQ ID NO: 50.
   and
b. a TCR beta chain CDR3 sequence with a TCR beta chain CDR3 sequence selected from the group consisting of SEQ ID NO: 47, SEQ ID NO: 49 and SEQ ID NO: 51.

In a currently preferred embodiment of the invention the TCR alpha and beta chain variable domain scaffold regions or CDRs of the TCR comprises and/or consists of one of the sequences selected from the group consisting of SEQ ID NO: 57, SEQ ID NO: 58, SEQ ID NO: 59, SEQ ID NO: 60, SEQ ID NO: 61, SEQ ID NO: 62 and SEQ ID NO: 83.

In a preferred embodiment the TCR comprises a paired alpha and beta chain, wherein the pairs are selected from the group consisting of SEQ ID NO: 57 and SEQ ID NO: 58; SEQ ID NO: 59 and SEQ ID NO: 60; SEQ ID NO: 61 and SEQ ID NO: 62; SEQ ID NO: 61 and SEQ ID NO: 83. Such pairs may also encompass sequences having at least 80% sequence identity to said sequences; such as at least 90% sequence identity, such as at least 95% sequence identity, such as at least 98% sequence identity or more.

A TCR according to the current invention can be selected from the group comprising chimeric, humanized and/or human TCRs.

A TCR according to the invention can comprise constant regions of the alpha and beta chains which have been modified to include cysteine residues to allow covalent connection by di-sulphide bridges or comprise no covalent connections between said paired alpha and beta chains. In embodiments, the alpha and/or beta chain comprise(s) a constant region of another mammal, such as, but not limited to, a mouse chimeric constant region(s). Further, the alpha and/or beta chain may have been modified to include cysteine residues to allow disulphide bridges.

In aspects, the TCR comprises a TCR alpha chain a TCR beta chain which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein.

In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The current invention in one aspect relates to a soluble TCR and/or a conjugated soluble TCR according to the current invention. In aspects, the TCR is a bi-specific T-cell engager (BiTE), or it is an immune mobilizing monoclonal T-cell receptor.

The TCR in one aspect TCR comprises a paired TCR alpha- and TCR beta-chain comprising a complete assembly of combinations of the following sequences:

| | SEQ ID NO | SEQ ID NO | SEQ ID NO | SEQ ID NO |
|---|---|---|---|---|
| SC1alpha | 11 | 13 | 11 | 13 |
| CDR1alpha | 32 | 34 | 32 | 34 |
| SC2alpha | 18 | 20 | 18 | 20 |
| CDR2alpha | 39 | 41 | 39 | 41 |
| SC3alpha | 25 | 27 | 25 | 27 |
| CDR3alpha | 46 | 48 | 50 | 48 |
| SC4alpha | 5 | 5 | 8 | 5 |
| SC5alpha | 54 | 54 | 54 | 54 |
| SC1beta | 12 | 14 | 15 | 14 |
| CDR1beta | 33 | 35 | 36 | 35 |
| SC2beta | 19 | 21 | 22 | 21 |
| CDR2beta | 40 | 42 | 43 | 42 |
| SC3beta | 26 | 28 | 29 | 28 |
| CDR3beta | 47 | 49 | 51 | 49 |
| SC4beta | 6 | 7 | 9 | 7 |
| SC5beta | 55 | 56 | 55 | 82 |

or sequences having at least 90% such as at least 95%, at least 97% or at least 99%, or 100% identity to said respective sequences.

In one aspect the TCR comprises a paired TCR alpha- and TCR beta-chain comprising the following combination of sequences:

| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 11 | SEQ ID NO: 32 | SEQ ID NO: 18 | SEQ ID NO: 39 | SEQ ID NO: 25 | SEQ ID NO: 46 | SEQ ID NO: 5 | SEQ ID NO: 54 |
| TCR-2 alpha | SEQ ID NO: 13 | SEQ ID NO: 34 | SEQ ID NO: 20 | SEQ ID NO: 41 | SEQ ID NO: 27 | SEQ ID NO: 48 | SEQ ID NO: 5 | SEQ ID NO: 54 |
| TCR-3 alpha | SEQ ID NO: 11 | SEQ ID NO: 32 | SEQ ID NO: 18 | SEQ ID NO: 39 | SEQ ID NO: 25 | SEQ ID NO: 50 | SEQ ID NO: 8 | SEQ ID NO: 54 |
| TCR-1 beta | SEQ ID NO: 12 | SEQ ID NO: 33 | SEQ ID NO: 19 | SEQ ID NO: 40 | SEQ ID NO: 26 | SEQ ID NO: 47 | SEQ ID NO: 6 | SEQ ID NO: 55 |
| TCR-2 beta | SEQ ID NO: 14 | SEQ ID NO: 35 | SEQ ID NO: 21 | SEQ ID NO: 42 | SEQ ID NO: 28 | SEQ ID NO: 49 | SEQ ID NO: 7 | SEQ ID NO: 56 |
| TCR-2 beta | SEQ ID NO: 14 | SEQ ID NO: 35 | SEQ ID NO: 21 | SEQ ID NO: 42 | SEQ ID NO: 28 | SEQ ID NO: 49 | SEQ ID NO: 7 | SEQ ID NO: 82 |
| TCR-3 beta | SEQ ID NO: 15 | SEQ ID NO: 36 | SEQ ID NO: 22 | SEQ ID NO: 43 | SEQ ID NO: 29 | SEQ ID NO: 51 | SEQ ID NO: 9 | SEQ ID NO: 55 |

or sequences having at least 90% such as at least 95%, at least 97% or at least 99%, or 100% identity to said respective sequences.

In one aspect the alpha and beta chain CDRs have the following sequences:

| CDR1 alpha | CDR2 alpha | CDR3 alpha | CDR1 beta | CDR2 beta | CDR3 beta |
|---|---|---|---|---|---|
| SEQ ID NO: 32 | SEQ ID NO: 39 | SEQ ID NO: 46 | SEQ ID NO: 33 | SEQ ID NO: 40 | SEQ ID NO: 47 |
| SEQ ID NO: 34 | SEQ ID NO: 41 | SEQ ID NO: 48 | SEQ ID NO: 35 | SEQ ID NO: 42 | SEQ ID NO: 49 |
| SEQ ID NO: 32 | SEQ ID NO: 39 | SEQ ID NO: 50 | SEQ ID NO: 36 | SEQ ID NO: 43 | SEQ ID NO: 51 |

In one aspect the alpha and beta chain CDR3s have the following sequences:

| CDR3 alpha | CDR3 beta |
|---|---|
| SEQ ID NO: 46 | SEQ ID NO: 47 |
| SEQ ID NO: 48 | SEQ ID NO: 49 |
| SEQ ID NO: 50 | SEQ ID NO: 51 |

### Nucleic acid sequences

In one aspect, the invention relates to one or more nucleic acid sequences encoding a TCR comprising a paired TCR alpha- and TCR beta-chain as disclosed, described and/or defined herein.

The nucleic acids of the invention are recombinant. As used herein, the term "recombinant" refers to (i) molecules that are constructed outside living cells by joining natural and/or synthetic nucleic acid segments to nucleic acid molecules that can replicate in a living cell, or (ii) molecules that result from the replication of those described in (i) above. For purposes herein, the replication can be *in vitro* replication or *in vivo* replication.

A desirable method of modifying the DNA encoding the polypeptide of the description employs the polymerase chain reaction as disclosed by Saiki R K, et al. (Saiki et al., 1988).

This method may be used for introducing the DNA into a suitable vector, for example by engineering in suitable restriction sites, or it may be used to modify the DNA in other useful ways as is known in the art.

Nucleic acid sequences encoding a TCR comprising a paired TCR alpha- and TCR beta-chains may be but are not limited to, linear or circular double stranded DNA, or linear or circular single stranded DNA. Such a sequence may contain replication-directing elements for self-amplification and/or may be circularized. The DNA may or may not be chemically modified.

Alternatively, nucleic acid sequences encoding a TCR comprising a paired TCR alpha- and TCR beta-chains may be but are not limited to linear or circular single stranded RNA. Such RNA may or may not be chemically modified.

A nucleic acid sequence encoding a TCR may be comprised in or by an RNA vector. Such an RNA vector may contain a 5' CAP, 5' UTR, 3' UTR and 3' poly(A) tail and Internal Ribosome Entry Site (IRES), and may contain substituted nucleotides such as pseudouridine. An RNA vector may contain replication-directing elements for self-amplification and/or may be circularized.

### Identity vs similarity

By an amino acid sequence having an amino acid sequence that is at least, for example 95% identical to a reference amino acid sequence, is intended that the amino acid sequence of e.g., the TCR, or CDR3, is identical to the reference sequence, except that the amino acid sequence may include up to 5 point mutations and/or deletions per each 100 amino acids of the reference amino acid sequence. In other words, to obtain a TCR, or CDR3 having an amino acid sequence that is at least 95% identical to a reference amino acid sequence, up to 5% of the amino acids in the reference sequence may be deleted or substituted with another amino acid, or a number of amino acids. In an amino acid sequence that is at least 95% identical to a reference amino acid sequence up to 5% of the amount of total amino acids in the reference sequence may be inserted and/or added into the reference sequence. The mutations and/or insertions of the reference sequence may occur at the amino or carboxy terminal positions of the reference amino acid sequence or anywhere between those terminal positions, interspersed either individually among amino acids in the reference sequence or in one or more contiguous groups within the reference sequence.

In the present invention, a local algorithm program is best suited to determine identity. Local algorithm programs, (such as Smith Waterman) compare a subsequence in one sequence with a subsequence in a second sequence and find the combination of subsequences and the alignment of those subsequences, which yields the highest overall similarity score. Internal gaps, if allowed, are penalized. Local algorithms work well for comparing two or more multi domain proteins, which have a single domain, or just a binding site in common.

Methods to determine identity and similarity are codified in publicly available programs. Preferred computer program methods to determine identity and similarity between two sequences include, but are not limited to BLASTX, BLASTP, BLASTN, and FASTA (Altschul et al 1990). The BLAST programs are publicly available from NCBI and other sources (BLAST Manual, Altschul et al, 1990). Each sequence analysis program has a default scoring matrix and default gap penalties. In general, a molecular biologist would be expected to use the default settings established by the software program used.

### Modalities for Gene delivery in TCR-T Engineering

TCR-T therapies rely on genetic modification of T cells to express non-endogenous TCRs that target specific pMHC. The process involves the introduction of TCR-encoding sequences into T cells to enable their ability to recognize and kill cancerous or infected cells. The manufacturing process can employ various approaches and the effectiveness of the generated TCR-Ts is highly dependent on the gene delivery/transfer method used. TCR-T generation can be classified into two main manufacturing approaches; ex vivo manufacturing, where T cells are isolated, modified, expanded outside the body, and infused into the patient, and; in vivo manufacturing, where T cells are modified directly within the patient without ex vivo manipulation. Both approaches may leverage viral vector-based delivery, non-viral vector-based delivery, with transient or stable expression of gene constructs, each with distinct advantages and limitations. Whichever method is chosen, it must achieve stable or transient TCR expression, efficient transduction or transfection, minimal genomic toxicity, scalability and clinical feasibility.

In aspects of the invention, the vector disclosed, described and/or defined herein is a nucleic acid vector, viral vector or a nanoparticle vector. In an aspect, the invention relates to a vector system directing the expression of a payload comprising one or more nucleic acid sequences encoding a TCR comprising a paired TCR alpha- and TCR beta-chains as disclosed, described and/or defined herein. In an aspect, the invention relates to a vector system delivering a gene editing package.

The vectors of the current invention can be designed for transient expression, for stable expression, or for both, and may be used in both *ex vivo* manufacturing and *in vivo* therapeutic approaches.

### Viral vectors

Viral vectors are often chosen due to their high transduction efficiency and ability to achieve stable integration or episomal expression. For manufacture of TCR-T cells, viral vectors can be engineered to package the nucleic acid sequences encoding at least a TCR comprising a paired TCR alpha- and TCR beta-chains that comprise of, but are not limited to, double stranded DNA (dsDNA), single stranded DNA (ssDNA), double stranded RNA (dsRNA) or single stranded RNA (ssRNA).

Most common viral vectors used to generate TCR-Ts are lentiviruses (LV), retroviruses (RV), adenoviruses (AV), adeno-associated viruses (AAV) and herpes simplex viruses (HSV). For example, LVs are widely used due to their ability to integrate into the host genome, ensuring stable, long-term expression of the therapeutic TCR. RVs, have for similar reasons also been extensively used in clinical manufacturing strategies for TCR therapy. Modified LV and RV that display tropism for T-cells may be used for targeted delivery of TCR gene constructs in vivo. AVs have high efficiency and non-integrating nature, yet are highly immunogenic and therefore repeated dosing is limited due to development of immune response in an in vivo setting. AAVs are feasible for TCR gene delivery due to their low immunogenicity, non-integrating nature and long-term expression. AV and AAV have been used to deliver DNA templates for targeted-endonuclease mediated nitration of gene constructs by homologous directed recombination.

### Non-Viral vectors

### Nucleic Acid Vectors

Nucleic acid vectors do not require viral production and therefore enable easier large-scale production. In the current context, a " nucleic acid vector", is any molecule or composition that has the ability to carry a nucleic acid sequence into a suitable host cell where synthesis of the encoded polypeptide can take place *in vivo.* Recombinant expression vectors designed to maintain the recombinant DNA in bacteria are also included.

Typically, and preferably, a vector comprises a nucleic acid sequence that has been engineered, using recombinant DNA techniques or synthetic synthesis that are known in the art, to incorporate a desired nucleic acid sequence (e.g., a nucleic acid sequence of the invention). Desirably, the vector is comprised of any type of nucleotides, including, but not limited to DNA and RNA, which can be single-stranded or double stranded, synthesized or obtained in part from natural sources, and which can contain natural, non-natural or altered nucleotides. The vectors of the invention are not naturally-occurring as a whole. However, parts of the vectors can be naturally-occurring. Preferably, the non-naturally occurring or altered nucleotides or inter-nucleotide linkages do not hinder the transcription or replication of the vector.

Said nucleic acid vector can be selected from the group consisting of expression vector, a cloning vector, phagemid, cosmid, transposon, episome, artificial chromosome or RNA. The invention relates to a nucleic acid vector comprising one or more nucleic acid sequences encoding a TCR comprising a paired TCR alpha- and TCR beta-chains as but are not limited to, linear or circular double stranded DNA, or linear or circular single stranded DNA, that may or may not be chemically modified DNA, linear or circular double stranded RNA or non-coding double-stranded RNA, linear or circular single stranded RNA, that may or may not be chemically modified RNA. A vector as described herein can comprise transacting genetic element, such as a microRNA, small hairpin (sh)RNA and/or long non-coding IcRNA sequence is designed to modulate a endogenous transcript within the target cell. The RNA vector may contain a 5' CAP, 5' UTR, 3' UTR and 3' poly(A) tail and IRES, and may contain substituted nucleotides such as pseudouridine, and may contain replication-directing elements for self-amplification and/or may be circularized.

The nucleic acid vector of the invention can be any suitable recombinant expression vector and can be used to transform or transfect any suitable host cell. Suitable vectors include those designed for propagation and expansion or for expression or both, such as plasmids and viruses, and are well known to those skilled in the art, as are methods to construct and use them. In a preferred embodiment, the recombinant expression vectors used include a nucleic acid vector, e.g., linear or circular double stranded DNA, or linear or circular single stranded DNA, or linear or circular RNA used to transduce a host cell during the production of a pharmaceutical preparation, as well as recombinant expression vectors designed to maintain the recombinant DNA in bacteria. Such vectors may direct transient or stable expression. Stable expression can be achieved by integration of nucleic acid construct into the host cell genome, or maintenance of expression vector nucleic acid in episomal or other structures that achieve nucleic acid maintenance and TCR-encoding construct expression.

Desirably, a vector according to the current invention comprises regulatory sequences, such as transcription and translation initiation and termination codons, which are specific to the type of host into which the vector is to be introduced, as appropriate and taking into consideration whether the vector is DNA- or RNA-based. The alpha and beta paired chains of a TCR of the present invention may be encoded by one or more nucleic acid sequences located in separate vectors or may be encoded by polynucleotides located in the same vector. In preferred embodiments, TCR-alpha and TCR-beta chains of the introduced TCR may be cloned into bicistronic constructs in a single vector.

A common nucleic acid vector gene delivery approaches *in vitro*/*ex vivo* are electroporation-based delivery, that uses electrical pulses to introduce plasmid DNA, mRNA and/or gene integration components into T cells, such as CRISPR/Cas RNPs or transposases.

Another delivery approach is to encapsulate DNA or RNA payloads into non-viral delivery particles such as nanoparticles, to create a nanoparticle vector, and polymer-based and peptide-based delivery systems that utilize synthetic carriers to transport nucleic acid into cells.

### Non-viral delivery particle vectors

Non-viral delivery particle vectors are engineered carriers used to introduce genetic material, proteins or other therapeutic agents into cells. Non-viral delivery particle vectors can be designed to increase stability, cellular uptake and targeted delivery while at the same time reducing immunogenicity and manufacturing complexity.

A non-viral delivery particle vectors can be selected from the group consisting of lipid nanoparticles (LNPs) or nanoparticles (NPs), cationic liposome complexes, cationic polymer complexes, cationic liposome-polymer complexes, exosomes, liposomes, and inorganic nanoparticles. In certain aspects, a nanoparticle vector (or a non-viral delivery particle) according to the current invention is a LNP comprising ionizable cationic lipid, a sterol, a phospholipid, a non-functionalized PEG-lipid, and a functionalized PEG-lipid wherein the functionalized PEG-lipid has been optionally conjugated with a binding moiety. The nanoparticle vector encapsulates a payload in the form of a nucleic acid, such as mRNA, for the purpose of transfecting a target cell. The mRNA payload may contain a linear expression construct with 5' cap, 5' UTR, 3' UTR and poly-A features, IRES, and may contain substituted nucleotides such as pseudouridine, and may contain replication-directing elements for self-amplification. The mRNA payload may also be circular.

### Genome editing-based gene integration

These technologies allow precise gene integration the can enhance TCR performance, improve safety, and enable allogenic TCR-T approaches. With these methods it is possible to modify the genome at specific sites and therefore avoid random integration, improving TCR expression and reducing safety risks. This approaches can be used for generation of fully allogeneic universal donor T cell products by eliminating endogenous TCR and HLA characteristics (Su et al 2017), (Qasim et al 2017), elimination of endogenous TCRs to increase expression and function of introduced transgenic TCR and eliminate potential cross-reactivities (Provasi et al. 2012), eliminating immune inhibitory receptors to engineer resistance to immune checkpoint ligands (Su et al 2016), discovery of novel targets for enhanced combinatorial immune engineering (Shifrut et al 2018). Most common gene editing methods are CRISPR/Cas9, used for precise knock-in of TCR genes or knock out of inhibitory receptors (eg PD-1, endogenous TCR) and TALENs/ZFNs which are programmable nucleases used for targeted TCR gene integration; however, other nucleases or endonucleases may also be suitable for gene editing.

### Modification of vector systems to improve T cell tropism, enhance safety and optimise expression

### T cell tropism Strategies for selective gene delivery

Viral vectors can comprise one or more cell surface receptors that bind to a ligand on a target cell and aid transduction; heterologous viral envelope glycoproteins; fusion glycoproteins; T cell activation or co-stimulation molecules, cytokines or cytokine-based transduction enhancers; and/or transmembrane proteins comprising a mitogenic domain and/or cytokine-based domain exposed on the surface and/or conjugated to the surface of the viral vector. Furthermore, to effectively transduce mammalian cells, Lentiviral viruses can be engineered to alter their tropism, by means of pseudotyping with various heterologous viral envelopes. The most common one being the envelope glycoprotein from vesicular stomatitis virus (VSV-G) (Joglekar et al 2017). The vector can further be pseudotyped by exchanging the VSV-G for other heterologous viral envelopes glycoproteins, such as those from the Measles virus, Baboon endogenous retrovirus, Cocal virus, Nipah virus or Sendai virus (Gutierrez-Guerrero et al 2020). For example, the Nipah envelope protein can be engineered to bind EpCAM, CD4, or CD8 (Bender et al 2016), CD3, CD5 or other markers suitable for targeted cellular delivery of a TCR-encoding nucleic acid vector.

### AAvs can be engineered for T cell targeting by altering the capsid proteins (e.g. AAV6).

The LNP can be targeted (tLNP) to deliver payload to cell types based on expression of cell surface markers (Tombácz et al. (2021), Breda et al. (2023)) for example LNP may be conjugated to CD5, CD3, CD4, CD8 amongst others, to be used for targeting CD8⁺ and CD4+ T cells (Wada (2018); Dalloul, (2009), Lee (2021)) or CD16 or NK1.1 for targeting NK cells, EpCAM for targeting epithelial cells or to another marker or a functional ligand thereof that targets cellular delivery to specific target cells. Such tLNP particles may be used both ex vivo and in vivo to direct the mRNA payload to a desired immune cell type (Parayath et al 2020), (Siebart et al 2024), (Vavassori et al 2023).

### Enhancing safety

Safety may be enhanced by using integration-deficient lentiviral vectors that prevent permanent genome modifications while maintaining high transduction efficiency; use of suicide switches and safety elements that allow selective elimination of engineered cells (eg iCaspase-9 safety switch (Straathof et al 2005), controlled depletion of TCR-T cells in case of adverse events (eg herpes-simplex virus thymidine kinase (HSV-TK) suicide gene, cellular elimination tags (CETs) and preventing expression in non-target cell populations using microRNA (miRNA)-based regulation.

### Optimising expression

Optimal expression can be achieved by careful selection of promoters and/or transgene optimization. Tissue-specific promoters are used to reduce off-T cell expression, inducible promoters are used to enable controlled expression based on environmental stimuli, while use of EF1a, PKG or viral LTR promoters ensures robust-TCR expression in T cells. Transgene optimization methods include but are not limited to codon optimization, incorporation of leader sequence to improve protein localization, and 2A peptide-based co-expression that allows simultaneous expression of TCR a and b chains.

In certain aspects, said vector further comprises a nucleic acid sequence encoding CD8α and/or CD8β. In certain aspects, said vector further and/or alternatively comprises a nucleic acid sequence encoding a truncated CD34 enrichment tag. The isolated TCR nucleic acid according to the current invention and the nucleic acid sequence encoding CD8α and/or CD8β and/or CD34 can operably be encoded within a single expression unit interconnected with an internal ribosome entry site or a nucleic acid sequence encoding a self-cleaving peptide, such as selected from the group consisting of P2A, E2A, F2A and T2A. In certain aspects the isolated TCR nucleic acid according to the current invention and the nucleic acid sequence encoding CD8α and/or CD8β and/or CD34 can be encoded by more than one expression unit with their own promoter and polyadenylation sequence.

A vector as described herein can comprise a native or synthetic promoter operably linked to the nucleotide sequence encoding the TCR, polypeptide, or protein (including functional portions and functional variants thereof), or to the nucleotide sequence which is complementary to or which hybridizes to the nucleotide sequence encoding the TCR, polypeptide, or protein. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter. Preferably strong promoters are used, such as retroviral long terminal repeats (LTRs), cytomegalovirus (CMV), murine stem cell virus (MSCV) U3, phosphoglycerate kinase (PGK), P-actin, ubiquitin, elongation factor (EF)-la (Tsuji et al., 2005), and a simian virus 40 (SV40)/CD43 composite promoter (Cooper et al., 2004; Jones et al., 2009), and the spleen focus forming virus (SFFV) promoter (Joseph et al., 2008), HCMV IE1 promoter. In a preferred embodiment, the promoter is heterologous to the nucleic acid being expressed.

In certain aspects, said vector further comprises a nucleic acid sequence encoding element to promote stable integration the nucleic acid into the host cell genome, including but are not limited to; homologous directed arms (HDR), long terminal repeat (LTR) and inverted terminal repeats (ITR).

A vector as described here in can further comprise further open reading frames that benefit cellular persistence, disease trafficking, metabolic fitness, evasion of immunosuppressive tumour microenvironment or other functional characteristics of a therapeutic cell product.

Optionally, the nucleic acid sequence of the vector may be modified such that it contains codon triplets designed for optimal transcription and translation, and which are optimised for use in host cells, such as, but not limited to insect, bacteria, yeast or human cells.

### Markers of integration

The vector can include one or more marker genes, which act as markers of integration. Marker of integration include genes encoding the likes of green fluorescent protein (GFP), red fluorescent protein (RFP) and blue fluorescent protein (BFP), EGFP as well as other fluorescent proteins, Streptavidin Binding Protein (SBP), b-galactosidase or chloramphenicol acetyltransferase (CAT). In addition, or alternatively, the recombinant expression vector can include one or more marker genes, which are selected from antibiotic resistance genes, deficiency markers. In some embodiments, selection markers are expressed with a polynucleotide that encodes a gene product of interest (eg a binding protein encompassed by the present invention, such as TCR). In one aspect, the recombinant expression vector includes marker genes, such as the truncated CD34 (CD34t) and/or CD19 that marks the transduced host T cells and acts as a selection marker of recovery. In a preferred embodiment, a truncated CD34t and/or CD19 molecule is expressed alongside the TCR of interest within the human cell.

### Modifications to enhance TCR-T expansion and persistence

Genetic modifications that improve T cell proliferation, survival and functionality before infusion may be achieved by cytokine signaling enhancements, eg IL-2, IL-7 and IL-15 overexpression, and the use of anti-apoptotic molecules, eg Bcl-2 overexpression or dominant-negative Fas, or by introduction of dominant-negative TGF-β receptor type II. Improvement of metabolic fitness for expansion can be achieved by enhancing mitochondrial biogenesis and glycolytic optimization, while reduced immune exhaustion can be achieved by combination therapy with checkpoint inhibition. The TCRs themselves can undergo codon optimization to improve expression and may also be provided with promoters that enhance expression and/or introduced into cells where the endogenous TCR chain(s) have been down-regulated, removed, or otherwise inactivated.

Methodologies for enhancement for infusion and persistence in vivo include modification for improved homing and trafficking such as chemokine receptor engineering (CXCR3, CCR5 or CCR7 to enhance trafficking to lymphoid organs or tumour sites or CCR4 to promote targeting to T regulatory cells.

Methodologies to enhance engraftment and in vivo persistence include enrichment for stem-like or central memory T cells to improve longevity and to reduce differentiation-associated exhaustion, avoid use of terminally differentiated effector T cells to prevent rapid decline post transfusion; expression of mutant IL-2 receptors can be used to enhance persistence. Pharmacological variants of IL-2 with differential activation of IL-2 receptor (IL2R) family members may also be used to modify the relative response of different sub-types of T-cells that have different IL2R assemblies.

Methodologies such as promoting cytokine-induced resistance, use of dominant-negative TGF-b receptor or CD47 overexpression may be used to resist the immunosuppressive effects of the tumour microenvironment.

### In-vivo TCR-T manufacturing

These novel approaches aim to modify T cells inside the patient, bypassing ex vivo processing, and may include LNP-mRNA delivery where mRNA encoding TCR is systematically delivered via LNPs; AAV-based in vivo TCR delivery where engineered AAV serotypes transduce circulating T cells; and extracellular vesicle-based TCR delivery which uses exosome-like vesicles to deliver engineered TCR genes to T cells. Delivery specific to target cells, e.g. CD3 or CD8 T-cells, is promoted by the same targeting moieties as may also be used for ex vivo manipulation, providing trophism for the target cell. Such targeting moieties are incorporated into the delivery system by, for example, covalent linkage, or may be selected variants of, e.g. AAV coat proteins. Payloads for this approach may include mRNA encoding TCRs intended for transient expression and may also include gene-editing payloads for germline modification of the target cells.

The above-mentioned viral vectors, non-viral vectors (nucleic acid and non-viral delivery particles) and gene editing approaches can be used during TCR-T manufacturing individually or as various combinations, for example the delivery of a payload combined with a gene editing package. Furthermore, non-viral delivery particles must be engineered to achieve T cell tropism for maximum therapeutic efficacy, minimizing off-target effects and improving safety profiles.

### Host Cells (Engineered cells)

Host cells according to the current invention are cells that have been engineered to contain the nucleic acids and expression vectors as described herein. As described herein but not limited to, the host cells may be primary cells derived from a donor, may be engineered, and may be used for immunotherapy (immunotherapy host cell, TCR-T cells), or they may be cultured mammalian cells. Such cultured cells may be engineered and may e.g. be engineered TCR presenting cells (eTPC). eTPCs may be an adherent cell or a suspended cell, i.e., a cell that grows in suspension. Preferred suitable host cells are of human origin and are known in the art, for instance Jurkat cells. For purposes of amplifying or replicating the recombinant expression vector, the host cell is preferably a prokaryotic cell, e.g., a DH5a cell. Other eukaryotic host cells may include yeast and insect cells.

The present description also relates to a host cell with the integration of a functional TCR that is capable of being expressed from the introduced recombinant vector in the host cell. In a preferred embodiment, the host cell comprising a TCR of the invention is a human T cell of the CD8⁺ and/ or CD4⁺ subtype. In some embodiments, the host cell is selected from the group consisting of a CD8⁺ T cell, a CD4⁺ T cell, T cell progenitor cell, NK cell, NK progenitor cell or differentiated stem cell. The host cells can be allogeneic, autologous, or engineered. In a preferred embodiment, the host cell used is an autologous human CD8⁺ T cell.

The T cells used according to the current invention can come from any source known in the art. For example, T cells can be differentiated *in vitro* from a hematopoietic stem cell population, or T cells can be obtained from a subject, who may or may not be the patient who is intended to be treated. T cells can be obtained from, e.g., peripheral blood mononuclear cells (PBMCs), bone marrow, lymph node tissue, cord blood, thymus tissue, tissue from a site of infection, ascites, pleural effusion, spleen tissue, and tumors. In addition, the T cells can be derived from one or more T cell lines available in the art. T cells can also be obtained from blood collected from a subject using any number of techniques known to the skilled artisan, such as, but not limited to, density gradient centrifugation, FICOLL^{™} separation and/or apheresis.

In one embodiment, the primary immune cells comprising a TCR (TCR-T also referred to as effector cells) of the invention are activated and/or expanded *in vitro.* Methods for activating and expanding T cells are known in the art. Generally, such methods include contacting PBMC or isolated T cells with a stimulatory agent and costimulatory agent, such as anti-CD3 and anti-CD28 antibodies, generally attached to a bead or other surface, in a culture medium with appropriate cytokines, such as IL-2. Anti-CD3 and anti-CD28 antibodies attached to the same bead serve as a "surrogate" antigen presenting cell (APC). One example is the Dynabeads^{®} system, a CD3/CD28 activator/stimulator system for physiological activation of human T cells.

A further aspect of the invention relates to an immortalized engineered cell expressing the TCR as disclosed, described and/or defined herein and/or the isolated nucleic acid sequence disclosed, described and/or defined herein, and/or which comprises a vector disclosed, described and/or defined herein.

Optionally, the engineered cell according to the current invention comprises a chromosomal gene knock out of one or more TCR gene(s), one or more HLA gene(s), or both, such as wherein the engineered cell comprises a knockout of an HLA gene selected from the group consisting of an a-1-macroglobulin gene, a-2- macroglobulin gene, a-3- macroglobulin gene, b-1-microglobulin gene, b-2-microglobulin gene, and combinations thereof, or such as wherein the engineered cell comprises a knockout of a TCR gene selected from a TCR alpha variable region gene, TCR beta variable region gene, TCR constant region gene, and combinations thereof.

An engineered cell according to the current invention can express CD8a and/or CD8b. Optionally the CD8 alpha and/or CD8 beta is fused to a truncatedCD34 enrichment tag. The engineered cells can be enriched using the truncatedCD34 enrichment tag.

Typically, the engineered cell disclosed, described and/or defined herein, originates from a hematopoietic progenitor cell, peripheral blood mononuclear cell (PBMC), cord blood cell, or immune cell. In an embodiment, the such immune cell is a T cell, cytotoxic lymphocyte, cytotoxic lymphocyte precursor cell, cytotoxic lymphocyte progenitor cell, cytotoxic lymphocyte stem cell, CD4+ T cell, CD8+ T cell, CD4/CD8 double negative T cell, gamma delta (gd) T cell, natural killer (NK) cell, NK-T cell, dendritic cell, or a combination thereof. In one embodiment, the engineered cell is derived from a hematopoietic stem cell (HSC) or an induced pluripotent stem cell (iPSC). The T cell can be a naive T cell, central memory T cell, effector memory T cell, or a combination thereof, or a primary T cell or a cell of a T cell line.

In a currently preferred embodiment, the engineered cell disclosed, described and/or defined herein does not express, or has a lower surface expression of, an endogenous TCR and is capable of producing a cytokine or a cytotoxic molecule when contacted with a target cell that presents a MAGE-A4 peptide, wherein the peptide consists of GVYDGREHTV (SEQ ID NO: 1) presented in a HLA-A*02 complex.

In embodiments, the engineered cell according to the current invention is capable of producing TNF-alpha, IL-2, and/or IFN-gamma, and/or a perforin and/or a granzyme, such as granzyme B. An engineered cell according to the current invention can be contacted with a target cell *in vitro* or *ex vivo* and is preferably capable of producing a higher level of cytokine or a cytotoxic molecule when contacted with a target cell with a heterozygous expression of MAGE-A4, such as an at least 1.05-fold higher level of cytokine or a cytotoxic molecule.

In embodiments, the engineered cell according to the current invention is capable of killing a target cell that presents a MAGE-A4 peptide, wherein the peptide consists of GVYDGREHTV (SEQ ID NO: 1) presented in a HLA-A*02:01 complex. The killing is determined by a killing assay and the ratio of the engineered cell and the target cell in the killing assay is from 1:16 to 1:1, wherein the target cell is a target cell pulsed with a range of between 1x10³ nM and 0.1 nM of a MAGE-A4 peptide, wherein the peptide consists of GVYDGREHTV (SEQ ID NO: 1) presented in a HLA-A*02 complex. In particular the target cell is a cell monoallelic for HLA-A*02:01.

In an embodiment, the engineered cell disclosed, described and/or defined herein is capable of killing a higher number of target cells when contacted with target cells with a heterozygous expression of a MAGE-A4 peptide, wherein the peptide consists of GVYDGREHTV (SEQ ID NO: 1) presented in a HLA-A*02:01 complex. Typically, the cell killing is at least 1.05-fold higher.

In aspects, the target cell is a cell line or a primary cell, such as selected from the group consisting of a cancer cell line, a primary cancer cell line, a transformed cell line, and an immortalized cell line, such as HEK293-, ARH -77-, A375-, U266-, NCI-H1755-, NCI-H1703-, or SAOS-2- derived cell lines. Such cell lines may be modified to stably express HLA-A*02:01 and/or luciferase reporters.

The current invention also relates to a population of engineered cells disclosed, described and/or defined herein as well as to a pharmaceutical composition comprising any one or more of the engineered cells or vectors of the current invention, such as wherein the vectors are selected from the group consisting of viral vectors, non-viral vectors, LNPs or any other carrier comprising the nucleic acid sequence described, disclosed and/or defined herein.

The engineered cells of the invention can substantially cross-react with cells and kill cells that express a polypeptide that contains the natural amino acid sequence of the peptide, such as MAGE-A4 (SEQ ID NO: 1), as defined in the aspects of the description. As can be derived from the scientific literature and databases (Rammensee et al 1999), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. In an aspect, one skilled in the art would have the ability given the teachings of the description to modify the amino acid sequence of a TCR, by modulation of the TCR CDR regions whilst maintaining recognition of the known anchor residues, and would be able to determine whether such TCR variants maintain the ability to bind MHC class I or II molecules/ MAGE-A4 (SEQ ID NO: 1) complexes.

In one embodiment, a soluble TCR of the invention can be produced and used. In this embodiment the host cell can be a cultured mammalian cell. The mammalian host cell can be an adherent cell or a suspended cell, i.e., a cell that grows in suspension. Preferred suitable host cells are from human and are known in the art, for instance HEK293 cells and ARH-77 cells. For purposes of amplifying or replicating the recombinant expression vector, the host cell is preferably a prokaryotic cell, e.g., a DH5a cell. Other eukaryotic host cells may include yeast and insect cells.

Transformation of appropriate cell hosts with a DNA construct of the present description is accomplished by well-known methods that typically depend on the type of vector used. With regard to transformation of prokaryotic host cells, see, for example, Cohen et al. (Cohen et al., 1972) and (Green and Sambrook, 2012). Electroporation is also useful for transforming and/or transfecting cells and is well known in the art for transforming yeast-cells, bacterial cells, insect cells and vertebrate cells.

Successfully transformed cells, i.e., cells that contain a DNA construct of the present description, can be identified by well-known techniques such as polymerase chain reaction (PCR). Alternatively, the presence of protein encoded by such a DNA construct in the supernatant can be detected using antibodies.

### Soluble TCR and/or a conjugated soluble TCR

The current invention in one aspect relates to a soluble TCR and/or a conjugated soluble TCR according to the current invention. In aspects, the TCR is a bi-specific monoclonal antibody (such as T cell engager (BiTE)), or it is an immune mobilizing monoclonal T-cell receptor against cancer (such as ImmTAC).

The invention further relates to a pharmaceutical composition comprising a soluble protein consisting of all or a functional part of a TCR as disclosed and/or defined herein and at least one pharmaceutically acceptable excipient, carrier or stabilizer.

A pharmaceutical composition of the current invention comprises a soluble TCR, a conjugated soluble TCR, a cell expressing a TCR, or a composition comprising a TCR, wherein the TCR is a TCR according to the current invention. Said soluble TCR can been conjugated to a radionuclide, a chemotherapeutic agent, a toxin, or another therapeutically active component, and at least one pharmaceutically acceptable excipient, carrier or stabilizer.

### Pharmaceutical compositions

A "pharmaceutical composition" is a composition suitable for administration to a human being in a medical setting. Preferably, a pharmaceutical composition has minimal bioburden and is produced according to Good Manufacturing Practice (GMP) guidelines.

The TCRs, nucleic acids, recombinant expression vectors, and host cells (including populations thereof) according to the present invention, can be formulated into a pharmaceutical composition. In preferred embodiment, the invention provides a pharmaceutical composition comprising any of the TCRs and functional variants thereof, nucleic acids, expression vectors, host cells such as, but not limited to, TCR-Ts (including populations thereof), and a pharmaceutically acceptable carrier. Alternatively, the pharmaceutical composition can comprise a TCR-T material according to the present invention in combination with another pharmaceutically active agents or drugs, such as a chemotherapeutic agent, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

In a preferred embodiment, the pharmaceutical composition is an autologous, transduced CD8+ and/or CD4+ T cell, infused into a patient following pre-treatment with a conditioning regime, which may include one or more of cyclophosphamide, fludarabine, decitabine or azacytidine.

The pharmaceutical composition of the present description may also include cryoprotective agents (CPAs) such as small-molecular-weight penetrating CPAs (e.g., dimethyl sulphoxide [DMSO], glycerol, ethylene glycol, and propylene glycol) and high-molecular-weight non-penetrating agents (e.g., sucrose, polyvinylpyrrolidone, and hydroxyethyl starch). In preferred embodiment the CPA is DMSO or is derived from DMSO.

In a preferred embodiment the pharmaceutical composition has minimal bioburden and is produced according to GMP guidelines.

Pharmaceutical compositions of the present description may include at least one host cell expressing a TCR of the present description (TCR-T), in a pharmaceutically acceptable carrier.

Pharmaceutical compositions of the present description may also include pharmaceutically acceptable excipients and/ or stabilizers.

In preferred embodiment, the composition is selected for parenteral delivery or for injection/ pumping directly into a solid tumour, into a blood vessel that feeds a tumour or into the volume surrounding a solid tumour. The preparation of such pharmaceutically acceptable compositions is within the ability of one skilled in the art. In certain embodiments, buffers are used to maintain the composition at physiological pH or at a slightly lower pH, typically within a pH range of from about 5 to about 8. In certain embodiments, when parenteral administration is contemplated, the composition is in the form of a pyrogen-free, parenterally acceptable aqueous solution comprising a composition described herein, with or without additional therapeutic agents, in a pharmaceutically acceptable vehicle. In certain embodiments, the vehicle for parenteral injection is sterile distilled water in which composition described herein, with or without at least one additional therapeutic agent, is formulated as a sterile, isotonic solution, properly preserved. In certain embodiments, the preparation involves the formulation of the desired molecule with polymeric compounds (such as polylactic acid or polyglycolic acid), beads or liposomes, that provide for the controlled or sustained release of the product, which are then delivered via a depot injection. In certain embodiments, implantable drug delivery devices are used to introduce the desired molecule.

Injectable formulations are in accordance with the invention. The requirements for effective pharmaceutical carriers for injectable compositions are well-known to those of ordinary skill in the art (see, e.g., Pharmaceutics and Pharmacy Practice, J. B. Lippincott Company, Philadelphia, Pa., Banker and Chalmers, eds., pages 238-250 (1982), and ASHP Handbook on Injectable Drugs, Trissel, 4th ed., pages 622- 630 (1986)). Preferably, when administering cells, e.g., T cells, the cells are administered via injection.

Preferably, the carrier is a pharmaceutically acceptable carrier. With respect to pharmaceutical compositions, the carrier can be any of those conventionally used and is limited only by chemico-physical considerations, such as solubility and lack of reactivity with the active compound(s), and by the route of administration. The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular TCR, as well as by the particular method used to administer the TCR. Accordingly, there are a variety of suitable formulations of the pharmaceutical composition of the invention.

### Uses of the TCRs, nucleic acids and/or vectors and pharmaceutical compositions comprising the same

In one aspect, the current invention relates to a TCR disclosed, described and/or defined herein for use as a medicament. In particular, the current invention relates to a TCR disclosed, described and/or defined herein for use in the treatment of a cancer in a patient carrying HLA-A*02, comprising administering to said patient said TCR or a pharmaceutical composition comprising said TCR, wherein said cancer is a solid malignancy expressing the peptide of SEQ ID NO: 1 and wherein said TCR or a pharmaceutical composition comprising said TCR is infused systemically into said patient or injected or pumped directly into a solid tumour, into a blood vessel that feeds a tumour or into the volume surrounding a solid tumour in said patient.

In aspects, said treatment comprises administering to said patient an engineered cell expressing the TCR, wherein said engineered cell is a T cell, T cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour or into the volume surrounding a solid tumour.

Typically, the use related to herein is for treating a disorder in a subject, wherein said subject carries HLA-A*02:01, however it can also be used for treating a disorder in a subject that carries an alternate subform of HLA-A*02, the use related to herein is for treating a disorder in a subject, wherein said subject carries an alternate subform of HLA-A*02.

The use related to herein is for treating a said subject, wherein said subject carries an alternate subform of HLA-A*02 other than HLA-A*02:03 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention, such as wherein said subject carries HLA-B*57:01.

The use related to herein is for treating a said subject, wherein said subject carries an alternate subform of HLA-A*02 other than HLA-A*02:03, HLA-A*02:04, HLA-A*02:05 and HLA-A*02:06 which alternate subform of HLA-A*02 is capable of specifically presenting the peptide of SEQ ID NO: 1 and which is specifically recognized by the TCR according to the current invention, such as wherein said subject carries a different HLA-A, HLA-B or HLA-C allele than HLA-A*02, that is capable of specifically presenting the peptide of SEQ ID NO: 1 and which is recognized by the TCR according to the current invention.

The use related to herein is for treating a said subject, wherein said subject carries an alternate subform of HLA-A*02 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention, such as wherein said subject carries HLA-A*68:02 and/or HLA-B*15:01.

The use related to herein may typically be for treating a disorder in a subject, wherein said subject carries an alternate subform of HLA-A*02 which alternate subform of HLA-A*02 is capable of specifically presenting the peptide of SEQ ID NO: 1 and wherein the HLA when presenting the peptide is recognized by a TCR disclosed, described and/or defined herein, and/or wherein said subject carries a different HLA-A, HLA-B or HLA-C allele than HLA-A*02, that is capable of specifically presenting the peptide of SEQ ID NO: 1, and which is recognized by the TCR according to the current invention. In addition the use related to herein may be for treating a disorder in a subject, wherein said subject carries an alternate allele than of HLA-A*02 that is able to present the peptide of SEQ ID NO: 1 so that the HLA and peptide are recognised by the TCR . Whether a specific TCR is suitable for use in a subject thus depends on the HLA allotypes carried by the subject. Table 25 provides an overview of included and excluded HLAs for TCRs according to the invention.

### Medical Treatments

In one aspect, the invention relates to a method of treating a disorder characterized by MAGE-A4 expression in a subject comprising administering to said subject a therapeutically effective amount of genetically engineered cells that express a TCR as disclosed and/or defined herein, or a pharmaceutical composition disclosed and/or defined herein, wherein said disorder is a solid malignancy expressing the peptide of GVYDGREHTV (SEQ ID NO: 1), wherein said engineered cell is a T cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour and/or into the volume surrounding a solid tumour.

Said method is in particular intended for treating a disorder in a subject, wherein said subject carries an HLA-A*02 allele capable of specifically presenting the peptide of SEQ ID NO: 1 and which is recognized by the TCR according to the current invention. In addition, said method can be used for treating a said subject, or wherein said subject carries a different HLA-A, HLA-B or HLA-C allele than HLA-A*02, that is capable of specifically presenting the peptide of SEQ ID NO: 1 and which is recognized by the TCR according to the current invention. Table 25 provides suitable HLAs for TCRs according to the present invention.

Said method is intended for treating a subject, wherein said subject carries an alternate subform of HLA-A*02 other than HLA-A*02:03 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention, such as wherein said subject carries HLA-B*57:01.

Said method is intended for treating a said subject, wherein said subject carries an alternate subform of HLA-A*02 other than HLA-A*02:03, HLA-A*02:04, HLA-A*02:05 and HLA-A*02:06 which alternate subform of HLA-A*02 is capable of specifically presenting the peptide of SEQ ID NO: 1 and which is specifically recognized by the TCR according to the current invention, such as wherein said subject carries a different HLA-A, HLA-B or HLA-C allele than HLA-A*02, that is capable of specifically presenting the peptide of SEQ ID NO: 1 and which is recognized by the TCR according to the current invention.

Said method is intended for treating a said subject, wherein said subject carries an alternate subform of HLA-A*02 which alternate subform of HLA-A*02 is capable of specifically presenting the peptide of SEQ ID NO: 1 and which is specifically recognized by the TCR according to the current invention, additionally said method can be used for treating a said subject wherein said subject carries a different HLA-A, HLA-B or HLA-C allele than HLA-A*02, that is capable of specifically presenting the peptide of SEQ ID NO: 1 and which is recognized by the TCR according to the current invention, such as wherein said subject carries HLA-A*68:02 and HLA-B*15:01.

In another embodiment the TCRs, the nucleic acid or the expression vector of the description are used in medicine. For example, the medicament can be used in the treatment of a cancer in a patient possessing HLA-A*02:01 comprising administering to said patient said TCR or a pharmaceutical composition comprising said TCR, wherein said cancer is a solid malignancy expressing the peptide of MAGE-A4 (SEQ ID NO: 1), and wherein said TCR or a pharmaceutical composition comprising said TCR is infused systemically into said patient or injected or pumped directly into a solid tumour, into a blood vessel that feeds a tumour or into the volume surrounding a solid tumour in said patient.

In aspects a pharmaceutical composition comprises a soluble protein consisting of all or a functional part of one or more TCR(s) or one or more engineered cell(s) or one or more vector(s). In aspects the invention relates to one or more compositions each comprising a soluble protein consisting of all or a functional part of one or more TCR(s) or one or more engineered cell(s) or one or more vector(s). Thus, treatment may comprise mono- or multiplexed TCR-T cell therapy, as well as the TCRs of the invention being included in a larger set of TCRs targeting HLA/peptides for individual patients.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of cancer in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset of the disease, or a symptom or condition thereof.

TCRs, nucleic acids and host cells of the present description, and pharmaceutical compositions thereof, may be administered to a subject in need thereof by routes known in the art, and may vary depending on the type of cancer to be treated.

TCRs of the present description may comprise a detectable label selected from the group consisting of a radionuclide, a fluorophore and biotin. TCRs of the present description may be conjugated to a therapeutically active agent, such as a radionuclide, a chemotherapeutic agent, or a toxin.

In preferred embodiments, compositions of the present description are administered to a subject using a single dose.

Effective doses of host cells expressing TCRs of the present invention include, for example at least about 10⁴, at least about 10⁵, at least about 10⁶, at least about 10⁷, at least about 10⁸, at least about 10⁹, at least about 10¹⁰ host cells per dose. In one embodiment, host cells of the present description are administered in a dose of between about 10⁴ to about 10¹⁰ cells per dose, preferably in a dose of between about 10⁵ to about 10⁹ cells per dose. In preferred embodiments, a single dose is administered.

In aspects, TCRs according to the present invention are used in personalised cancer immunotherapy, such as, but not limited to, being administered in combination with a vaccine. In particular the invention relates to a combination of a tumour vaccine with a TCR-T cell therapy.

### Medicament

In an aspect, the invention relates to a method of treating a disorder characterized by MAGE-A4 expression in a subject comprising administering to said subject a therapeutically effective amount of genetically engineered cells that express a TCR as disclosed and/or defined herein, or a pharmaceutical composition disclosed and/or defined herein, wherein said disorder is a solid malignancy expressing the peptide of GVYDGREHTV (SEQ ID NO: 1), wherein said engineered cell is a T cell, T cell progenitor cell, NK cell, NK progenitor cell or a differentiated stem cell and which is either autologous or allogenic, and wherein about 10⁴ to about 10¹⁰ engineered cells in combination with a vaccine are infused systemically into said subject or injected or pumped directly into a solid tumour, into a blood vessel that feeds a tumour and/or into the volume surrounding a solid tumour.

Alternatively, the vaccine may be administered concomitantly with, before, or after administration of the engineered cells.

In general, the following vaccine compositions may be used as a medicament to prime T-cell responses within a subject.

The invention in an aspect relates to a method of treating a disorder characterized by MAGE-A4 expression in a subject comprising administering to said subject a therapeutically effective dose regime of a pharmaceutical composition consisting of a soluble protein that is a hybrid of the variable domains of a TCR as disclosed and/or defined herein and an activation domain, and where the pharmaceutical composition is administered in combination with a vaccine as discussed in the following.

In general, the following vaccine compositions may be used as medicaments to prime and support T-cell responses within a subject:
a) Vaccine composition contains sequence(s), designed to enable either or both of MHC class I and class II presentation of the same target epitope, and hence stimulation of therapeutic CD8 cells as well as helper CD4 cells against the same target, respectively. Such vaccine compositions may be delivered by RNA, DNA or peptide compositions, including liponanoparticles, in vivo electroporation, incorporation into viral particles, and antibody-mediated trafficking amongst other options, It is appreciated by those skilled in the art that different formulations and designs of vaccines can be optimised to balance and direct MHC class I vs class II responses, that different routes of administration can be exploited to balance responses, that different timings of prime/boost strategy can be exploited alongside TCR cell therapy, and that different immune-cell tropisms can be designed and exploited to maximise effects.

Presentation of MHC class I epitopes, including on professional APCs, would be expected to support direct expansion of therapeutic cells, which would be beneficial for directing the initial extent of T cell engraftment and expansion. Such an effect may be important in prophylactic or adjuvant settings (i.e., wherein a patient receives an additional treatment for the disease, such as e.g. chemotherapy or other cancer treatments), where tumour cell levels may be low as compared to a primary or metastatic setting.

Presentation of MHC class II epitopes on professional APCs would be expected to support the expansion of CD4⁺ T cells directed to the same target epitope, which would be expected to be a key mechanism. CD4⁺ T cells are known to promote antitumor immunity by numerous mechanisms including enhancing antigen presentation, co-stimulation, T cell homing, T cell activation, and effector function (Nelde et al, 2021). These effects are mediated at sites of T cell priming and at the tumor microenvironment (Melssen and Slingluff, 2017). CD4⁺ T cells harbor a crucial role for effective antitumor immunity and could further improve and sustain T cell reactivity (Bijker et al, 2007) and tumor clearance (Zwaveling et al, 2002). CD4⁺ T helper cells induce and intensify more permanent tumor immune control (Spitzer et al, 2017), epitope spreading (Hu et al, 2014), CD8⁺ T cell expansion and survival (Giuntoli et al 2002), as well as tumor immune cell infiltration (Bos and Sherman 2010). In addition, CD4⁺ T cells also exhibit direct antitumor effector function (Quezda et al, 2010).

b) Vaccine contains sequence(s), delivered by RNA, DNA or peptide compositions (including liponanoparticles, in vivo electroporation, incorporation into viral particles, and antibody-mediated trafficking amongst other options), designed to enable either or both of MHC class I and class II presentation of the additional target epitopes, and hence stimulation of therapeutic CD8⁺ cells as well as helper CD4⁺ cells against complementary targets. For example, a vaccine encoding patient-tumour specific neoantigens, or other antigens known to be over-expressed in the specific tumour type of interest, may be used to support a targeted T cell therapy.

Conceptually, such a vaccine in the form of, for example, a polyvalent mRNA expression construct formulated in an LNP particle, could be administered both prior to, or alternatively subsequent to, a TCR-T, using a prime-boost schedule.

c) Further designs and administration schedules using vaccines that encode both tumour-specific neoantigens as well as MAGE-A4 can be administered.

### Experimental section

The present invention is further illustrated by the following non-limiting experiments.

### Figure legends

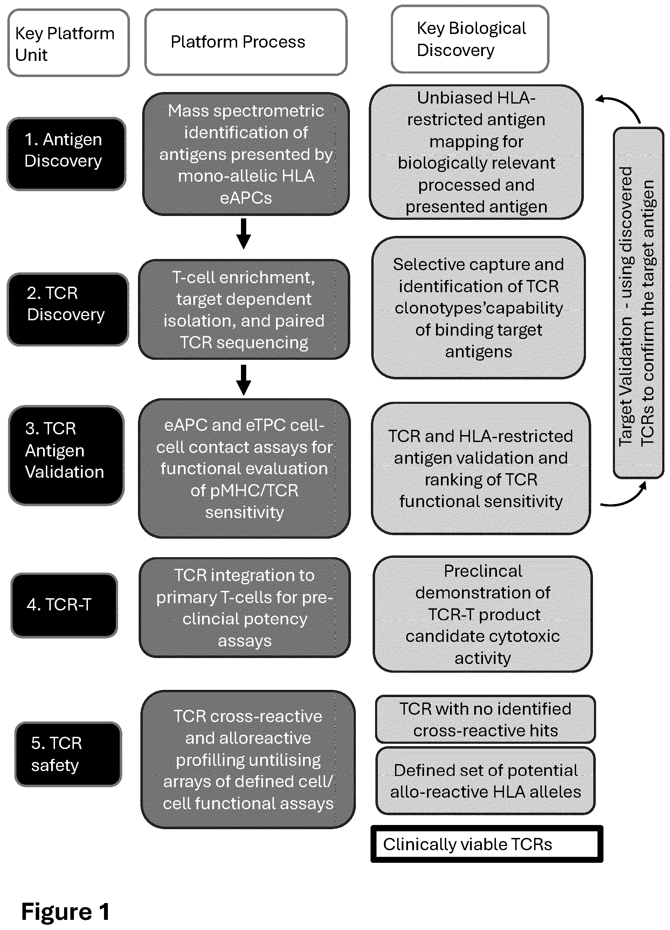

***Figure 1******: Summary of a TCR discovery platform focused on delivering clinically safe TCRs***
   The figure summarizes the Key Platform Units (left column) and the platform processes (middle column) that are used for discovery of clinically viable TCRs. A key biological discovery achieved at each step is shown (right column).
   **Step 1.** Antigen Discovery - Unbiased mass spectrometry (MS) is used for identification of peptides presented by mono-allelic HLA antigen presenting cells (eAPCs). This approach allows unbiased HLA-restricted mapping for discovery of biologically relevant, processed and presented peptides.
   **Step 2.** TCR discovery - The antigen identified in Step 1 is used for T cell enrichment, target-dependent isolation, and paired TCR sequencing, allowing for selective capture of TCR clonotypes and identification of TCR clonotypes' capability of binding target peptides.
   **Step 3.** TCR antigen validation - eAPC and (engineered TCR-presenting cell) eTPC cell contact assays are used for functional evaluation of pHLA/ TCR sensitivity. TCR and HLA-restricted peptides are validated and the TCRs are ranked based on functional sensitivity in defined cell-cell functional contact assays. TCRs with low sensitivity are removed from further analysis. The discovered TCRs can in turn be used to validate the target antigen presented by the eAPC lines as well as in orthogonal cancer cell lines.
   **Step 4.** TCR-T - Candidate TCRs are integrated into primary T cells to create TCR-Ts and TCR-T potency assays are performed, demonstrating that the TCR-Ts are functional and that they possess selective cytotoxic activity.
   **Step 5.** TCR safety assessment- this step in the TCR discovery platform ensures that only de-risked, clinically viable TCRs progress to the clinic. Cross-reactive epitopes within the human genome are predicted from fingerprint profiles of the TCRs using a computer algorithm. The identified cross-reactive epitopes (hits) are then presented on monoallelic eAPCs and defined cell-cell functional contact assays are performed. TCRs with no identified cross-reactive hits are selected and a defined set of potential allo-reactive HLA alleles is determined by screening against a library of monoallelic HLA-expressing cell lines covering 86 most common HLA haplotypes. All the above steps ensure that the end-products of the TCR discovery platform are clinically viable TCRs.

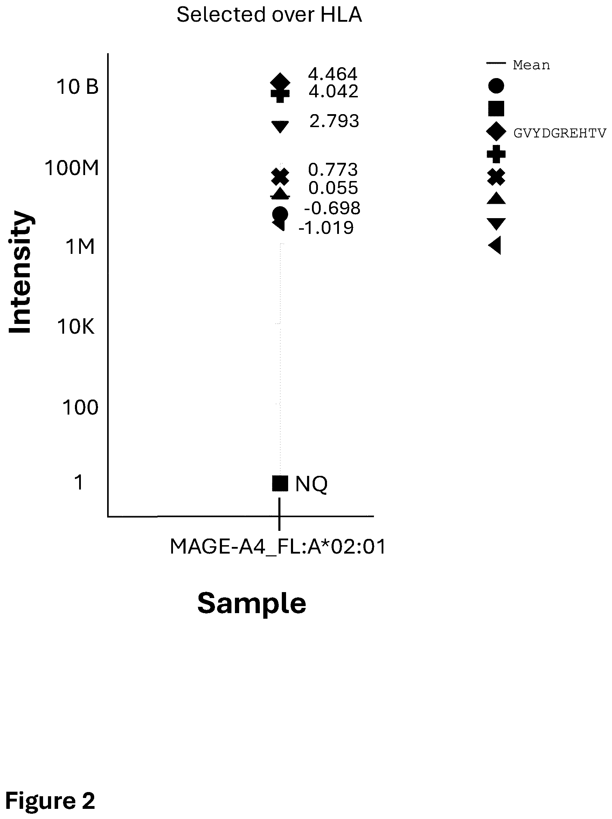

***Figure 2******: Detection of MAGE-A4 peptides***
   The mean intensities of peptides released from affinity purified A*02:01 pHLA complexes, as measured by unbiased mass spectrometry on a Lumos instrument, are shown, with z-scores relative to the mean (-) of all observations in the sample shown for specific observations of peptides derived from MAGE-A4. MAGE-A4_FL: A*02:01 monoallelic eAPCs expressing a full-length version of the MAGE-A4 protein (SEQ ID NO: 2). Intensity is plotted on Y-axis, against the sample on X-axis.

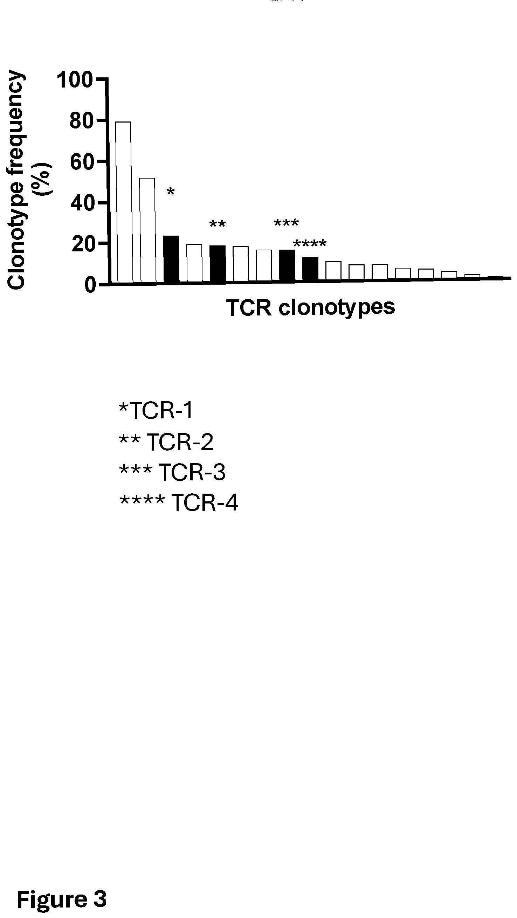

***Figure 3******: Selective expansion of TCRs specific for MAGE-A4.***
   Frequency distribution of TCR clonotypes enriched from MAGE-A4-stimulated expansions, using tetramers specific for MAGE-A4. MAGE-A4 TCR clonotypes (X axis) (n=17) are plotted against the percentage frequency for each particular clonotype observed (Y-axis). Clonotypes which were counted at least 3 times were considered enriched. TCRs are shown as black bars, * TCR-1, ** TCR-2,***TCR-3 and ****TCR-4 and have been marked retrospectively.

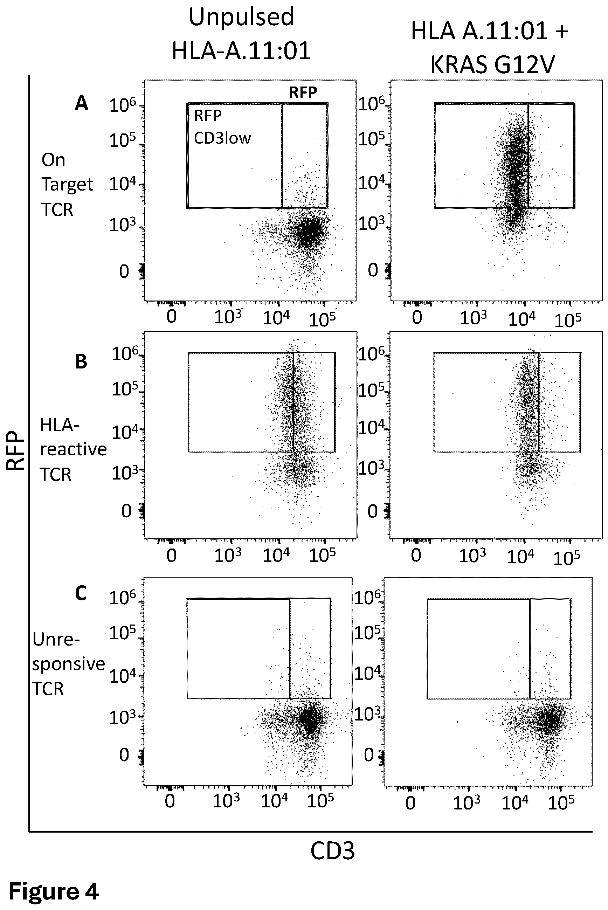

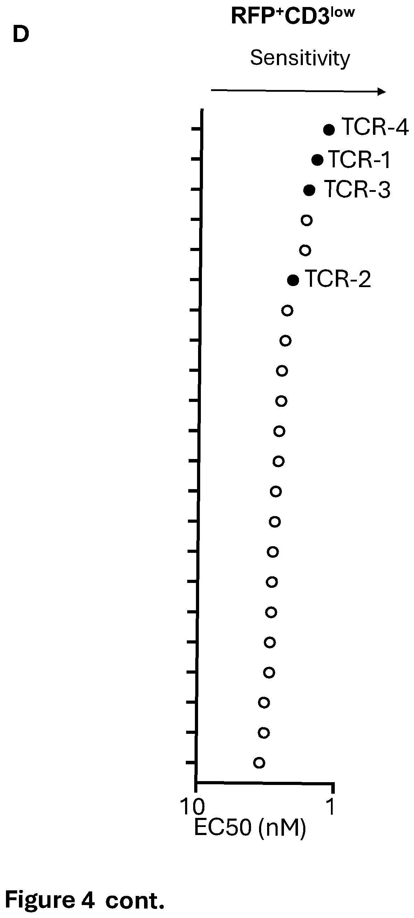

***Figure 4******: Functional evaluation of sensitivity of identified MAGE-A4-sensitive TCRs*** Sensitivity of the identified MAGE-A4 specific TCRs was functionally evaluated in eTPC-eAPC cell co-culture assays. Briefly, engineered TCR presenting cell (eTPC) lines as described in WO 2018/083317 were generated to present the MAGE-A4 TCRs (eTPC-t) and were co-cultured with engineered antigen presenting cells (eAPC) expressing monoallelic HLA-A*02:01 loaded with a concentration range of the MAGE-A4 peptide. Sensitivity was measured by detection of the RFP (RFP⁺) signal in combination with CD3 decrease (CD3^{low}) as described in Materials and Methods. Figures 4 A-C show flow panel validation and the gating strategy used for detection of activated RFP⁺CD3^{low} cells. **A)** Shows a representative of an "on target MAGE-A4-specific TCR" unstimulated with MAGE-A4 (left panel) or stimulated with MAGE-A4 (right panel). eTPC cells co-cultured with monoallelic eAPC without the MAGE-A4 peptide loaded (i.e. unpulsed) eAPC HLA-A*02:01 cells were used to define the flow gates that capture activated cells i.e., those that showed low CD3 staining with the CD3 antibody and positive RFP signal. Therefore, in panel A, the eTPC cells were not activated and are shown below the set gates, they are RFP negative (RFP⁻) except for some cells with autofluorescence, and CD3^{high} (left panel). Following co-culture of pulsed eAPC HLA-A*02:01 with the MAGE-A4 peptide and the eTPCs, the eTPC cells were activated which was observed by increase in RFP⁺, and decrease in CD3 (CD3^{low}) (right panel) and therefore most of the cell population was found within the gates set for defining the RFP⁺CD3^{low} population. **B)** A TCR that reacts with the HLA allele presented on the eAPCs (i.e., HLA-A*02:01) without the target peptide is a representative of a "HLA-reactive TCR". The RFP/CD3 profile does not change upon stimulation with the MAGE-A4 (right panel), it is always RFP⁺CD3^{low}. **C)** Shown is a representative of an "unresponsive TCR", (RFP⁻CD3^{high}) in both unstimulated (left panel) and stimulated cells (right panel). **D)** Flow cytometric data was used to construct sigmoidal curves from which the half maximal effective concentration (EC50) values were obtained. The sensitivity of the TCRs was based on the EC50 values. **D)** shows EC50 (nM) distribution obtained from the RFP⁺CD3^{low} values of the MAGE-A4-responsive TCRs (n=17) (X-axis) plotted against each TCR. All MAGE-A4-responsive TCRs, TCR-1, TCR-2, TCR-3 and TCR-4, are marked by full circle, retrospectively.

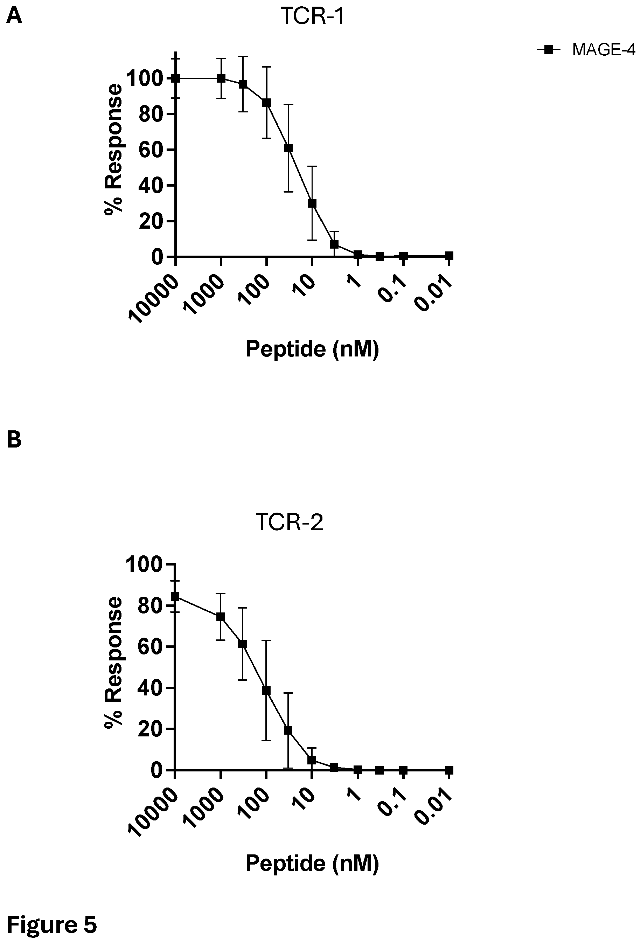

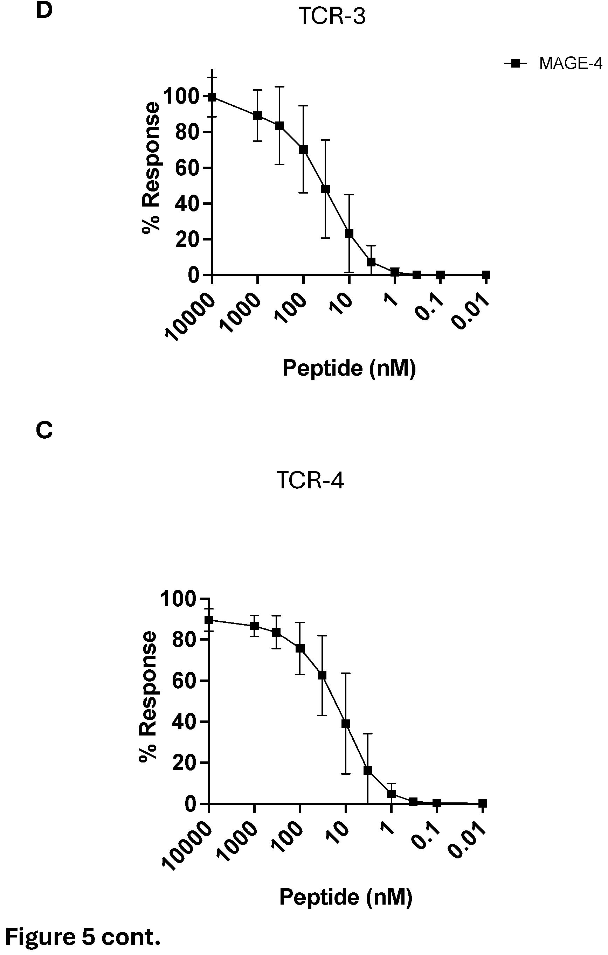

***Figure 5******: Functional evaluation of sensitivity and specificity of the MAGE-A4 TCRs pulsed with target peptides***
   In further examination of the TCR sensitivity, the ability of the MAGE-A4-specific TCRs to specifically recognize and bind MAGE-A4 peptides was analyzed. The figure shows representative sigmoidal curve profiles of the four selected MAGE-A4-specific TCRs. eTPCs expressing the TCR-1 (**A**), TCR-2 (**B**), TCR-3 (**C**) and TCR-4 (**D**) were co-cultured with monoallelic HLA-A*02:01 eAPCs- pulsed with a titration range of MAGE-A4 peptide. Percentage response (Y-Axis) of activation, as measured by RFP⁺ CD3^{low}, is plotted against the peptide concentration in nM (X-axis). All four TCRs recognized MAGE-A4 peptide with high specificity and sensitivity.

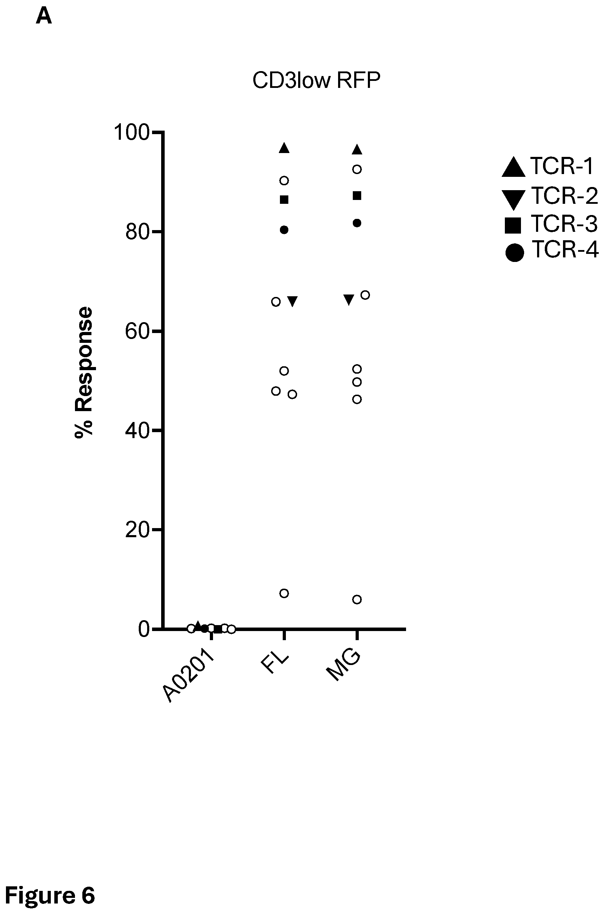

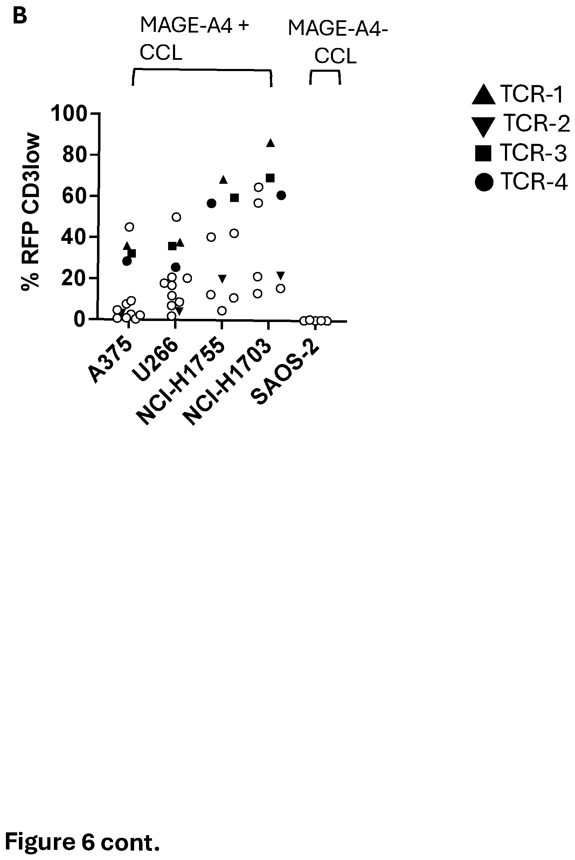

***Figure 6******: Evaluation of MAGE-A4-responsive TCRs to constitutively expressed target peptides***
   To further validate the interaction between the MAGE-A4-sensitive TCRs and their target peptides, we utilized the monoallelic eAPCs that constitutively express the target peptides and measured the response in the eTPCs expressing the MAGE-A4-specific TCRs. **A)** Response of MAGE-A4-responsive TCR-ts (n=17) co-cultured with the monoallelic HLA-A*02:01 eAPCs or the monoallelic HLA-A*02:01 eAPCs constitutively expressing the MAGE-A4 peptide as a minigene (MG) (SEQ ID NO: 3) or a full length (FL) (SEQ ID NO: 4) construct. Percentage response was measured by RFP⁺CD3^{low} signal. MAGE-A4 specific TCRs are shown as triangle (TCR-1), upside down triangle (TCR-2), square (TCR-3) and circle (TCR-4). **B**) % RFP⁺CD3^{low} response of MAGE-A4-responsive TCR-ts (n=17) co-cultured with cancer cell lines known to express MAGE-A4 via HLA-A*02:01 (A375, U266, NCI-H1703) and compared to a cell line which does not express MAGE-A4 (SAOS-2).

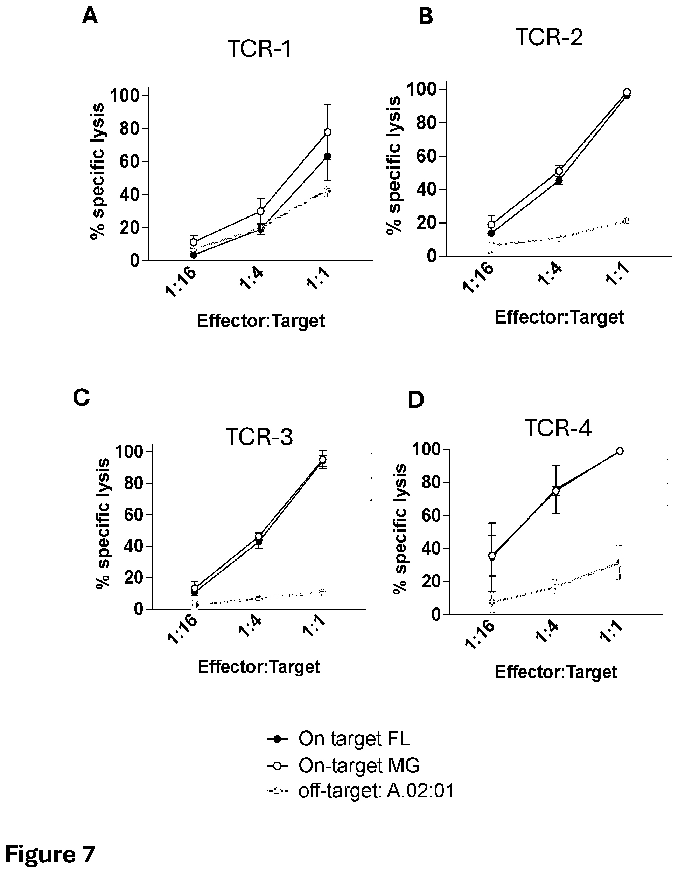

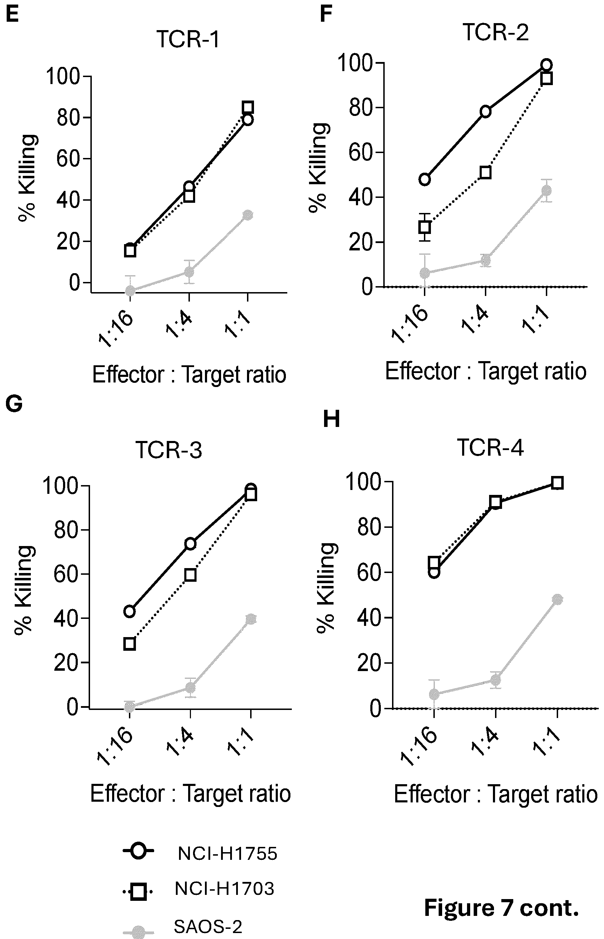

***Figure 7******: MAGE-A4-specific TCRs mediate potent T cell killing of cells presenting the MAGE-A4 peptide via HLA-A*02:01***
   Cytotoxic lysis mediated by the MAGE-A4-sensitive TCRs, (**A-D**) MAGE-A4 FL full circle (SEQ ID NO: 4), MAGE-A4 MG open circle (SEQ ID NO: 3) compared to off target HLA-A*02:01 Grey circle quantified as loss of luciferase activity after 24 hour co-culture with target eAPC-pa cells. Target cells are monoallelic eAPC-pa constitutively expressing HLA-A*02.01 in combination with MAGE-A4 full length (FL) (SEQ ID NO: 4) peptide or eAPCs expressing HLA-A*02:01_MAGE-A4 minigene (MG) (SEQ ID NO: 3) or HLA-A*02:01 only eAPCs (used as off-target control). Y-axis shows % specific lysis, while X-axis shows the effector (TCR-T) to Target ratios. **E-H)** Cytotoxic activity of TCR-T cells expressing the TCRs (TCR-T^{TCR-1}, TCR-T^{TCR-2}, TCR-T^{TCR-3}, TCR-T^{TCR-4}) against a panel of cancer cell lines expressing MAGE-A4 in the HLA-A*02:01 (NCI-H1755, H1703 and SAO), as determined by Luciferase Assay. Cancer cell line not expressing MAGE-A4 (SAOS-2) were used as controls. The Y-axis shows % specific lysis, while X-axis shows the effector (TCR-T^{TCR-1}, TCR-T^{TCR-2}, TCR-T^{TCR-3}, TCR-T^{TCR-4}) to Target cell ratios. All test conditions were prepared in duplicates. Percentage killing = 100 - ((RLUtest condition)/ (RLUtarget only)*100); RLU = relative luminescence units.

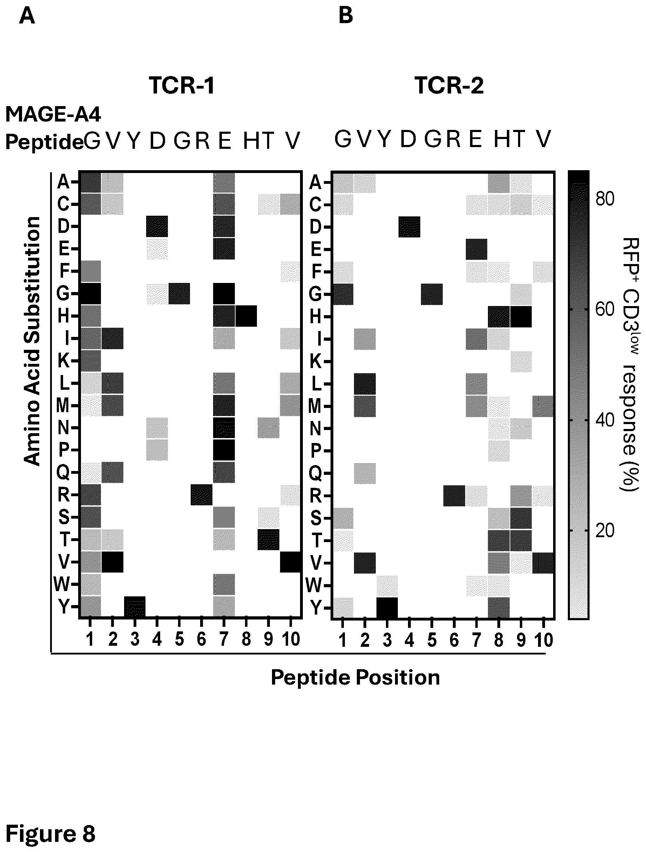

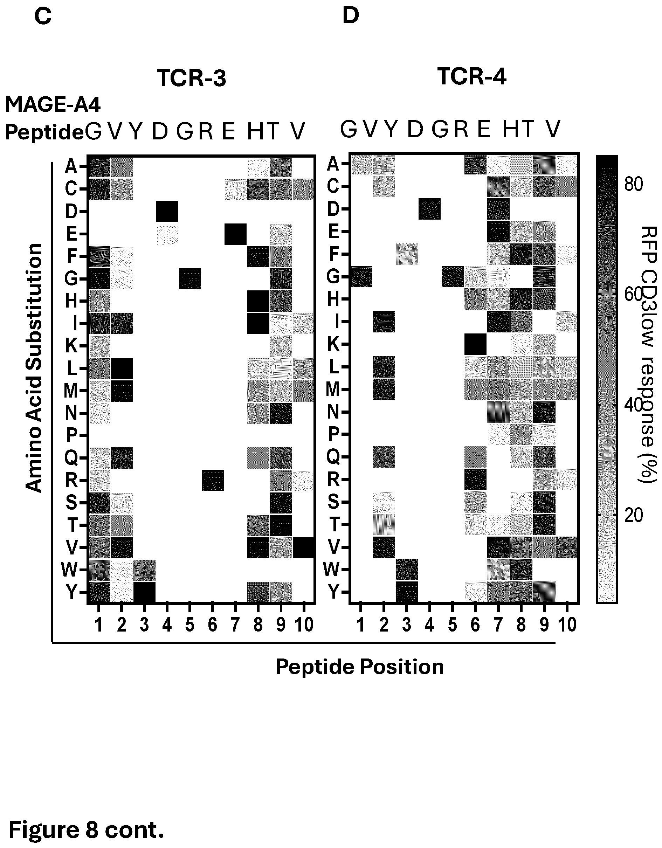

***Figure 8******: Fingerprinting profile of MAGE-A4-sensitive TCRs***
   Fingerprinting profile of the MAGE-A4-sensitive TCRs. eTPC-t cell lines expressing the MAGE-A4- sensitive TCRs, were stimulated with a library of monoallelic eAPCs stably expressing HLA-A*02:01 as well as the MAGE-A4 peptide (eAPC-pa epitope mutagenesis library). Each cell line expressed a point mutation in a certain position. The Y-axis represents the amino acid substitution and the X-axis the peptide position where the substitution has been introduced. Activation of the eTPC-ts is measured by RFP⁺CD3^{low} signal. **A)** Peptide scan of MAGE-A4- sensitive TCR, TCR-1; **B)** Peptide scan of MAGE-A4- sensitive TCR-2; **C)** Peptide scan of MAGE-A4- sensitive TCR, TCR-3; **D)** Peptide scan of MAGE-A4-sensitive TCR-4. The darker the colour, the higher the % response due to that particular amino acid substitution in the peptide sequence.

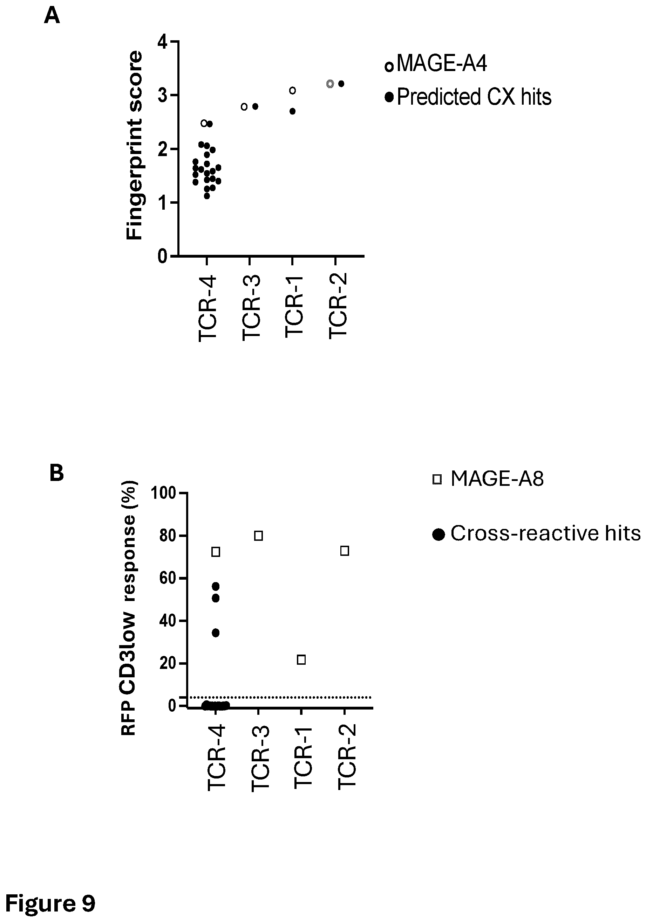

***Figure 9******: Cross-reactive profiling of the MAGE-A4- sensitive TCRs***
   The fingerprint profiles from Figure 8 were analysed by a computer algorithm that generates a fingerprint score which when screened against the whole human proteome allows the prediction of cross-reactive hits (CX). **A)** Predicted cross-reactive hits for the MAGE-A4-sensitive TCRs, TCR-1, TCR-2, TCR-3 and TCR-4. Based on the Fingerprint score (Y-axis), many cross-reactive hits were predicted for TCR-4, and only one cross-reactive hit was predicted for TCR-3, TCR-1 and TCR-2. **B)** The sequences of the predicted cross-reactive hits were subsequently used to generate MG constructs that were inserted into the HLA-A*02:01 presenting cells (eAPC-pa Cross-reactive library). These cells were then contacted with the eTPC cells expressing the TCRs (eTPC-t), TCR-1, TCR-2, TCR-3 and TCR-4, for 18 hours. The response of the eTPC-ts was analysed by flow cytometry by measuring RFP⁺CD3^{low} signals. TCR-4 which had many hits predicted showed 4 cross-reactive hits in the cell-cell cross-reactive contact assay. TCR-2, TCR-1 and TCR-3 had one cross-reactive hit each in the functional cell-cell cross-reactive assay. The cross-reactive hit was MAGE-A8, which is also a cancer testis antigen. TCRs that showed response in RFP⁺CD3^{low} signal above the 4% threshold in the cell-cell contact assays were considered as cross-reactive and therefore based on these results they were considered as unsafe for use in the clinic.

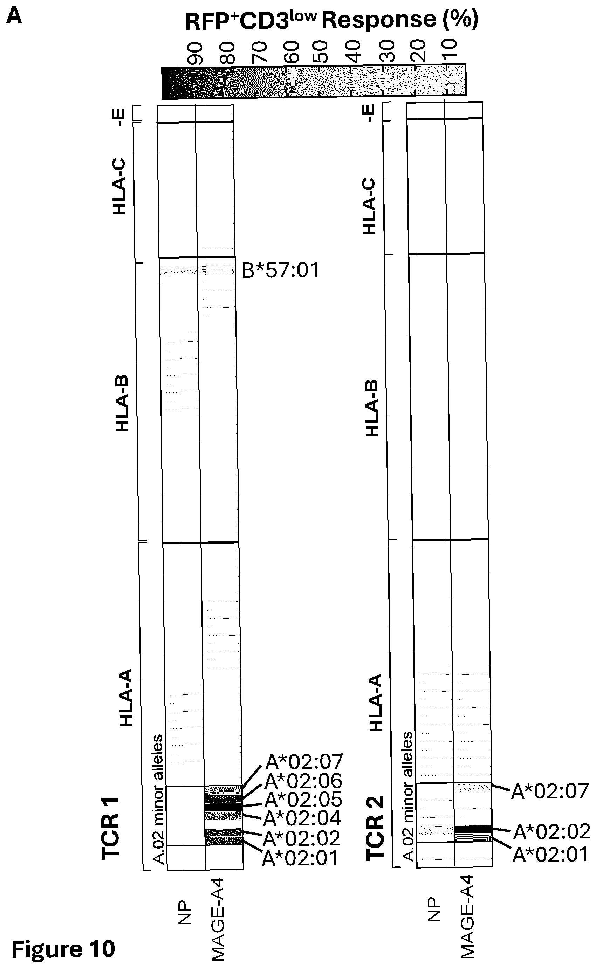

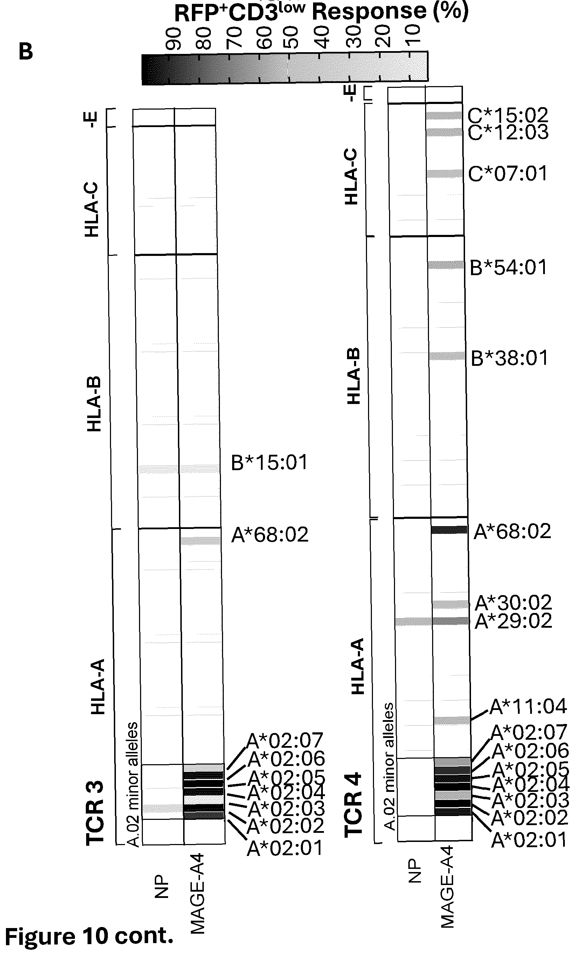

***Figure 10******: Allo- and para-reactivity profiles of the MAGE-A4- sensitive TCRs***
   To assess the alloreactivity of the TCRs, functional cell-cell contact assays were applied again, wherein the responder cell lines (eTPC-t expressing TCR-1, TCR-2, TCR-3 or TCR-4) were co-cultured with a library of monoallelic HLA-expressing cell lines (eAPC-p alloreactive library) covering the most common haplotypes. 86 different HLA alleles were tested. The read-out of the interaction was TCR-activation, i.e., flow cytometric analysis of RFP⁺CD3^{low} signal. Para-reactivity was assessed by pulsing the library with MAGE-A4 peptide. **A**) Alloreactivity and para- reactivity profiles TCR-1 (left panel) and TCR-2 (right panel). Y-axis shows the HLA alleles tested, X-axis is the RFP⁺CD3^{low} signal in the absence (NP= no peptide) or presence of MAGE-A4. TCR-1 showed alloreactivity to HLA-B*57:01 and para-reactivity when the peptide was added to members of the HLA-A*02 group (01, 02, 04, 05, 06 and 07) and weakly para-reactive with HLA-B*57:01. TCR-2 showed some alloreactivity to HLA-A*02:02 and para-reactivity to members of the HLA-A*02 group (01, 02 and 07). **B**) Alloreactivity and para- reactivity profiles TCR-3 (left panel) and TCR-4 (right panel). Y-axis shows the HLA alleles tested, X-axis is the RFP⁺CD3^{low} signal in the absence (NP= no peptide) or presence (MAGE-A4) of MAGE-A4. TCR-3 showed alloreactivity to HLA-A*02:02 and HLA-A*15:01, and in the presence of MAGE-A4 peptide, signal was detected from members of the HLA-A*02 group (01, 02, 03, 04, 05, 06 and 07) and from HLA-A*68:02 and HLA-B*15:01. TCR-4 showed alloreactivity to HLA-A*29:02. In the presence of the peptide, signal was detected from HLA-A*02 group (01, 02, 03, 04, 05, 06 and 07), HLA-A*11:04, HLA-A*29:02, HLA-A*30:02, HLA-A*68:02, HLA-B*38:01, HLA-B*54:01 and HLA-C*07:01, HLA-C*12:03 and HLA-C*15:02.

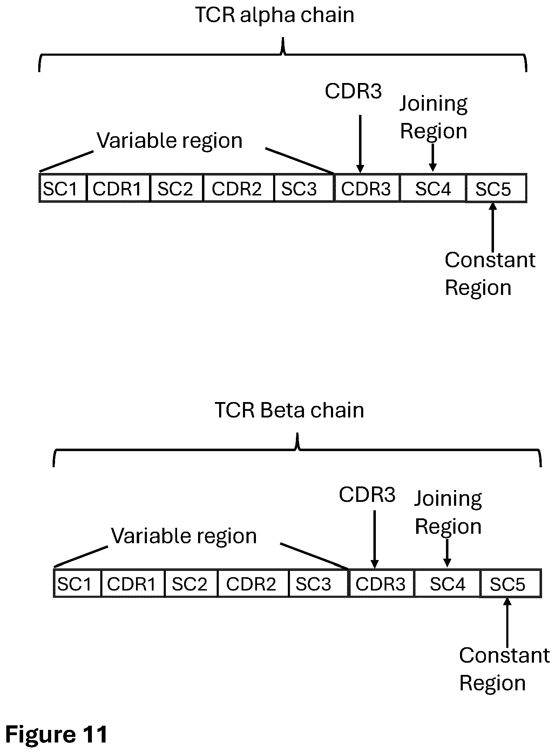

***Figure 11******. Schematic for alignment of scaffold nomenclature used in the specification with TCR chain structure.***
   SC = scaffold, CDR = complementary determining region. SC1, CDR1, SC2, and CDR2 together may be referred together as the variable region, CDR3 is referred to as CDR3, SC4 may be referred to as joining region, and SC5 may be referred to as constant region. The use of the terms "alpha" and "beta" specify the relevant TCR chain.

### Materials and Methods

### Cell Engineering

### Generation of eAPC-p and eAPC-pa by recombinase-mediated cassette exchange (RMCE)

Generation of eAPC-p (eAPC expressing HLA) and eAPC-pa (eAPC expressing HLA and a antigenic molecule) lines was achieved by RMCE as described previously in WO2018-083316, using the following open reading frame (ORF) including the gene of interest (GOI). The cells of this specification were generated as described bellow. Briefly, ARH-77 HLA-null line was generated using vectors containing the heterospecific recombinase sites flanking a marker (described in Table 1), (SEQ ID NO: 79 and SEQ ID NO: 80). The monoallelic HLA line (eAPC-p) was constructed by RMCE by electroporation with a vector (SEQ ID NO: 81) containing HLA.A*02:01 as a GOI (in the HLA monoallelic library construction described in Example 5 other HLAs were used as GOI). After electroporation, cells were incubated in culture medium RPMI 1640 with Glutamax-I + 10% FBS (37°C, 5% CO2) for two days, before analysis. To generate eAPC-pa lines, eAPC-p were further electroporated with a cassette containing the antigen ORFs as GOI 2 (SEQ ID NO:2-4).

eAPC-pa cell lines were generated to be employed in subsequent cell : cell co-culture assays (also interchangeably referred to as cell : cell contact assays or responder assays throughout this specification). eAPC-pa lines produced were designed to express either the full length MAGE-A4 peptide (MAGE-A4_FL) or a shortened version of the MAGE-A4 peptide referred to as the Minigene (MAGE-A4_MG). The MG contains candidate epitope +/- up to 9 amino acids of flanking sequence. More specifically, MG and FL expressing cell lines were generated with Ubiquitin fusion degradation signal (UFD).

eAPC-pa cell lines were also used to generate the Epitope Mutagenesis Library wherein each eAPC-pa cell line expresses a mutant variant of a target peptide antigen (Example 5), or for producing the Cross-Reactive Library (Example 5) where each cell line expresses a putative cross-reactive antigen determined by the TCRs unique fingerprinting.

### Electroporation conditions

For each reaction, 4x10⁶ cells were electroporated in 500 ml RPMI 1640 with GlutaMAX^{™} using the Gene Pulser Xcell^{™} (Bio-Rad) with the following setting: Square Wave 285V, pulse length 12.5 ms and 2 pulses with 1s interval. The cells were electroporated with vectors according to Table 1. For eAPC-p, the DNA concentrations used for the gene of interest (GOI- HLA) encoding vector (SEQ ID NO: 81) was 57mg, 13mg for the Flp vector (CMVpro_FLPo_Sv40pA_V2) and 5mg for the reporter gene vector (pcDNA 3.1 turbo/pMax GFP). For eAPC-pa, eAPC-p lines were additionally electroporated with 57mg of a second GOI containing vector (SEQ ID NO: 2-4). Following electroporation, the cells were transferred to culture vessels and grown in RPMI 1640 with GlutaMAX^{™}+10% FBS (catalogue number 10500064, Gibco) and 37°C, 5% CO2.

**Table 1. Vectors used for DNA delivery by electroporation.**

| Vector | Description |
|---|---|
| F14-GOI-1-F15 (SEQ ID NO:79) | RMCE site 1 |
| FRT-GOI-2-F3 (SEQ ID NO:80) | RMCE site 2 |
| FRT-GOI-2-F3 (SEQ ID NO:81) | Vector carrying the HLA-A*02:01 ORF of the genetic sequence to be integrated |
| FRT-GOI-2-F3 (SEQ ID NO: 2-4) | Vector carrying the MAGE-A4 ORF of the genetic sequence to be integrated |
| CMVpro_FLPo_Sv40pA_V2 | Flp vector encoding an optimized FLIP recombinase |
| Described in WO2018-083316 | |
| pcDNA 3.1 turbo/pMax GFP | Vector encoding a reporter gene used to assess DNA delivery efficiency and as a selection marker of integration |
| Described in WO2018-083316 | |

### Sorting of polyclonal GFP-expressing cells

Cells electroporated with the GOI or the pcDNA 3.1 turbo/pMax GFP were sorted for transient GFP expression. Cells were harvested (3-4 M cells per electroporation), centrifuged at 200 x g for 5 min, and the supernatant removed. The cell pellet was washed once with DPBS 1X (Gibco) and resuspended in DPBS 1X +2%FBS (Gibco) before sorting in RPMI 1640 with Glutamax-I + 20% FBS +1% Antibiotic Antimycotic solution (cat. number 15240-062, Gibco) in Influx^{™} instrument (BD Biosciences). Cells positive for the reporter gene (GFP⁺) were sorted as a polyclonal population into 5 mL polypropylene round-bottom FACS tubes containing culture medium. Following sorting, the cell suspension was centrifuged at 200 x g for 5 min, and the supernatant removed. Depending on the number of sorted cells, cells were transferred into a suitable vessel containing appropriate volume of culture medium (RPMI 1640 medium with Glutamax-I +10% FBS (Gibco)), and grown at 37°C, 5% CO2.

### Single cell sorting based on stable GOI integration

To obtain a population of cells constitutively expressing the integrated protein or marker, cells were sorted 9 to 11 days after the first GFP⁺ selection. At that stage, the cells that have successfully integrated the GOI become negative for the pre-existing gene cassettes (BFP⁻ or c-Myc⁻ or RFP⁻), and positive for the integrating cassette (SBP⁺ and GOI (HLA)).

Next, about 0.5-2 million ARH-77 cells were centrifuged at 200 x g for 5 min, washed once with cold staining buffer (SB) consisting of DPBS 1X + 2%FBS (Gibco) and the supernatant was removed. The cell pellet was resuspended in staining solution (50 ml for 0.5-2 Million cells) containing the conjugated antibodies (summarised in Tables 6 and 8) in DPBS 1X+2% FBS and the samples were incubated in the dark for 30-45 min at 4°C. Cells were then washed with SB, resuspended in 300-600 ml of SB (depending on the cell number) and transferred to a clean FACS tube. Single cell sorting was achieved through standard cell sorting methodologies using the Influx^{™} instrument (BD Biosciences). For the sorting of single cells for monoclonal population generation, single cells displaying the phenotype of interest were deposited per well into 96-well plates, containing pre-warmed 200 ml of RPMI 1640 medium with Glutamax-I +20% FBS+1% Antibiotic Antimycotic solution and the cells were grown in the incubator at 37°C, 5% CO2. Two weeks after sorting, 50 ml of RPMI 1640 medium with Glutamax-I +10% FBS was added to each well. Monoclonal cell populations were used after 3 weeks.

### Single cell sorting of eAPC-p

In the eAPC-p (eAPC-HLA) expression of the analyte HLA (GOI) is detected through cell surface HLA staining. Depending on the availability of antibodies, a pan-HLA (i.e., HLA-ABC) can be used (Table 2).

**Table 2. Standard antibodies for eAPC-p staining for monoclonal sorting**

| HLA Class | Antibody | Cat number | Provider | Antibody amount (mg) | Total staining Volume (ml) |
|---|---|---|---|---|---|
| I | Anti-HLA-ABC Alexa-APC-Cy7 | 311425 | BioLegend | 0.6 | 50 |
| Staining buffer (SB) = DPBS1X+2%FBS | | | | | |

From eAPC-p, eAPC-pa populations can be derived using discrete (a single ORF delivered into an eAPC-p) or pool (mix of several ORF delivered into an eAPC-p population) processes. Cell populations are stained with the antibody panel shown below (Table 3).

**Table 3. Standard eAPC-pa staining for single cell sorting**

| Antibody | Cat number | Provider | For up to 3M cells | |
|---|---|---|---|---|
| | | | Antibody amount | Total staining Volume |
| Anti-SBP-Alexa-647 | sc-101595 AF647 | SantaCruz Biotech | 0.6 mg | 50ml |
| Anti-c-Myc-Alexa-488 | sc-40 AF488 | SantaCruz Biotech | 0.6 mg | |
| Staining buffer (SB) = DPBS1X+2%FBS | | | | |

Individual eAPC-pa were selected, and single cell sorted (monoclones) based on loss of signal of the pre-existing gene cassette (c-Myc and red fluorescent proten (RFP) (c-Myc/RFP⁻)) and gain of signal of the selection marker of integration Streptavidin-Binding Peptide SBP (SBP⁺).

### Phenotypic screening of monoclonal cell lines

A sample of 50,000 cells of the outgrown monoclonal population were harvested, centrifuged at 200 x g for 5 min. The cell pellet was washed once with cold staining buffer (SB) consisting of DPBS 1X + 2%FBS (Gibco), centrifuged at 200 x g for 5 min, and the supernatant removed. Cell pellets were resuspended in appropriate volume of staining solution (25 ml for 30-100,000 cells) containing the conjugated antibodies in DPBS 1X+2% FBS and incubated in the dark for 30-45 min at 4°C. Cells were washed with cold SB and resuspended in 15 ml of SB and analysed by flow cytometry using the iQue Cell Analyzer (Sartorious).

### eAPC-p

The individual eAPC-p monoclone populations were screened for the expression of the target HLA. Monoclones were stained with a anti-HLA conjugated antibody and analysed by flow cytometry. Depending on the aAPX (HLA) to be analysed and the instrument used, a panel of antibodies conjugated to different fluorochromes targeting different epitopes on the GOI (i.e., HLA Class I) is used (Table 4).

**Table 4. Antibodies for phenotypic characterisation of eAPC-p monoclone population.**

| $Staining buffer \left(SB\right) = DPBS 1 X + 2 % FBS$ | | | | | |
|---|---|---|---|---|---|
| HLA Class | Antibody | Cat number | Provider | Antibody amount (mg) | Total staining Volume (ml) |
| I | Anti-Human HLA-ABC PE-Cy7 | 561349 | BD Biosciences | 0.1 | 25 |
| | Anti-Human HLA-ABC PE-Cy5 | 555554 | BD Biosciences | 0.025 | 25 |
| | anti-Human HLA-ABC V450 | 561346 | BD Biosciences | 0.001 | 25 |
| | anti-Human HLA-C PE | 566372 | BD Biosciences | 0.1 | 25 |
| | anti-Human HLA-A2 PE-Cy7 | 561347 | BD Biosciences | 0.05 | 25 |
| | anti-Human HLA-A2 BV786 | 741008 | BD Biosciences | 0.001 | 25 |
| | Anti-c-Myc-Alexa-488 | sc-40 AF488 | SantaCruz Biotech | 0.6 ug | 25 |
| | anti-c-Myc-Alexa-405 | sc-40 AF405 | SantaCruz Biotech | 0.1 | 25 |
| | anti-SBP tag-Alexa-488 | sc-101595 AF488 | SantaCruz Biotech | 0.1 | 25 |

Monoclonal cell lines with an HLA⁺ fluoresce signal compared to the HLA^{null} eAPC cell line control demonstrate integration of the GOI and thus expression of their analyte aAPX (i.e., HLA-A*02:01), and are therefore validated as eAPC-p cell lines.

### eAPC-pa

The phenotypic characterisation of the eAPC-pa cells is based on the loss of fluorescence of the selection marker(s) in the receiver cassette (c-Myc/RFP⁻), and gain of the selection marker(s) of integration of the donor cassette (SBP⁺) determined by antibody staining. Eligible monoclones retain their HLA allele expression. The screening is performed by flow cytometry as described above. The following antibodies targeting different HLA alleles or analyte antigenic molecules, or antibodies conjugated to different fluorochromes can be used (Table 5).

**Table 5. Antibody staining panel for phenotypic characterisation of eAPC-pa**

| Antibody | Cat number | Provider | For up to 100 000 cells | |
|---|---|---|---|---|
| | | | Antibody amount (mg) | Total staining Volume (ml) |
| anti-SBP tag-Alexa-488 | sc-101595 AF488 | SantaCruz Biotech | 0.1 | 25 |
| Anti-c-Myc-Alexa-405 | sc-40 AF405 | SantaCruz Biotech | 0.1 | 25 |
| Anti-Human HLA-ABC PE-Cy5 | 561349 | BD Biosciences | 0.1 | 25 |
| Staining buffer (SB) = DPBS 1X+2%FBS | | | | |

eAPC-pa monoclone populations that show a strong fluorescent signal from the donor cassette, and a loss of fluorescent signal from the receiving cassette, compared to controls, demonstrate expression of analyte aAM selection marker and therefore inferred aAM expression. Co-expression of aAPX (i.e., HLA) and the aAM selection marker validates eAPC-pa lines.

Plots of c-Myc vs RFP showing loss of c-Myc/RFP signal in monoclonal population(s) in comparison to the parental eAPC-p, and plots of SBP vs HLA showing gain of the SBP cassette (while retaining the HLA signal), indicating a successful integration event.

To confirm the identity of the aAM-ORF encoded in eAPC-pa monoclones, such cell lines were subjected to genetic characterisation by sequencing the Barcode encoded within the 3' UTR of the aAM-ORF.

### Genotypic analysis of monoclonal cell lines

Genomic DNA was extracted using the QIAamp^{®} DNA Mini kit (cat num 51306, Qiagen), Quick-DNA 96 Plus kit (cat number D4070 D4071, Zymo Research) according to the manufacturer's instructions, and PCR was conducted using primers that target adjacent and internal regions of the HLA ORF or aAM-ORF, thereby selectively amplifying successful integration couple events. Comparison was made to an unmodified parental line, wherein the HLA or aAM-ORF is lacking.

PCR reactions were done using either purified genomic DNA (gDNA) or crude cell lysates. For crude cell lysates, 70,000-150,000 ARH-77 derived cells were harvested, pelleted and washed with of 1X DPBS. Cell pellets were resuspended in 20ml of low EDTA TE buffer, the cells were lysed by heating for 10min at 98°C, lysates are centrifuged at >800 x g for 5min and the PCR reaction master mix was prepared according to Table 6.

**Table 6. PCR reagents**

| Reagent | Per reaction (ml) | Cat Number | Provider |
|---|---|---|---|
| 5X Phusion HF buffer | 6 | F-549L | Thermo Fisher Scientific |
| dNTPs | 0.3 | 10083252 | Thermo Fisher Scientific |
| Phusion Hot Star II DNA polymerase (2U/ml) | 0.3 | F-549L | Thermo Fisher Scientific |
| Forward primer (100mM stock) | 0.15 | - | |
| Reverse primer (100mM stock) | 0.15 | - | |
| H₂O | 21.1 (22.1)* | 129114, 129117 | Qiagen |
| Cell sample | 2 (1)* | - | - |

| | | | |
|---|---|---|---|
| *Volumes to add when using purified gDNA. | | | |

PCR products were run on a 1% or 2% Agarose gel beside a 50 bp or 1 kb ladder, in 1X TAE buffer, at 120 V for 25-30 min. DNA was stained with 10.000 dilution of SYBR safe DNA gel stain (cat num S33102, Invitrogen Life Technologies) and gel image captured using the Fusion SL UV-camera (Vilber).

### HLA amplification

To validate the HLA expression, PCR amplicons were generated with two set of primers that primed on the AAVS1 genomic sequence distal to the region encoded by the homologous arms (Table 7) and using the PCR conditions described in Table 8.

**Table 7. PCR and sequencing primers for HLA Class / Barcode PCR**

| Region | Primer | Sequence | Expected Product size | Sequencing Primer |
|---|---|---|---|---|
| Barcode | Forward | CATCATCACCATCACCACTGAC SEQ ID NO: 65 | 196 bp | CAC CACTGACTATAGTCA SEQ ID NO: 67 |
| | Reverse | GAATCCTGACTCCTCGACTAG SEQ ID NO: 66 | | |

**Table 8. PCR cycler program for HLA Class /**

| Step | Temperature | Duration | Number of cycles |
|---|---|---|---|
| 1. Pre-heating | 98°C | Hold | 1 |
| 2. Initial Denaturation | 98°C | 30 sec | 1 |
| 3. Denaturation | 98°C | 10 sec | 1 |
| 4. Annealing | 64°C | 20 sec | |
| 5. Extension | 72°C | 20 sec | |
| 6. Go to step 3 | - | - | 34x (total 35 cycles) |
| 7. Final extension | 72°C | 5 min | 1 |
| 8. Cooling | 12°C | Hold | 1 |

### aAM-ORF amplification

To validate integration of the aAM-ORF in the engineered monoclones, PCR amplification of the aAM-ORF region utilising primers targeting the ORF-Full length or ORF-Minigene Barcodes (Table 9).

The ORF-Minigenes were designed with proteasome degradation signals (UFD) (Table 9). The PCR amplification conditions for the FL ORF are summarised in Table 10 and for the MGs in Table 11.

**Table 9. PCR and sequencing primers for ORF-Full length Barcode, and ORF-Minigene Barcode**

| Region | ORF | Tag | Primer | Sequence | Product size (bp) | Sequencing Primer |
|---|---|---|---|---|---|---|
| Barcode | Full length | N/A | Fwd | ATCTGGACGGCT TCGACTT SEQ ID NO:68 | 165 | GGCTTCGACTTC TGTAGC SEQ ID NO: 70 |
| | | N/A | Rev | ATCCGTATGGTG ACAAGACG SEQ ID NO: 69 | | |
| Barcode | Minigene | UFD | Fwd | TCGTCAAGACTC TGACTGGT SEQ ID NO: 71 | 450 | GTAAGACCATCA CCCTG SEQ ID NO: 73 |
| | | | Rev | GGAGCTACAGAA GTCGAAG SEQ ID NO: 72 | | |

**Table 10. PCR cycler conditions for ORF-full length (FL) barcode amplification**

| Step | Temperature | Duration | Number of cycles |
|---|---|---|---|
| 1. Pre-heating | 98°C | Hold | 1 |
| 2. Initial Denaturation | 98°C | 30 sec | 1 |
| 3. Denaturation | 98°C | 10 sec | 1 |
| 4. Annealing | 64°C | 20 sec | |
| 5. Extension | 72°C | 20 sec | |
| 6. Go to step 3 | - | - | 34x (total 35 cycles) |
| 7. Final extension | 72°C | 5 min | 1 |
| 8. Cooling | 12°C | Hold | 1 |

**Table 11. PCR cycler conditions for ORF-Minigene barcode amplification**

| Step | Temperature | Duration | Number of cycles |
|---|---|---|---|
| 1. Pre-heating | 98°C | Hold | 1 |
| 2. Initial Denaturation | 98°C | 30 sec | 1 |
| 3. Denaturation | 98°C | 10 sec | 1 |
| 4. Annealing | 64°C | 20 sec | |
| 5. Extension | 72°C | 40 sec | |
| 6. Go to step 3 | - | - | 34x (total 35 cycles) |
| 7. Final extension | 72°C | 5 min | 1 |
| 8. Cooling | 12°C | Hold | 1 |

Sequencing of the aAM-ORF and HLA amplicons is performed by a third party (Eurofins Genomics). Raw .ab1 files are downloaded and analysed using the Geneious Prime analysis software (2022.1.1, Java Version 11.0.14.1+1 (64 bit)). Sequence reads are annotated and inspected for parameters such as the Sequence %HQ, UGI Mean PHRED, UGI ambiguities and UGI mutations.

### Engineering eTPC-t cells presenting a full-length functional TCRαβ using bidirectional DNA delivery vectors.

### Electroporation of Jurkat, Clone E6-1

For each reaction, 5x10⁶ cells were electroporated in 500 ml CTS Opti-MEM I Reduced Serum Medium (Thermo Fisher Scientific) using the Gene Pulser Xcell^{™} (Bio-Rad) with the following setting: Square Wave 265V, pulse length 15 ms and 2 pulses with 1s interval.

For integration of the bidirectional donor vectors encoding the TRA and TRB ORFs cells were electroporated with 10ug of the vector encoding the Flp recombinase enzyme (CMVpro-Flp-sv40pA-V2) and 55 mg of the integrating vectors (i.e., TCR encoding vectors). After electroporation, cells were incubated and further expanded in culture medium RPMI 1640 with Glutamax-I (Life Technologies) + 10% FBS (37°C, 5% CO2) for 7 days prior to the T cell enrichment.

### Magnetic Cell Sorting of eTPC-t

Isolation of cells expressing the desired TCR was achieved by following a single round of Miltenyi TCR- Indirect-positive selection and using the MultiMACS Cell24 Separator Plus (Miltenyi Biotec) for simultaneous multisample separation. Engineered T cells were labeled with phycoerythrin (PE)-conjugated anti-TCRαβ antibody (Thermo Fisher Scientific, Cat.Number: 12-9986-42, Working Dilution:1/10) and incubated for 10 min. Cells were washed twice with pre-cooled stain buffer (Dulbecco's phosphate-buffered saline (DPBS) with 2% Foetal bovine serum (FBS)) and resuspended in stain buffer with anti-PE MicroBeads (Miltenyi Biotec, Cat.No:130-048-801, Working Dilution:1/4) according to the manufacturer's instructions and incubated for 15min. All incubations were performed at 4 °C and protected from light. To quantify the integration rate of the bidirectional TCR donor construct pre-enriched cells, flow through (negative fraction) and positive fraction were analyzed by flow cytometry using the iQue Screener Plus. Engineered T cells (eTPC-t) were cultured and outgrown for 7-10 days and were subsequently subjected to an additional phenotypic analysis prior to cryopreservation.

### Phenotypic analysis by Flow cytometry

For phenotypic analysis cell polyclones were assessed for TCR⁺/CD3⁺ expression and the loss of the pre-existing selection markers (BFP⁻/SBP⁻). The anti-TCRαβ antibody conjugated with PE (Thermo Fisher Scientific, Cat.No: 12-9955-41, 12-9955-42, Working Dilution:1/25), the anti-CD3 antibody conjugated with BV650 (BD Biosciences, Cat. Number: 564003, 563999, Working Dilution:1/50), and the anti-SBP antibody conjugated with AF488 (Santa Cruz Biotech, Cat.No: sc-101595 AF488, Working Dilution: 1/50) comprised the antibody panel used for the analysis. Cells were incubated at 4°C, for 20 min, washed twice with pre-cooled stain buffer DPBS containing 2% Foetal bovine serum (FBS) and subsequently resuspended in stain buffer to be analyzed using the iQue Screener Plus instrument. Pure populations of engineered T cells (eTPC-t) were cryopreserved, and cell aliquots were obtained for the mycoplasma test and gDNA sequencing to verify the integration of TRA/TRB at the right genomic locus.

### Genetic characterization by genomic DNA sequencing

Primers used to assess integration of the TRA-ORF, annealed to the TRA-C segment (Reverse primer SEQ ID NO: 75) and the EF1a promoter (Forward primer SEQ ID NO: 74) that is a pre- existing part of the genomic receiving sites. Primers used to assess integration of the TRB-ORF, annealed to the TRB-C segment (Reverse primer SEQ ID NO: 77) and the EF1a promoter (Forward primer SEQ ID NO: 74) that is a pre- existing part of the genomic receiving sites. PCR products were run on a 1% Agarose gel in 1XTAE buffer, using the PowerPac Basic (Bio-Rad), stained with 10,000 dilution of SYBR Safe and analyzed with Fusion SL (Vilber Lourmat).

Amplicon sequencing primers, (SEQ ID NO: 76) and (SEQ ID NO: 78) were used to amplify TRA-C and TRB-C segments to genotypically confirm the TRA/ TRB integrated ORFs.

**Table 12. PCR reaction for amplification of TRA or TRB.**

| PCR mix | µl per reaction |
|---|---|
| 5xPhusion HF buffer | |
| dNTPs (20mM) | 0.25 |
| Phusion HSII | 0.25 |
| Fwd 100uM | 0.15 |
| Rev 100mM | 0.15 |
| H20 | 18.2 |
| Cell sample / DNA | 1 |

**Table 13. PCR cycling reactions for amplification of TRA or TRB.**

| PCR conditions common for TRA/TRB | | | |
|---|---|---|---|
| Initial Denaturation | 98°C | 30sec | 1 cycle |
| Denaturation | 98°C | 10sec | 35 cycles |
| Annealing | 69°C | 20sec | |
| Extension | 72°C | 15sec | |
| Final Extension | 72°C | 5min | 1 cycle |
| Hold | 12°C | hold | |

### Engineering of luciferase expressing eAPC-pa lines using RMCE

eAPC target cell lines expressing HLA-A*02:01 and MAGE-A4 were generated using RMCE as described in earlier section. RMCE was further used to introduce Firefly luciferase into the eAPC line expressing HLA-A*02:01.

### Engineering of luciferase expressing eAPC-pa lines using directed endonuclease

A polycistronic construct expressing EF-1 alpha promotor-driven Firefly Luciferase and green flourescent protein (GFP) was used as homology-directed repair (HDR) template to introduce these markers into eAPC lines expressing HLA-A*02:01 and MAGE-A4 by directed endonuclease-mediated knock-in into the CCR5 locus. Firefly Luciferase and green flourescent protein (GFP) was separated by a P2A cleavage site in the HDR construct.

For nucleofection, 2 million cells were prepared in 20 µl Lonza SF buffer. Pre-incubated RNP and DNA mixture was added to cells and mixed gently. This mixture was then transferred into 16-well Nucleocuvette Strips (Lonza Bioscience) and pulsed with the code EN-138. For every 2 million cells, 40pmol RNP and 2ug plasmid DNA were used. After nucleofection, pre-warmed RPMI with 10% FBS was gently added to the cuvette and the cuvette was placed in a 37°C tissue culture incubator for 15 min for cell recovery. Monoclones of the engineered lines were generated by flourescence activated cell sorting (FACS) using GFP as the selection marker.

### Engineering of HLA-A*02:01 and Luciferase expressing cancer cell lines

VSV-G pseudotyped third-generation lentivirus purchased from Vectorbuilder was used to introduce HLA-A*02:01 ORF into commonly used cancer cell lines expressing MAGE-A4.

The lentiviral vector additionally contained Firefly Luciferase and GFP ORFs with the three ORFs separated by T2A and P2A self-cleavage sites and expression of all driven by the EF-1 alpha promoter.

50,000 cells per transduction were seeded the day before transduction. 1,000,000-5,000,000 p24 transduction units of virus-like particles (VLPs) were added per transduction followed by LentiBoost (Sirion Biotech) at a 100 fold dilution. Cells were incubated for 10 min at RT and spinoculated at 30 min and 800g at 32°C. Following spinoculation cells were placed in a tissue culture incubator. On the next day media was exchanged. Seven days after transduction, purity of cell lines was measured and the cells were sorted to monoclones or polyclones with a purity of >90% using FACS and GFP expression.

**Table 14. Summary of mammalian cell lines used throughout the specification**

| **Unmodified cell line** | **Source** | **Specification** |
|---|---|---|
| HEK 293 | DSMZ | ACC305 |
| NCI-H1755 | ATTC | CRL-5892 |
| NCI-H1703 | ATTC | CRL-5889 |
| SAOS-2 | DSMZ | ACC243 |
| ARH-77 | DSMZ | ACC512 |
| A375 | Merck | 88113005 |
| U-266 | FenicsBIO | CL-1272 |
| Jurkat clone E6.1 | Merck | 88042803-1VL |

### Primary Cells

Collection of healthy donor peripheral blood and leukapheresis products were undertaken with written consent, granted by the Swedish Ethical Review Authority. Peripheral blood mononuclear cells and primary human CD8⁺ T cells were isolated from apheresis product from healthy donors using CliniMACS CD8⁺ reagent according to the manufacturer's instructions (Cat. Number 170-076-703, Miltenyi Biotec).

### CD8⁺ T cell activation and nucleofection

Cryopreserved CD8⁺ T cells were recovered for 24 hours in RPMI-1640 medium with 10% FBS before activation. For activation, CD8⁺ T cells were plated at an initial concentration of 1 million cells/mL in AIM V (Cat. Number 11534536, Thermo Fisher Scientific) medium in presence of 5% serum replacement (SR) (Cat. Number A2596102, Thermo Fisher Scientific) and 100U/ml IL-2 (Cat. Number 130-097-748, Miltenyi Biotec). T Cell TransAct (Cat. Number 130-111-160, Miltenyi Biotec) was added to the culture at a 1:100 dilution. After 72 hour stimulation, activated CD8⁺ T cells were centrifuged and gently resuspended in P3 buffer with supplement, as part of the P3 Primary Cell 4D-Nucleofector^{™} X Kit (Cat. Number V4XP-3032, Lonza Bioscience) at 2 million cells per 20 µl. 30 pmol ribonucleoprotein (RNP) and 2 mg plasmid deoxynucleic acid (DNA) were pre-incubated and added into the suspension. This mixture was then transferred into 4D-Nucleofector cuvette (Cat. Number V4XP-3032, Lonza Bioscience) and pulsed with code EH115. After nucleofection, pre-warmed AIM V medium with 5% SR and 100U/ml IL-2 was gently added to the cuvette and the cuvette was placed in a 37°C tissue culture incubator for 15 min to allow for cell recovery. After recovery, the cells were transferred to prewarmed AIM V medium with 5%SR, 100U/ml IL-2 and 2 mM HDR enhancer. After overnight incubation, nucleofected cells were collected by centrifugation and replated in fresh AIM V medium with 5%SR, 100U/ml IL-2, without HDR enhancer to allow expansion. 7 days after nucleofection, CD34⁺ cells were enriched using CD34 MicroBead Kit according to the manufacturer's instructions (Cat. Number 130-046-702, Miltenyi Biotec).

### Identification of MAGE-A4 peptides via mass spectrometry

### Metal affinity chromatography

ARH-77 cells were cultured in RPMI supplemented with 10% FBS at 37°C and 5% CO2. Harvested cells were washed once with ice-cold PBS and lysed in ice-cold lysis buffer (150 mM Sodium chloride (NaCl), 1x PBS, 1% 3-[(3-cholamidopropyl)dimethylammonio]- 1-propanesulfonate (CHAPS), 5 mM Imidazole, 0,2 mM iodoacetamide, 5% glycerol, and 1x Halt protease inhibitor cocktail (Thermo Scientific)), vortexed and incubated at 4 °C for 20 minutes. Cleared lysate was mixed with HisPurTM nickel-nitriloacetic acid (Ni-NTA) Resin (Thermo Scientific) and rotated for 2 hours at 4°C. After removal of the lysate unbound fraction, the resin was washed twice with high salt buffer (250 mM NaCl, 1x PBS, 25 mM Imidazole) and twice with low salt buffer (50 mM NaCl, 1x PBS). Washed beads were harvested in low salt wash buffer and transferred to spin columns (Thermo Scientific). The bound fraction was eluted in 15% Acetonitrile and 0,2% TFA and ultrafiltered over 3kD Ultra Centrifugal Filter (Amicon). The peptide fraction was reduced and alkylated in a one-step reaction with 10 mM Tris(2-carboxyethyl)phosphine hydrochloride (TCEP), 50 mM chloroacetamide and in 100mM Triethylammonium bicarbonate buffer (TEAB) shacking for 30 minutes at 37°C. The sample was then immediately re-acidified in 5% FA and subjected to solid phase extraction on Hypersep Retain-CX columns (Thermo). The sorbent was activated with methanol (MeOH) and equilibrated with 0.2% TFA prior to sample loading. The column was later washed with MeOH and then water prior to elution of the absorbed peptide fraction in elution buffer (MeOH, 5% NH4OH). Samples were transferred to HPLC-glass vials, vacuum-dried and stored at -20°C prior to LC-MS/MS analysis.

### Mass spectrometry

Peptides were re-suspended in 10 mL Solvent (10% formic acid (FA), Milli-Q) prior LC-MS/MS analysis. Each sample was analyzed on a timsTOF Pro2 (Bruker, United States) connected to a nanoElute system (Bruker, United States), injecting 2 mL from each sample vial. The nanoElute system was equipped with an UHPLC analytical column (Aurora Ultimate nanoflow with CSI fitting; 25 cm x 75 mm ID, 1.7 mm C18; lonOpticks) heated to 50 °C, and connected to a CaptiveSpray (Bruker, United States) ion source.

Mobile phase buffers consisted of Solvent A (2% ACN, 0.1% FA, Milli-Q) and Solvent B (ACN, 0.1% FA). The peptides were eluted during a 55 min gradient with a flow rate set to 0.250 mL/min. The gradient started at 2% of solvent B, raised to 4% within 2 min, to 18% within 33 min, to 26% within 7 min, to 36% within 3 min, to 95% within 1 min, and maintained at 95% for 9 minutes. The nano spray voltage was set to 1.5 kV, with dry gas flow set to 3 L/min and 180 °C.

The mass spectrometer acquisition was configured to operate in a data dependent acquisition (DDA) mode, using trapped ion mobility spectrometry (TIMS) with the scan mode set to Parallel Accumulation-Serial Fragmentation (PASEF), high sensitivity detection and positive mode. The precursor scan range was set between m/z 100 and 1700. The TIMS 1/K0 values ranged from 0.7 and 1.42, with the ramp and accumulation times of 166 ms, and a duty cycle of 100%. The PASEF precursor region polygon left boundary was set at m/z 180 for low 1/K0 and m/z 1300 at max 1/K0, and the right boundary at m/z 1700 for both high and low 1/K0 (0.7 and 1.42). The quadrupole energy was set to 5.0 eV and low mass m/z 200. The collision cell energy was set to 10 eV and collision RF to 1500.0 Vpp.

### Data analysis

The MS acquisition data files were processed using PEAKS Online X (Bioinformatics Solutions Inc., Canada) using database search, De Novo sequencing, post-translational modifications (PTM) search, and SPIDER algorithms. The library search was preformed against a composite FASTA file including standard Human proteome library sequences and short sequences corresponding to SNP and indel events known to be present in ARH-77 and supplemented with the MAGE-A4 included in the experiment; a FASTA file with a list of common LC-MS/MS contaminants was included in the search. The precursor and fragment mass error tolerances were set to 15 ppm and 0.05 Da, respectively, and the peptide lengths ranged between 7 and 25 amino acids. The De Novo search parameters were set to be the same as the library search with the Average Local Confidence (ALC) set to 50%. The PTM search was set to detect all built-in modifications, and with De Novo ALC set to 15%. The SPIDER algorithm was employed to find mutations and sequence variants. The peptide false discovery rate (FDR) was set to 1% and the proteins -10LogP ³ 20 and proteins unique peptides ³ 1. The results from PEAKS Online X search were further analyzed using Bruker-provided and proprietary software (Python scripts) implementing the clustering methods of Andreatta et al (2017) so as to confirm concordance to expected pHLA repertoire characteristics to assess the quality of the immunopeptides identified.

### TCR Discovery

### Naive CD8⁺ T cell enrichment

Cryopreserved PBMCs from healthy HLA-A*02:01 donors were thawed and rested overnight at 10 Million cells/ mL in RPMI1640 with 2mM L-glutamine and 10%FBS. The next day, PBMCs were counted using the NucleoCounter NC-200 (ChemoMetec) and enriched using the EasySep^{™} Mouse Naïve CD8⁺ T Cell Isolation Kit (StemCell) following the manufacturer's instructions. Purity of the CD8 naive T cells was validated by flow cytometry using surface antibody staining against CD45R0, CD27, CD95, CD8 and CD4 and analysis on the cell analyzer BD LSRFortessa.

### Selective expansion of target-specific TCRs

Enriched naive CD8 T cells were co-cultured with engineered antigen presenting cells (eAPCs) constitutively expressing the HLA-A*02:01 at a ratio of 20:1 (CD8⁺ T cells: eAPC). In a round bottom 96 wells plate, 5000 naive CD8⁺ T cells were co-cultured with 250 eAPCs and 1mM MAGE-A4 peptide in 100mL of OpTmizer CTS supplemented with 2mM GlutaMax and 10% human serum for 7 days. 1 day and 7 day after the seeding, 50mL of interleukin 2 (IL-2) at 100 U/mL and 25 U/mL respectively was added to support expansion of specifically activated T cells by the MAGE-A4 peptide.

### Harvesting and sorting of MAGE-A4 specific T cells

14 days after expansion, cells were harvested and counted using the NucleoCounter NC-200. Cells were stained with A*02:01 MAGE-A4 MHC tetramer conjugated with phycoerythrin (produced in house, with minor modifications, according to method described previously https://tetramer.yerkes.emory.edu/support/protocols) for 30min at room temperature (RT). A*02:01 MAGE-A4 MHC tetramer-stained CD8⁺ T cells were magnetically enriched using anti-phycoerythrin (PE) Microbeads (Miltenyi) following the manufacturer's instructions. The enriched fraction was stained for extracellular markers against cMyc, SBP and CD8 to distinguish eAPC from CD8⁺ T cells and CD8⁺ A*02:01 MAGE-A4 MHC-PE+ cells were single cell sorted using FACS sorting.

### TCR selection

The identified unique TCRs from each human donor were extracted and counted. The absolute count and frequency of one TCR pair relative to the total number of pairs identified per donor was determined. A total count of more than 3 observations was considered to be an expanded clonotype.

### Peptide Pulse assay

### eTPC-t and eAPC-p cells harvesting

An aliquot of actively growing cultures of eTPC-t cells and eAPC-p cells (0.4-1.0 x 10⁶ cells/mL) was taken for cell counting using CountBright Absolute Counting Beads (Thermofisher) read on an iQue Screener Plus (Sartorius). The desired number of cells were harvested, washed once with complete media and resuspended at a concentration of 0.7M/mL in complete media (RPMI 1640 with L-glutamin and 10%FBS).

### Contacting eTPC-t and eAPC-p in an eTPC:eAPC system with exogenous antigenic molecules

Co-culture assays were performed in round bottoms 96 wells plate at a 1:2 eTPC-t to eAPC-p ratio. 100uL of eAPC-p (70,000 cells) were seeded together with 50uL of eTPC-t (35,000) and 50uL of peptide was added. Peptide concentration used ranged from 0 to 10 mM final. Cells were co-cultured for 18h at 37C before functional reading by flow cytometry.

### TORES² Plasmid production

### Vector library assembly and cloning

The construction of vectors described in the examples comprises a variety of methods well known to those skilled in the art, and specific reaction compositions are outlined in detail in WO2019/016175. The following key materials in table 15 were used in the described procedures:

**Table 15:**

| **Product** | **Supplier** | **Supplier Number** |
|---|---|---|
| Acc651 | New England BioLabs | R0599L |
| BbsI HF | New England BioLabs | R3539L |
| DH5alpha competent cells | Thermo Fisher Scientific | 18265017 |
| DNA clean and concentrator kit | Zymo Research | D4030 |
| EcoR1 | New England BioLabs | R3101S |
| Notl | New England BioLabs | R3189L |
| QIAamp DNA Mini kit | Qiagen | 51306 |
| QIAquick Gel Extraction kit | Qiagen | 28704 |
| Qiagen Plasmid Plus Midi kit | Qiagen | 12945 |
| T4 ligase | New England BioLabs | M0202L |
| T4 ligase buffer 10x | New England BioLabs | B0202S |
| Xbal | New England BioLabs | R0145S |
| Xhol | New England BioLabs | R0146S |

### Oligonucleotide duplex encoding CDR3 (odeCDR3) assembly

odeCDR3 were routinely assembled by annealing partially complementary single stranded oligonucleotides. A detailed description of reaction composition and conditions is provided in WO2019/016175. The following key materials in table 16 were used in the described procedures:

**Table 16**

| **Product** | **Supplier** | **Supplier Number** |
|---|---|---|
| T4 ligase buffer 10 x | New England BioLabs | B0202S |
| T4 PNK | New England BioLabs | M0201L |

### TCR reconstitution

Operation of a TORES2 to reconstitute full-length TCR ORFs is described in detail in WO2019/016175. The following key materials in table 17 were used in the described procedures:

**Table 17:**

| **Product** | **Supplier** | **Supplier Number** |
|---|---|---|
| BsaI-HF | New England BioLabs | R3535L |
| rCutSmart buffer 10 x | New England BioLabs | B6004S |
| DH5alpha competent cells | Thermo Fisher Scientific | 18265017 |
| NotI-HF | New England BioLabs | R3189L |
| QIAamp DNA Mini kit | Qiagen | 51306 |
| T4 Ligase | New England BioLabs | M0202L |
| T4 Ligase buffer 10 x | New England BioLabs | B0202S |

### Fluorescence activated cell sorting (FACS)

Single HLA-multimer positive CD8⁺ T cells were sorted by FACS for amplification and sequencing of TCR chains. This was achieved through standard cell sorting methodologies using a *BDInflux* instrument. Briefly, cells were stained with HLA-multimer reagent on ice for 10 mins, then with CD3 and CD8 antibodies as markers of CD8⁺ T cells. Cells with CD3+CD8+Multimer+ signal were sorted to PCR plate pre-loaded with 5 µL of nuclease-free water. Specimens were snap-frozen until subsequent processing. The following key materials in table 18 were used in the described procedures:

**Table 18:**

| **Product** | **Supplier** | **Supplier Number** |
|---|---|---|
| Anti-CD8 (clone RPA-T8) (BV510) | BD | 563256 |
| Anti-CD3 (clone SK7) (APC-H7) | BD | 560176 |

### Sequencing of TCR alpha and beta chains from single T cells

Singly FACS-sorted T cells were subjected to a two-step amplification process that entails a V-region specific primer collection for each TRA and TRB, followed by paired nested PCR reactions that create TRA and TRB amplicons for sequence analysis as described in WO2019/016175. This procedure is described previously (Han et. al. 2014). The following materials in table 19 were used in the described procedures:

**Table 19**

| **Product** | **Supplier** | **Supplier Number** |
|---|---|---|
| 2x Reaction Mix | Thermo Scientific | 12574035 |
| 5X Phusion HF Buffer | Thermo Fisher Scientific | F-549S |
| dNTPs | Thermo Fisher Scientific | 10297018 |
| Nuclease free water | Qiagen | 129114 |
| Phusion Hot Start II DNA Polymerase | Thermo Fisher Scientific | F-549S |
| SuperScript^{®} III One- Step RT-PCR System with Platinum^{®} Taq High Fidelity DNA Polymerase | Thermo Scientific | 12574035 |

### Responder assay (eAPC:eTPC co-culture assays)

This procedure was used to evaluate and validate the specificity and sensitivity of a TCR for an antigen presented on a defined HLA background, evaluate cross-reactivity, and/or define an alloreactivity profile for specific TCRs.

### Cell line preparation

Selected analyte eAPC-pa and eTPC-t were thawed at least 4 days before their planned responder assay setup to allow cell recovery and growth. Both the eAPC-pa and eTPC-t cells were maintained independently in log-phase with cell concentrations between the 0.2-1 Million/ ml in Roswell Park Memorial Institute (RPMI) 1640 medium (Gibco) supplemented with 10%FBS (Gibco).

### Assay set up and Cell Staining

eAPC-pa and eTPC-t cells were separately counted using iQue flow cytometer (Sartorius) and plated in 2:1 ratio (70,000 eAPC-pa and 35,000 eTPC-t cells per well) in 200 µl final volume in a 96 well round-bottom plates and incubated overnight (18 hours) at 37 °C, 5% CO₂, 90 % RH. Cell analysis was conducted through cell surface marker staining and flow cytometry analysis using the iQue instrument (Sartorious) using the following antibody panel (Table 20). The antibodies were added directly into the cell suspension in the 96-well round-bottom assay plate. After overnight incubation, 25 µl of the antibody mix was directly added to the culture plates, and cells were mixed using the microplate shaker at room temperature for 5 sec and incubated at 2-8°C for 40-60 min. Following the incubation, cells were centrifuged for 4 min at 400 x g and the supernatants were aspirated. The cell pellets were resuspended in 20 µl of 1.5X CellFix (4% Paraformaldehyde) solution and mixed at room temperature using microplate shaker 5 sec. Cells were incubated at 2-8 °C for 15 min before acquisition on the iQue cytometer.

**Table 20. The antibody staining panel for the eTPC-t. Antibody mix prepared in Staining Buffer consisting of 2% FBS (Gibco) in DPBS (Thermo Fisher Scientific)**

| Antibody | Catalogue number | Supplier |
|---|---|---|
| BD Horizon^{™} BV786 | 564159 | BD Biosciences |
| Mouse anti-Human | | |
| CD80 | | |
| BD Pharmigen^{™} | 557696 | BD Biosciences |
| Alexa Fluor 488 | | |
| Mouse Anti-Human | | |
| CD8 | | |
| BD OptiBuild^{™} | 740202 | BD Biosciences |
| BV510 Anti-Human | | |
| CD3 | | |

### Quality Control Measures

### eAPC-pa

The eAPC-pa were subjected to two quality control procedures: a) genotyping confirmation by direct Sanger sequencing and b) intracellular Flag staining. Genotyping was described in the cell engineering section.

### Intracellular FLAG staining

To confirm ORF minigene expression, proteasomal degradation was inhibited with addition of MG-132 (Cat number 474787, Merck), according to manufacturer's instructions and followed by intracellular FLAG staining. Following proteosome inhibition, eAPC-pa were stained with 25µl of Death Cell Marker (DCM) Aqua BV-510 dye (Zombie AquaTM Fixable Viability Kit, Cat num. 423102, BioLegend) diluted 1:100 with DPBS and incubated in dark while shaking for 20 mins at RT. Cells were washed with 250µl Stain buffer (DPBS +2%FBS), centrifuged at 400g for 4 minutes, and supernatant removed. Intracellular Flag staining was performed using the Cytofix/Cytoperm Fixation/Permeabilization solution kit (Cat num. 554714, BD Biosciences) according to manufacturer's instructions. For cellular permeabilization, cell pellets were resuspended in 100µl Cytofix/Cytoperm solution and incubated for 20 mins at 4°C. Washed twice with 250µl PermWash Buffer (diluted 1:10 with water) at 400g for 3 minutes. Supernatant was removed and cells were stained with anti-DYKDDDDK-PE (FLAG) antibody (Cat num 130-128-816, Miltenyi) for 30-60 minutes at 4°C in the dark. Following the incubation, the cells were washed twice with 250µl PermWash Buffer at 400g for 3 minutes. Cell pellets were resuspended in 20µl of Stain Buffer (DPBS +2%FBS) or 1% PFA and incubated for 10-15 min at RT and data was acquired on the iQue instrument.

### eTPC-t genotyping

The eTPC-t were subjected to quality control by genotyping confirmation using Sanger sequencing to confirm integration of the desired TCR chains. Described in detail in the Cell engineering section.

### Mutagenesis epitope libraries for fingerprinting

Mutagenesis epitope libraries for the MAGE-A4 peptide were created by substituting all possible naturally occurring single amino acids at each position within the MAGE-A4 (GVYDGREHTV) peptide, Table 21.

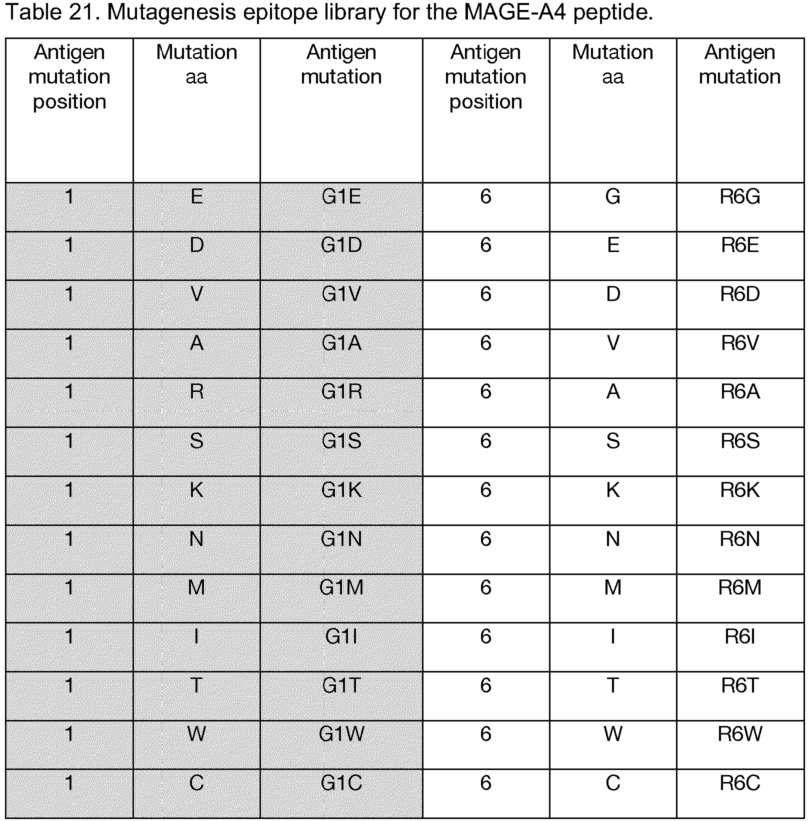

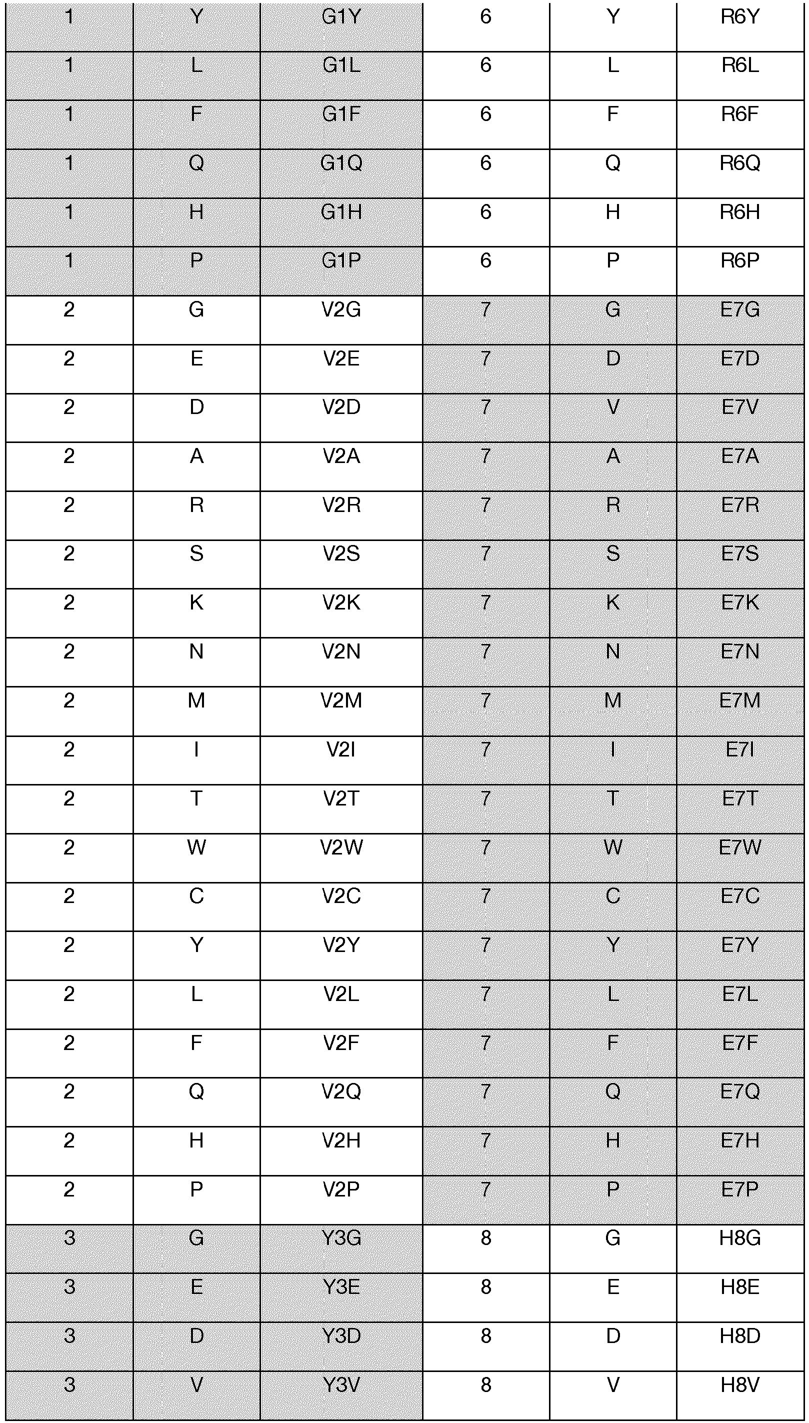

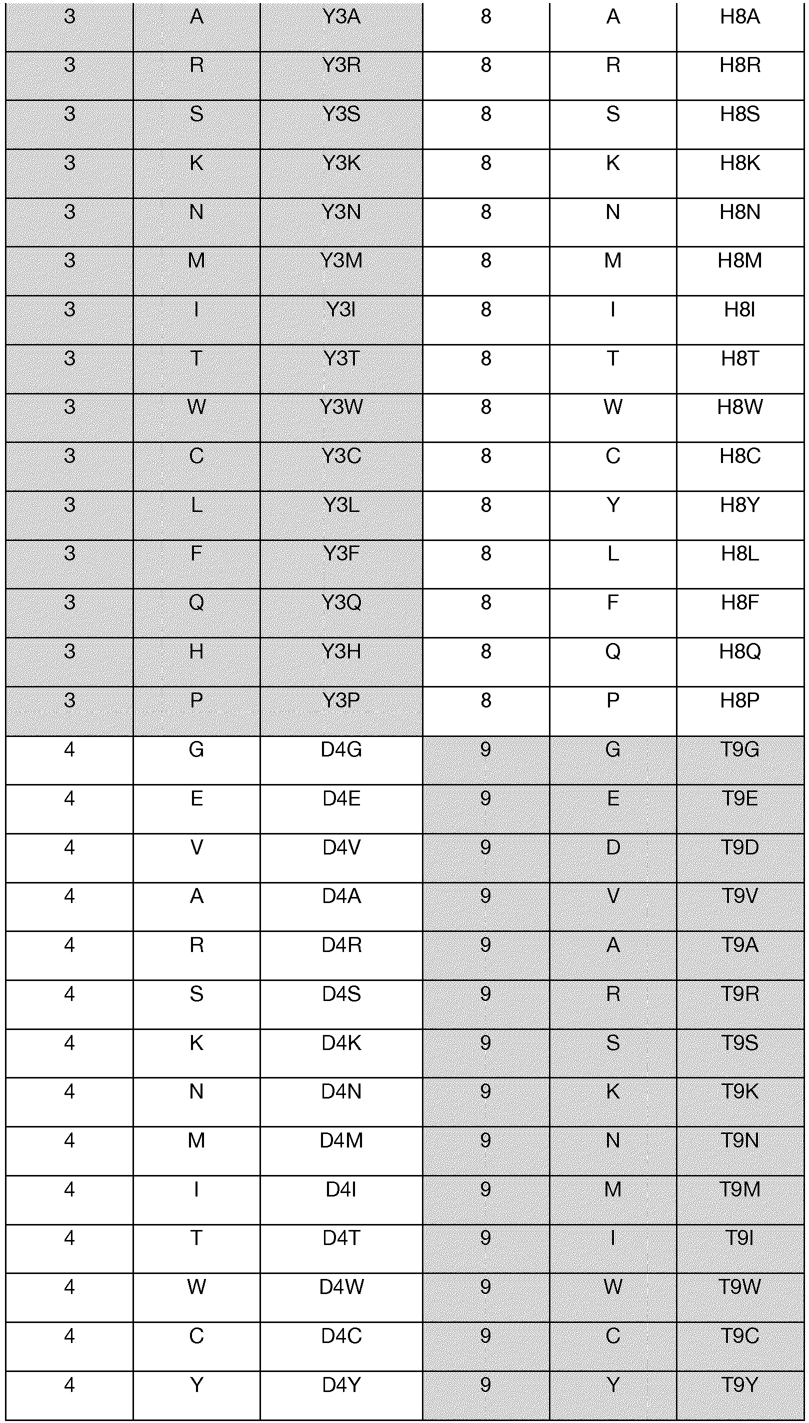

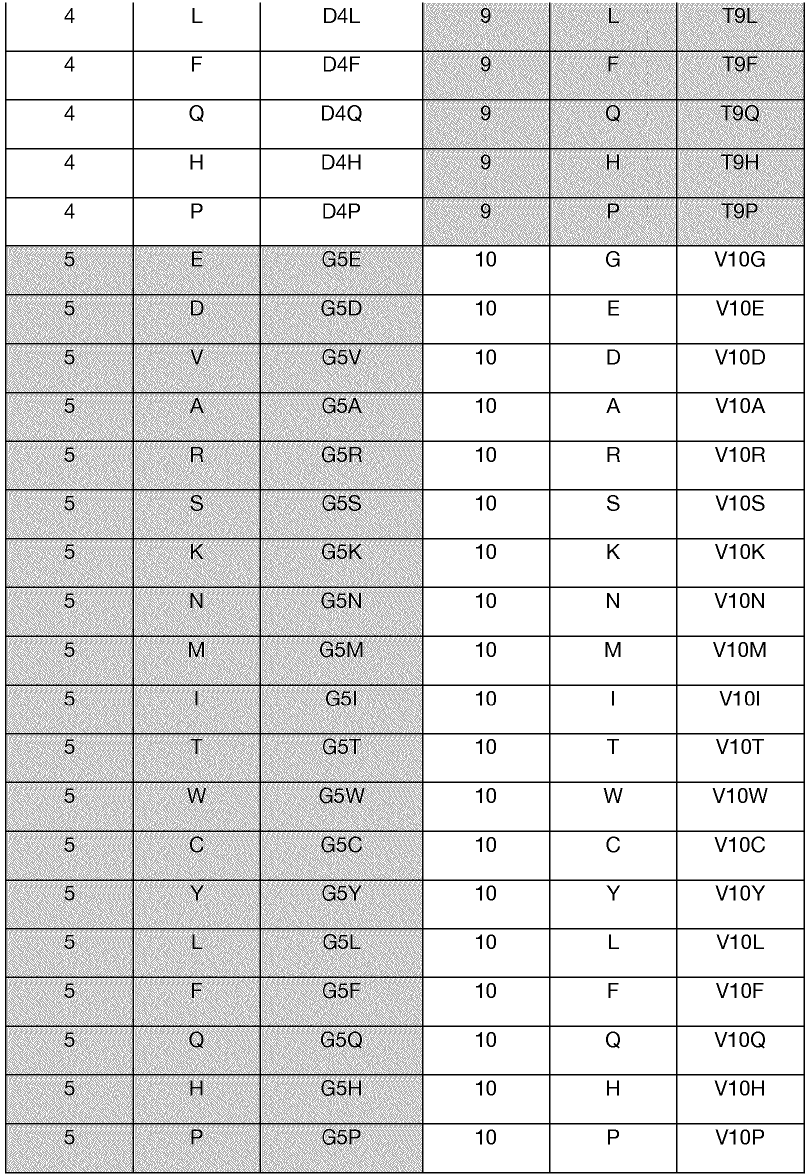

The mutated peptides were then used as aAM-ORFs to prepare eAPC-pa cells as described in the cell engineering section.

Responder assay was then performed with the TCR candidates as described.

### Derivation of Fingerprint score and identification of cross-reactive candidates

A matrix describing the response data, as percentage of maximal signal (position x amino acid), is converted into a PSSM (position-specific score matrix), using a fixed value multiplier. The PSSM matrix is then systematically aligned to all possible peptides in the human proteome (UniProt), thus identifying all epitope-length peptides where the linear series of residues are all positively scored by the PSSM, irrespective of the intensity of the scoring values per se, and which routinely comprises the base list of potential cross-reactivity candidates.

To provide a mechanism for relative ranking, the PSSM is converted to a log-
odds score matrix, applying a PSI-BLAST style pseudo-count (Altschul et al 2009), the positive values of which are used to derive a relative scoring metric for each candidate (referred to as flat score). Candidates are also scored for similarity to the HLA-A*02:01 using a pseudocount-corrected log-odds matrix derived from bulk immunopeptidomics data of eluted peptides from that HLA. The epitope sequence is additionally aligned and scored to all possible peptides within the human proteome using BLASTP "short" E and p-value scores with a BLOSUM62 matrix, so as to identify any additional candidates that may be closely structurally related. The above algorithms are implemented as custom python scripts.

### Cross-reactivity eAPC-pa Library construction

The fingerprint score was then screened against the whole human proteome to find matches to these tolerated positions and therefore predict potential cross-reactive hits. According to the fingerprint score, a number of cross-reactive hits were predicted and used to generate the cross-reactivity eAPC-pa library. ORFs of the identified cross-reactive hits were engineered into eAPC-p (HLA-A*02:01 monoallelic cells) by RMCE as described. The resulting eAPC-pa cells were then used in the responder assays with the eTPC-t expressing the TCRs.

### eAPC-p HLA Monoallelic library construction

A library of 86 monoallelic HLA-expressing eAPCs covering the most common HLA haplotypes was generated. The HLAs used to construct the library are summarised in Table 23. The eAPC-p cells were generated by RMCE as described in the cell engineering section.

**Table 23. A list of HLA haplotypes used in the alloreactivity assay. The HLA sequences were obtained from EBI (https://www.ebi.ac.uk/ipd/imgt/hla/) and the EBI accession numbers are listed under HLA name.**

| **HLA-A subtype alleles** | **HLA-B subtype alleles** | **HLA-C subtype alleles** | **HLA-E subtype alleles** |
|---|---|---|---|
| HLA-A*01:01 | HLA-B*07:02 | HLA-C*01:02 | HLA-E*01:01 |
| HLA00001 | HLA00132 | HLA00401 | HLA00934 |
| HLA-A*01:02 | HLA-B*07:05 | HLA-C*02:02 | HLA-E*01:03 |
| HLA00002 | HLA00137 | HLA00404 | HLA00936 |
| HLA-A*01:03 | HLA-B*08:01 | HLA-C*03:03 | |
| HLA00003 | HLA00146 | HLA00411 | |
| HLA-A*02:01 | HLA-B*13:01 | HLA-C*03:04 | |
| HLA00005 | HLA00152 | HLA00413 | |
| HLA-A*02:02 | HLA-B*13:02 | HLA-C*04:01 | |
| HLA00007 | HLA00153 | HLA00420 | |
| HLA-A*02:03 | HLA-B*14:01 | HLA-C*05:01 | |
| HLA00008 | HLA00157 | HLA00427 | |
| HLA-A*02:04 | HLA-B*14:02 | HLA-C*06:02 | |
| HLA00009 | HLA00158 | HLA00430 | |
| HLA-A*02:05 | HLA-B*15:01 | HLA-C*07:01 | |
| HLA00010 | HLA00162 | HLA00433 | |
| HLA-A*02:06 | HLA-B*15:02 | HLA-C*07:02 | |
| HLA00011 | HLA00165 | HLA00434 | |
| HLA-A*02:07 | HLA-B*15:03 | HLA-C*08:01 | |
| HLA00012 | HLA00166 | HLA00445 | |
| HLA-A*03:01 | HLA-B*18:01 | HLA-C*08:02 | |
| HLA00037 | HLA00213 | HLA00446 | |
| HLA-A*11:01 | HLA-B*27:05 | HLA-C*12:02 | |
| HLA00043 | HLA00225 | HLA00453 | |
| HLA-A*11:02 | HLA-B*35:01 | HLA-C*12:03 | |
| HLA00044 | HLA00237 | HLA00455 | |
| HLA-A*11:03 | HLA-B*35:02 | HLA-C*14:02 | |
| HLA00045 | HLA00238 | HLA00462 | |
| HLA-A*11:04 | HLA-B*35:03 | HLA-C*15:02 | |
| HLA00046 | HLA00239 | HLA00467 | |
| HLA-A*11:05 | HLA-B*35:05 | HLA-C*16:01 | |
| HLA00047 | HLA00241 | HLA00475 | |
| HLA-A*11:09 | HLA-B*35:08 | | |
| HLA01310 | HLA00244 | | |
| HLA-A*23:01 | HLA-B*35:12 | | |
| HLA00048 | HLA00249 | | |
| HLA-A*24:02 | HLA-B*37:01 | | |
| HLA00050 | HLA00265 | | |
| HLA-A*24:03 | HLA-B*38:01 | | |
| HLA00053 | HLA00267 | | |
| HLA-A*24:04 | HLA-B*40:01 | | |
| HLA00054 | HLA00291 | | |
| HLA-A*24:07 | HLA-B*40:02 | | |
| HLA00057 | HLA00293 | | |
| HLA-A*24:10 | HLA-B*44:02 | | |
| HLA00060 | HLA00318 | | |
| HLA-A*24:17 | HLA-B*44:03 | | |
| HLA00067 | HLA00319 | | |
| HLA-A*25:01 | HLA-B*49:01 | | |
| HLA00071 | HLA00340 | | |
| HLA-A*26:01 | HLA-B*50:01 | | |
| HLA00073 | HLA00341 | | |
| HLA-A*29:02 | HLA-B*51:01 | | |
| HLA00086 | HLA00344 | | |
| HLA-A*30:01 | HLA-B*51:02 | | |
| HLA00089 | HLA00346 | | |
| HLA-A*30:02 | HLA-B*52:01 | | |
| HLA00090 | HLA00362 | | |
| HLA-A*31:01 | HLA-B*53:01 | | |
| HLA00097 | HLA00364 | | |
| HLA-A*32:01 | HLA-B*54:01 | | |
| HLA00101 | HLA00367 | | |
| HLA-A*33:01 | HLA-B*55:01 | | |
| HLA00104 | HLA00368 | | |
| HLA-A*33:03 | HLA-B*57:01 | | |
| HLA00106 | HLA00381 | | |
| HLA-A*34:02 | HLA-B*58:01 | | |
| HLA00109 | HLA00386 | | |
| HLA-A*66:01 | | | |
| HLA00112 | | | |
| HLA-A*66:02 | | | |
| HLA00113 | | | |
| HLA-A*68:01 | | | |
| HLA00115 | | | |
| HLA-A*68:02 | | | |
| HLA00117 | | | |
| HLA-A*80:01 | | | |
| HLA00130 | | | |

### Cytotoxic assays

TCR-T cells (effector cells) were generated by directed endonuclease-mediated knock-in of the TCRs (TCR-T^{TCR-1}, TCR-T^{TCR-2}, TCR-T^{TCR-6}, TCR-T^{TCR-9} and TCR-T^{TCR-10}) into the endogenous TRAC locus and subsequently enriched for successfully edited cells, as described in material and methods, using CD34 microbead kit (Miltenyi Biotec, according to manufacturer's instructions). Target cells were used in cytotoxity assays with varied effector-to-target (E:T) ratio which involve monoallelic eAPCs constitutively expressing cognate HLA-A*02:01 in combination with antigen MAGE-A4 FL, as well as relevant control cell lines expressing HLA-A*02:01 MAGE-A4 MG and HLA-A*02:01 (off-target control); or common cancer cell lines natively expressing MAGE-A4 or not expressing MAGE-A4, retrovirally transduced to express HLA-A*02:01. All target cells recombinantly express Firefly Luciferase. Target cells were plated at 10,000 cells (eAPC) or 20,000 cells (cancer lines) per well in a 96-well plate. Adherent cancer cells were left to adhere and TCR-T cells were 2-fold serially diluted to obtain E:T ratios from 1:1 to 1:16. All test conditions were prepared in duplicates. After 24h of co-culture, target cell killing was assessed via luciferase activity of remaining viable cells in co-cultures compared to target only control wells and calculated as: % killing = 100 - ((RLU test condition)/(RLU target only)*100); RLU = relative luminescence units.

### Quantification of mRNA transcript abundance by ddPCR

Target gene RNA expression was established from 2ng - 25ng RNA (RNeasy Mini, QIAGEN) using a digital droplet PCR (ddPCR) one-step RT-ddPCR Kit for probes (Biorad) with the probes containing FAM or HEX and ZEN-IBFQ dual quencher (IDT). Assays were performed in duplex with TATA box binding protein (TBP) as reference and expression quantified as the ratio over TBP. Amplification was performed for 40 cycles with 60 sec annealing/ extension at 58-63°C. Droplets were acquired on a QX200 droplet reader (Biorad) and analyzed with QX Manager software (Biorad).

### Examples

The TCR discovery platform described in Figure 1, begins with the identification of HLA-restricted peptides derived from a target of interest (Step 1), which is then used in the TCR discovery (Step 2). Next, is the TCR and Antigen validation step (Step 3) where the platform's main focus is on delivering an effective and safe (derisked) TCR (Steps 4 and 5). The following examples describe each platform process and the key biological discovery of each step as shown in Figure 1.

### Example 1. Mass Spectrometric identification of MAGE-A4 peptides presented by mono-allelic HLA-A*02:01 eAPCs.

Herein is described the unbiased HLA-restricted peptide mapping for biologically relevant, processed and presented MAGE-A4 peptides, using mass spectrometry (MS) (Figure 2). Antigen presenting cells (eAPC) engineered to express single HLA class I allele (HLA-A*02:01) were generated as described in WO 2018/083316. The HLA-A*02:01 open reading frame (ORF) also encoded a C-terminal 6xHistidine tag for capture by metal affinity chromatography as previously described (WO 2018/083316). The monoallelic eAPCs were transfected with a full length (FL) version of inactivated MAGE-A4 protein (MAGE-A4_FL) (SEQ ID NO: 2).

Peptides released from affinity purified HLA-A*02:01 pHLA complexes were measured by unbiased mass spectrometry on a Lumos instrument. Figure 2 shows the z-scores relative to mean intensity of all observations for the specific observations of 8 peptides derived from the MAGE-A4. In general, the z-score is the intensity of a specific peptide as compared to the intensity of all other peptides in the sample. This means that calculating z-scores takes the raw intensity score of the peptide and subtracts the population mean and the resulting figure is divided by the population standard deviation. Z-scores are calculated from log₁₀ transformed data. 8 epitopes of the MAGE-A4 were observable, demonstrating that the MAGE-A4 peptide is processed. Out of the 8 peptides detected, one was not quantified.

In conclusion, the analysis confirmed 8 processed and presented MAGE-A4 peptides restricted to HLA-A*02:01 class I allele. Peptide GVYDGREHTV had the highest z-score.

### Example 2. Selective expansion of TCR clonotypes specific for identified MAGE-A4 peptides.

After confirming the existence of the target peptides, the next step is to use the identified MAGE-A4 peptides to discover TCR clonotypes that specifically bind them (Figure 1 Key platform Unit 2. TCR discovery).

To discover MAGE-A4-specific TCRs, naive CD8T cells derived from healthy donor peripheral blood mononuclear cells (PBMCs) were co-cultured with monoallelic engineered antigen presenting cells (eAPCs) constitutively expressing the HLA-A*02:01 in the presence of the MAGE-A4 peptide for 7 days. Therefore, in this experimental set up, naive CD8 T cells expressing a TCR capable of recognizing the MAGE-A4 peptide loaded and presented by the eAPC were selectively activated. Upon activation, CD8 T cells proliferated and expansion was supported by addition of exogenous interleukin 2 (IL-2) for 7 days. Expanded CD8 T cells were stained with Major Histocompatibility Complex (MHC) multimer (a PSMA tetramer that contains the MAGE-A4 peptide) and magnetically enriched before single cell sorting by fluorescence activated cell sorting (FACS). Alpha beta TCR pairs were sequenced and the frequency of appearance of each TCR clonotype was counted (Figure 3 A). If a TCR clonotype was observed at least 3 times it was considered enriched.

In conclusion, 17 TCR clonotypes that have the capability of recognizing MAGE-A4 presented by HLA-A*02:01 were successfully expanded and enriched.

### Example 3. Validation of sensitivity of the identified MAGE-A4- specific TCRs to HLA-A*02:01-presented MAGE-A4 peptides

Following the discovery of the peptides derived from the MAGE-A4 and the TCRs specific for these peptides, the TCR sensitivity was determined (Figure 1, Step 3).

To functionally evaluate the sensitivity of the identified MAGE-A4-specific TCRs, cell-cell contact assays with peptide-pulsed eAPCs contacted with TCR-expressing eTPCs (eAPC-p - eTPC-t) were applied as described briefly here. Firstly, the TCRs were expressed into engineered TCR presenting cells (eTPCs) containing a synthetic reporter element that responds to TCRsp engagement as described in WO 2018/083317. Briefly, the eTPCs have been engineered to contain a Response element that comprises of a Driver-Activator component and an Amplifier-Report component, wherein both units utilize synthetic promoters. The Driver is a synthetic promoter that is responsive to the native TCR signalling pathways, encoding three sets of tandem transcription factor binding sites for NFAT-AP1-NFkB (3xNF-AP-NB). Upon transcriptional activation, the Driver induces expression of the Activator protein, a synthetic designed transcription factor derived by fusion of the Herpes VP16 activation domain, the GAL4 DNA binding domain and two nuclear localization signals at the N- and C-terminals (NV16G4N), to which the cognate DNA recognition sequence is present 6 times in tandem in the Amplifier promoter. Both the Driver and Amplifier promoters utilize the core promoter sequence (B recognition element (BRE), TATA Box, Initiator (INR) and transcriptional start site) from HCMV IE1 promoter, immediately 3' of the respective transcription factor binding sites. The Amplifier upon transcriptional activation drives expression of the reporter, red fluorescent protein (RFP). To functionally evaluate the sensitivity of the enriched MAGE-A4 specific TCRs, the responder eTPC-ts expressing the MAGE-A4-specific TCRs were co-cultured with the monoallelic eAPCs presenting the HLA-A*02:01 that were either unpulsed or pulsed with a concentration gradient of the MAGE-A4 peptide. Following the peptide pulsed cell-cell contact assays, the cells were stained with CD3 antibodies and the samples were analysed for CD3 expression and RFP signal using flow cytometry. CD3 downregulation reflects the internalisation of the CD3/TCR complex following its interaction with its target and is usually associated to the strength of the interaction (avidity). The more sensitive a TCR is the more downregulation in the CD3 should be observed. It is initially a safety mechanism that the T cells have to avoid overactivation and the risk of damaging healthy cells. Stimulation of the TCR/CD3 complex results in RFP expression and CD3 downregulation by the eTPC-t, which is proportional to the strength of activation. The decrease in CD3 in combination with RFP is therefore used as a signal of TCR engagement and activation.

As an example, the flow cytometric profile of three representative MAGE-A4-responsive TCRs is shown in Figure 4 A-C and described below. Figure 4A is a representative of a MAGE-A4-specific TCR (on Target TCR) that is unpulsed with MAGE-A4 peptide (left panel) or pulsed with MAGE-A4 (right panel). The unpulsed eAPC HLA-A*02:01 cells stained high with the CD3 antibody and exhibited low RFP expression. Pulsing of the eAPC HLA-A*02:01 with MAGE-A4, led to increased number of cells with the RFP⁺CD3^{low} signature, thus confirming TCR activation. Figure 4B is a representative of a TCR that reacts with the HLA allele presented on the eAPCs, HLA-A*02:01, regardless of whether the target antigen is present (HLA-reactive). The RFP/CD3 profiles did not change when the cells were pulsed with MAGE-A4, but remained RFP⁺CD3^{low}. Figure 4C is a representative of a TCR that is unresponsive to the MAGE-A4, the cells have RFP⁻/ CD3^{high} signature in both unpulsed (left panel) and pulsed cells (right panel).

The flow cytometric data obtained from the peptide pulse assays for all MAGE-A4-responsive TCRs was used to construct sigmoidal curves from which the half maximal effective concentration (EC50) values were obtained. The data from the peptide pulse assays is represented in two ways - Figure 4 which shows that many of the MAGE-A4-responsive TCRs identified in the TCR discovery step are sensitive to the MAGE-A4. This is shown by plotting the TCRs against the EC50 - increased sensitivity is shown with EC50 values moving towards the right. Many of the identified TCRs showed high sensitivity. Secondly, Figure 5 focuses on selection of TCRs that were responsive to MAGE-A4 peptide and the obtained data is presented to demonstrate a more detailed response curve. In Figure 5 A-D the MAGE-4 specific TCRs (A) TCR-1, B) TCR-2, C) TCR-3 and D) TCR-4) when incubated with eAPC HLA-A*02:01 cells pulsed with a concentration range of MAGE-A4 peptides. The EC50 for TCR-1 and TCR-2 were respectively, 27.06 nM and 127.30 nM. The EC50 of TCR-3 was 42.71 nM and the EC50 value of TCR-4 was 14.99 nM.

Next, to further validate the interaction between MAGE-A4 -responsive TCRs and the MAGE-A4 peptide, an assay was performed to look for a response when the target peptide is endogenously expressed and presented by the antigen presenting cells, Figures 6. Therefore, monoallelic HLA-A*02:01 eAPCs or the monoallelic HLA-A*02:01 eAPC line constitutively expressing MAGE-A4 peptide either as a minigene (MG) (SEQ ID NO: 3) or as a full-length peptide (FL) (SEQ ID NO: 4) were co-cultured with responder eTPC-ts expressing the MAGE-A4-responsive TCRs (Figure 6A). The response of eTPC-t responder cells was measured by RFP signaling in combination with CD3 downregulation (RFP⁺CD3^{low}).

A large gradient in the response of the responder cells expressing the MAGE-A4-responsive TCRs was observed after incubation with the eAPC HLA-A*02:01 expressing the FL (SEQ ID NO: 4) or the MG (SEQ ID NO: 3) MAGE-A4 constructs (Figure 6A). No difference in response was observed between the constructs. Importantly, the TCRs identified in the peptide pulse sensitivity assays were also detected in this experiment and are marked retrospectively. Next, a panel of cancer cell lines (CCLs) known to express MAGE-A4 peptide via HLA-A*02:01 was used in the cell-cell contact assay. CCLs were incubated with the eTPC-t cells expressing the MAGE-A4-specific TCRs (eTPC-t^{TCR-1}, eTPC-t^{TCR-2}, eTPC-t^{TCR-3} and eTPC-t^{TCR-4} (Figure 6B). A CCL that does not express the MAGE-A4 peptide (SAOS-2) was used as control.

In conclusion, several MAGE-A4-responsive TCRs were identified. Furthermore, the TCR - antigen functional validation step within the platform demonstrated that the selected TCRs mediate functional activation by peptide pulse with MAGE-A4 or endogenous expression of MAGE-A4 presented via HLA-A*02:01.

### Example 4: MAGE-A4 specific TCRs mediate potent T cell killing of cells presenting the MAGE-A4 peptide in the HLA-A*02:01 context

As part of the TCR de-risking process, the cytotoxic potency of the MAGE-A4-specific TCRs was analysed by using primary T cells transduced with the TCRs (TCR-T^{TCR-1}, TCR-T^{TCR-2}, TCR-T^{TCR-3} and TCR-T^{TCR-4}), co-cultured with luciferase-expressing eAPCs (Figure 7 A-D) and the ONE-Glo Luciferase Assay System (Promega) according manufacturer's instructions. During Luciferase activity-based cell-cell killing assays, firefly luciferase enzymatically converts in an ATP-dependent reaction chromogenic luciferin to oxyluciferin emitting luminescence that can be sensitively and efficiently measured using a plate-based spectrophotometer. Firefly luciferase when ectopically expressed in cells is highly sensitive and specifically measures cell viability as its protein levels and enzymatic activity rapidly decline in non-viable cells and when exposed to most cell culture media (Coombe et al, 1998). The cytotoxic potency of the primary T cells transduced with the TCRs was also assessed on a selection of cancer cell lines expressing MAGE-A4 within the HLA-A*02:01 context and compared to the SAOS-2 CCL that does not express MAGE-A4 (Figure 7 E-H).

TCR-T cells (effector cells) were generated by directed endonuclease-mediated knock-in of the TCRs (TCR-T^{TCR-1}, TCR-T^{TCR-2}, TCR-T^{TCR-3} and TCR-T^{TCR-4}) into the endogenous TRAC locus and subsequently enriched for successfully edited cells, as described in material and methods, using CD34 microbead kit (Miltenyi Biotec, according to manufacturer's instructions). Monoallelic eAPCs (Target cells, ARH-77) constitutively expressing HLA-A*02:01 in combination with endogenously expressing MAGE-A4 full length (FL) peptide, as well as monoallelic eAPCs expressing HLA-A*02:01 and MAGE-A4 minigene (MG) peptide and HLA-A*02:01 only (off-target control), were used in cytotoxity assays with variation in effector-to-target (E:T) ratios. For variation in E:T ratio, target cells were plated at 10,000 per well in a 96-well plate and TCR-T cells were 2-fold serially diluted to obtain E:T ratios of 1:1 to 1:16. Cytotoxicity assays were performed by luciferase activity readout after a 24h co-culture of TCR-T and target eAPC-p or -pa lines. The target cell killing was assessed via luciferase activity of remaining viable cells in co-cultures compared to target only control wells and calculated as: % killing = 100 - ((RLU test condition)/(RLU target only)*100); RLU = relative luminescence units.

Rapid apoptotic death of the target cells was induced by TCR-T cells expressing TCR-2, TCR-3 or TCR-4 against the target cells expressing the MAGE-A4 FL (SEQ ID NO: 4), or MAGE-A4 MG (SEQ ID NO: 3) (Figure 7 B-D). Additionally, absence of killing of off-target eAPC expressing HLA-A*02:01 confirms the absence of overt off-target reactivity.

The cytotoxic killing potential of the effector TCR-Ts (TCR-T^{TCR-1}, TCR-T^{TCR-2}, TCR-T^{TCR-3} and TCR-T^{TCR-4}) on cancer cell lines expressing the MAGE-A4 via HLA-A*01:02 (Figure 7 F-H). Using immortalized cell lines from public domain collections, two model lines that are known to natively express MAGE-A4 and have a gradient of HLA-A*02:01 surface density were generated. The cell lines expressing MAGE-A4, were transduced to express HLA-A*02:01 as well as Firefly luciferase and nuclear localizing green fluorescent protein (GFP) to support luciferase and real-time imaging based killing assays, respectively. The cell lines have HLA-A*02:01 surface median flourescent intensities (MFI)s raging from 11 5027 (NCI-H1755) to 26 258 (SAOS-02) as measured by flow cytometry (Table 24). MAGE-A4 expression levels were measured using digital droplet polymerase chain reaction (ddPCR) and with the exception of NCI-H1755, the HLA-A*02:01 levels are largely consistent across the cell lines.

**Table 24. Characteristics of cancer lines used in cytotoxicity assays**

| | NCI-H1755 | NCI-H1703 | SAOS-2 |
|---|---|---|---|
| MAGE-A4 status | positive | positive | negative |
| MAGE-A4 expression a | 37.1 | 8.00 | 0.03 |
| HLA-A*02:01 density b | 115 027 | 28218 | 26 598 |

^{a}MAGE-A4 expression relative to the housekeeping gene TATA-box binding protein (TBP) ^{b}HLA-A*02:01 surface density is the median fluorescence intensity (MFI) established by antibody staining and flow cytometry.

Very strong killing of both cancer lines across varying MAGE-A4 expression levels and HLA-A*02:01 surface levels was observed. Killing was near-complete at E:T of 1:1 but was also notably strong at E:T of 4:1 with signs of killing observed even at E:T ratios of 16:1, with slight indication of killing of the SAOS-2 control line which does not express MAGE-A4 at 1:1 E:T ratio. The results confirmed that the cytotoxic activity of the TCR-T^{TCR-1}, TCR-T^{TCR-2}, TCR-T^{TCR-3} and TCR-T^{TCR-4} cells is potent enough to deliver strong killing of cell lines derived from cancers that natively express MAGE-A4.

In conclusion, potent and selective toxicity of primary T cells expressing TCR-T^{TCR-1}, TCR-T^{TCR-2}, TCR-T^{TCR-3} and TCR-T^{TCR-4} against cells presenting the MAGE-A4 peptide via HLA-A*02:01 was observed. While target cells not expressing MAGE-A4 were not killed by the TCR-T^{TCR-2}, TCR-T^{TCR-3} and TCR-T^{TCR-4} effector cells thus demonstrating no overt off-target reactivities.

### Example 5. Cross-reactive and allo-reactive profiling of MAGE-A4 TCRs.

The TCRs that passed the functional evaluation sensitivity tests were next triaged into assays that determined their safety profile and ultimately end up with de-risked TCRs (Figure 1, Step 5). The first step in the safety assessment, the MAGE-A4 TCRs undergo profiling for fingerprinting (Figure 8). The present example shows the fingerprinting of MAGE-A4 TCRs using positional scanning peptide matrix. A peptide library, containing all possible naturally occurring single amino acids at each position within the MAGE-A4 peptide, was created (the Epitope Mutagenesis Library). Each library peptide was then engineered into eAPC to generate a library of monoallelic HLA-A*02:01 eAPCs stably expressing the mutated version of the MAGE-A4 epitope. The eAPC cells were then co-cultured for 18 hours with TCR-Ts expressing TCRs (either TCR-1, TCR-2, TCR-3 or TCR-4). Activation of the responder cells was measured by RFP signal in combination with CD3 downregulation, using flow cytometry. The results were then plotted as a fingerprint profile, wherein the "y-axis" represents the amino acid substitutionsand the "x-axis" the position where it each substitution has been introduced. The darker the colour, the higher the response due to that particular amino acid substitution in the epitope sequence. In this example, fingerprint profiles of the 4 TCRs are presented (Figure 8 A-D). The fingerprint profiles demonstrate that the TCRs are unique in their ability to recognize the target peptide and reveal where tolerances are in that TCR-target peptide engagement. Based on these tolerances, a computer algorithm was generated and used to obtain a fingerprint score. The fingerprint score is then screened against the whole human proteome to find matches to these tolerated positions and therefore predict potential cross-reactive hits (Figure 9 A). According to the fingerprint score, many cross-reactive hits were predicted for TCR-4 but only one cross-reactive hits was predicted for TCR-3, TCR-1 and TCR-2 (Figure 9A). The sequences from the predicted cross-reactive hits were then used to create minigene (MG) constructs that were introduced into the monoallelic HLA-A*02:01 eAPCs. Subsequently, the cells were co-cultured with the eTPC-t responder cells expressing the TCRs, TCR-1, TCR-2, TCR-3 or TCR-4. To assess the cross-reactivity potential of the TCRs, the activation of the eTPC-t responder cells was measured by flow cytometry following the co-culture. A threshold of 4% was applied as a cut off for cross-reactivity and anything above this threshold was considered cross-reactive. For TCR-4 (SEQ ID 63 - alpha chain and SEQ ID 64 - beta chain or SEQ ID NO 84 Beta chain) the cross-reactivity assay has confirmed 4 of the predicted cross-reactive hits (MAGE-A8, MAGE-A10, VPS13D, ZNF106). TCR-3, TCR-1 and TCR-2 had 1 cross-reactive hit confirmed (Figure 9B). The predicted and actual cross-reactive hit is MAGE-A8 peptide GLYDGREHSV (SEQ ID NO: 85). The TCRs demonstrated cross-reactivity to MAGE-A8 peptide. As part of the safety profile, allo-reactivity and para-reactivity were determined for the 4 TCRs (Figure 10). Here, the responder eTPC-ts expressing the MAGE-A4-responsive TCRs were co-cultured for 18 hours with a library of monoallelic HLA-expressing eAPCs covering the most common HLA haplotypes. The response was measured by flow cytometry by detection of RFP expression and reduction in CD3 signal (RFP⁺CD3^{low}). TCR-1 showed weak allo-reactivity to HLA-B*57:01. After pulsing the HLA eAPC library with MAGE-A4, peptide-specific activation (para-reactivity) via several members of the HLA-A*02 supergroup (01, 02, 04, 05, 06 and 07) was observed and to some extent activation through the HLA-B*57:01. TCR-2 was weakly allo-reactive with HLA-A*02:02. Para-reactivity was observed following peptide pulse assay with MAGE-A4 with fewer members of the HLA-A*02 supergroup (01, 02 and 07). TCR-3 was weakly allo-reactive towards HLA-A*02:02 and HLA-B*15:01. Para-reactivity was observed following peptide pulse assay with MAGE-A4 with members of the HLA-A*02 supergroup (01, 02, 03, 04, 05, 06 and 07), HLA-A*68:02 and HLA-B*15:01. TCR-4 was allo-reactive towards HLA-A*29:02 and para-reactive towards members of the HLA-A*02 supergroup (01, 02, 03, 04, 05, 06 and 07), HLA-A*11:04, HLA-A*29:02, HLA-A*30:02, HLA-A*68:02, HLA-B*38:01, HLA-B*54:01 and HLA-C*07:01, HLA-C*12:03 and HLA-C*15:02.The allo-reactivity and para-reactivity results are summarized in Table 25 below.

**Table 25.**

| **TCR-ID** | **Recognition (Alloreactivity) "off-HLA"** | **Specific recognition (Parareactivity) "on-target-off-HLA"** |
|---|---|---|
| TCR-1 | B*57:01 | A*02:01, A*02:02, A*02:04, A*02:05, A*02:06; A*02:07, B*57:01 |
| TCR-2 | A*02:02 | A*02:01, A*02:02, A*02:07 |
| TCR-3 | A*02:02, B*15:01 | A*02:01, A*02:02, A*02:03, A*02:04, A*02:05, A*02:06; A*02:07, A*68:02, B*15:01 |
| TCR-4 | A*29:02 | A*02:01, A*02:02, A*02:03, A*02:04, A*02:05, A*02:06; A*02:07, A*11:04, A*29:02, A*30:02, A*68:02, B*38:01, B*54:01, C07:01, C*12:03, C*15:02 |

In conclusion, we have identified and validated 3 MAGE-A4-specific TCRs with a defined set of potential allo-reactive HLA alleles. Together, with the knowledge gained from the HLA allo-reactivity assays it is possible to proactively mitigate clinical risks and further tailor the use of the validated TCRs during clinical trials depending on the HLA profile of each patient.

### List of References

Altschul, S F, Gish W, Miller W, Myers EW and Lipman DJ. 1990. Basic Local Alignment Search Tool. J. Mol. Biol. 215:403-10.
Altschul SF, Gertz ME, Agarwala R, Schäffer AA and Yu Y-K. 2009 Nucleic Acids Res. Feb; 37(3): 815-824
Andreatta M, Alvarez B and Nielsen M. 2017. Nucleic acids Res. 45(1): 458-463
Banker GS and Chalmers RK, Pharmaceutics and Pharmacy Practice, (1982) Publisher J. B. Lippincott Company, Philadelphia, pages 238-250
Bender R.R., Muth A., Schneider I.C., Friedel T., Hartmann J., Plückthun A., Maisner A., Buchholz C.J. Receptor-Targeted Nipah Virus Glycoproteins Improve Cell-Type Selective Gene Delivery and Reveal a Preference for Membrane-Proximal Cell Attachment. PLoS Pathog. 2016;12:e1005641.
Bijker MS; van den Eeden SJF; Franken KL; Melief CJM; Offringa R; van der Burg SH. CD8+ CTL Priming by Exact Peptide Epitopes in Incomplete Freund's Adjuvant Induces a Vanishing CTL Response, whereas Long Peptides Induce Sustained CTL Reactivity. J Immunol (2007) 179 (8): 5033-5040
Bos R and Sherman LA, CD4+ T cell help in the tumour milieu is required for recruitment and cytolytic function of CD8+ T lymphocytes. Cancer Res, 2010, 70:8368-8377
Boulter JM, Glick M, Todorov PT, Baston E, Sami M, Rizkallah P & Jakobsen BK (2003) Stable, soluble T-cell receptor molecules for crystallization and therapeutics. Protein Eng 16, 707-711
Breda L, Papp T. E., Triebwasser M. P., Yadegari A., Fedorky M. T., Tanaka N., Abdulmalik O., Pavani G., Wang Y. and Parhiz H. In vivo hematopoietic stem cell modification by mRNA delivery. Science 381, 436-443 (2023)
Caballero, O. L. & Chen, Y. T. Cancer/testis (CT) antigens: potential targets for immunotherapy. Cancer Sci. 100, 2014-2021 (2009)
Cassioli C, Baldari CT. The Expanding Arsenal of Cytotoxic T Cells. Front Immunol. 2022;13:883010.
Choi J, Goulding S, Conn B, McGann C, Dietze J, Kohler J, Lenkala D, Boudot A, Rothenberg DJurcott P, Srouji J, Foley K, Rooney M, van Buuren M, Gaynor R, Abelin J, Addona A, Juneja V.Reports Methods 2021:1:100084.
Cohen, S. N., Chang ACY and Hsu L. Nonchromosomal antibiotic resistance in bacteria: Genetic transformation of Escherichia coli by R-factor DNA. Proceedings of the National Academy of Sciences 69, 2110-2114 (1972)
Coombe DR, Nakhoul AM, Stevenson SM, Peroni SE, Sanderson CJ. Expressed luciferase viability assay (ELVA) for the measurement of cell growth and viability. J Immunol Methods. 1998;215(1-2):145-150.
Cooper L.J.N.Topp M.S.Pinzon C.Plavec I.Jensen M.C.Riddell S.R.Greenberg P.D (2004) Enhanced transgene expression in quiescent and activated human CD8+ T cells. Hum. Gene Ther.15648-658*.*
Dalloul A. CD5: a safeguard against autoimmunity and shield for cancers. Autoimmun Rev. (2009), 8(4):349-53
Ducreux M, Cuhna AS, Caramella C, et al. Cancer of the pancreas: ESMO Clinical Practice Guidelines for diagnosis, treatment and follow-up [published correction appears in Ann Oncol. 2017 Jul 1;28(suppl_4):iv167-iv168]. Ann Oncol. 2015;26 Suppl 5:v56-v68.
Daudi, S. et al. Expression and immune responses to MAGE antigens predict survival in epithelial ovarian cancer. PLoS ONE 9, e104099 (2014)
Giuntoli II RL, Lu J, Kobayashi H, Kennedy R and Celis E. Direct costimulation of tumour-reactive CTL by Helper T cells potentiate their proliferation, survival and effector function. Clin Cancer Res, 2002, 8:922-931
Green, M. and Sambrook, J. (2012) Molecular Cloning: A Laboratory Manual. 4th Edition, Vol. II, Cold Spring Harbor Laboratory Press, New York.
Gure et al Clin Cancer Res 2005, 11:8055-8062
Gutierrez-Guerrero A, François-Loïc Cosset and Els Verhoeven. Lentiviral Vector Pseudotypes: Precious Tools to Improve Gene Modification of Hematopoietic Cells for Research and Gene Therapy. 2020 Viruses, 12 (9): 1016
Han A, Glanville J, Hansmann L and Davis MM. Linking T cell receptor sequence to functional phenotype at the single cell level. Nat. Biotech. 2014 32(7): 684-92
Hobbs GA, Gunawardena HP, Baker R, Campbell SL. Site-specific monoubiquitination activates Ras by impeding GTPase-activating protein function. Small GTPases. 2013;4(3):186-192
Hu Y, Petroni GR, Olson WC, Czarkowski A, Smolkin ME, Grosh WW, Chianese-Bullock KA and Slingluff CL. Immunologic hierarchy, class II MHC promiscuity, and epitope spreading of a melanoma helper peptide vaccine. Cancer Immunol Immunother, 2014, 63:779-786
Ishihara, M. et al. MAGE-A4, NY-ESO-1 and SAGE mRNA expression rates and co-expression relationships in solid tumours. BMC Cancer 20, 606 (2020)
Ito M, Hiramatsu H, Kobayashi K, et al. NOD/SCID/gamma(c)(null) mouse: an excellent recipient mouse model for engraftment of human cells. Blood. 2002;100(9):3175-3182. doi:10.1182/blood-2001-12-0207
lura, K. et al. Cancer-testis antigen expression in synovial sarcoma: NY-ESO-1, PRAME, MAGEA4, and MAGEA1. Hum. Pathol. 61, 130-139 (2017).
Joglekar AV and Sandoval S. Pseudotyped Lentiviral Vectors: One vector, many guises. Hum Gene Ther Methods (2017) 28:291-301
Jones S.Peng P.D.Yang S.Hsu C.Cohen C.J.Zhao Y.Abad J.Zheng Z.Rosenberg S.A.Morgan R.A.2009. Lentiviral vector design for optimal T cell receptor gene expression in the transduction of peripheral blood lymphocytes and tumor-infiltrating lymphocytes. Hum. Gene Ther.20630-640.
Joseph A.Zheng J.H.Follenzi A.Dilorenzo T.Sango K.Hyman J.Chen K.Piechocka-Trocha A.Brander C.Hooijberg E.Vignali D.A.Walker B.D.Goldstein H.2008. Lentiviral vectors encoding human immunodeficiency virus type 1 (HIV-1)-specific T-cell receptor genes efficiently convert peripheral blood CD8 T lymphocytes into cytotoxic T lymphocytes with potent in vitro and in vivo HIV-1-specific inhibitory activity. J. Virol.823078-3089.
Karlin S and Altschul, S.F. Methods for assessing the statistical significance of molecular sequence features by using general scoring schemes. Proc Natl Acad Sci U S A. 1990 Mar; 87(6): 2264-2268.
Kim et al J Mol Med 2012, 29:656-662
Leidner R, Silva NS, Huang H, Sprott D, Zheng C, Shih Y-P, Leung A, Payne R, Sutcliffe K, Cramer J, Rosenberg S, Fox B, Urba W, Tran NE. Engl 1 Med 2022;386:2112-2119.
Li et al Clin cancer Res 2005, 11:1809-1814
Melssen M and Slingluff CL, Curr Opin Immunol. 2017 Aug; 47: 85-92). CD4+ T cells harbor a crucial role for effective antitumor immunity
Muranski P, Antony PA, Restifo NP and Allison JP. Tumor-reactive CD4+ T cells develop cytotoxic activity and eradicate large established melanoma after transfer into lymphopenic hosts, J. exp. Med, 2010, 207:637-650
Nelde A, Rammensee HG and Waltz JS, The peptide vaccine of the future, Mol Cell Proteomics, 2021, 20, 100022
Otte et al Cancer Res 2001, 61:6682-6688
Parayath NN, Stephan SB, Koehne AL, Nelson PS and Stephan MT. In vitro-transcribed antigen receptor mRNA nanocarriers for transient expression in circulating T cells in vivo. Nat Commun. 2020. https://doi.org/10.1038/s41467-020-19486-2
Pharmaceutics and Pharmacy Practice, J. B. Lippincott Company, Philadelphia, PA., Banker and Chalmers, eds., pages 238-250 (1982)
Poole P, Karuppiah V, Hartt A, Haidar J, Moureau S, Dobrzycki T, Hayes C, Row- ley C, Dias J, Harper H, Barnbrook K, Hock M, Coles C, Yang W, Aleksic M, Bence Lin A, Robinson R, Dukes J, Liddy N, Van der Kamp M, Plowman G, Vui- depot A, Cole D, Whale A, Chillakuri C.Nature Commun 2022:13:5333.
Provasi E., Genovese P., Lombardo A., Magnani Z., Liu P.Q., Reik A., Chu V., Paschon D.E., Zhang L., Kuball J., et al. Editing T cell specificity towards leukemia by zinc finger nucleases and lentiviral gene transfer. Nat. Med. 2012;18:807-815
Qasim W., Zhan H., Samarasinghe S., Adams S., Amrolia P., Stafford S., Butler K., Rivat C., Wright G., Somana K., et al. Molecular remission of infant B-ALL after infusion of universal TALEN gene-edited CAR T cells. Sci. Transl. Med. 2017;9
Quezada SA, Simpson TR, Peggs KS, Merghoub T, Vider J, Fan X, Blasberg R, Yagita H,
Rammensee, H., Bachmann, J., Emmerich, N. P., Bachor, O. A. & Stevanović, S. SYFPEITHI: database for MHC ligands and peptide motifs. Immunogenetics 50, 213-219 (1999).
Rawla P, Sunkara T, Gaduputi V. Epidemiology of pancreatic cancer: Global trends, etiology and risk factors. World J Oncol. 2019;10(1):10-27.
Robinson RA , McMurran C, McCully ML and Cole DK. Engineering soluble T cell receptors for therapy. The FEBS Journal 288 (2021) 6159-6173
Saiki RK, Gelfand DH, Stoffel S, Scharf SJ, Higuchi, Horn GT, Mullis KB, Erlich HA. Primer-directed enzymatic amplification of DNA with a thermostable DNA polymerase. Science. 1988; 239(4839):487-91
Siebart JC, Chan CS, Yao X, Su F-Y, and Kwong GA. In vivo gene delivery to immune cells. Curr. Op. in Biotech. 2024. Volume 88, August 2024, page 103169
Sharma, P. et al. Cancer-testis antigens: expression and correlation with survival in human urothelial carcinoma. Clin. Cancer Res. 12, 5442-5447 (2006)
Shifrut E., Carnevale J., Tobin V., Roth T.L., Woo J.M., Bui C.T., Li P.J., Diolaiti M.E., Ashworth A., Marson A. Genome-wide CRISPR Screens in Primary Human T Cells Reveal Key Regulators of Immune Function. Cell. 2018;175:1958-1971
Spitzer MH, Carmi Y, Reticker-Flynn NE, Kwek SS, Madhireddy D, Martins MM, Gherardini PF, Prestwood TR, Chabon J, Bendall SC, Fong L, Nolan GP, and Engleman EG. Cell, 2017, 168:487-502
Straathof K.C., Pule M.A., Yotnda P., Dotti G., Vanin E.F., Brenner M.K., Heslop H.E., Spencer D.M., Rooney C.M. An inducible caspase 9 safety switch for T-cell therapy. Blood. 2005;105:4247- 4254
Su S., Zou Z., Chen F., Ding N., Du J., Shao J., Li L., Fu Y., Hu B., Yang Y., et al. CRISPR-Cas9-mediated disruption of PD-1 on human T cells for adoptive cellular therapies of EBV positive gastric cancer. Oncoimmunology. 2017;6
Su S., Hu B., Shao J., Shen B., Du J., Du Y., Zhou J., Yu L., Zhang L., Chen F., et al. CRISPR-Cas9 mediated efficient PD-1 disruption on human primary T cells from cancer patients. Sci. Rep. 2016;6:20070
Tajima et al Lung Cancer, 2003, 42:23-33
Tilseda CM, Sadiq BA, Papp TE, Areesawangkit P, Kimura K, Noguera-Ortega E, Scholler J, Cerda N, Aghajanian H, Bot A, Mui B, Tam Y, Weissman D, June C-H, Albelda SM and Parhiz H, 2024, IL7 increases targeted lipid nanoparticle-mediated mRNA expression in T cells in vitro and in vivo by enhancing T cell protein translation. 2024. PNAS 2024 Vol. 121 No. 13
Tombácz I., Laczkó D, Shahnawaz, Muramatsu H, Natesan A, Yadegari A, Papp TE, Alameh M-G, Shuvaev V, Mui BL, Tam YK, Muzykantov V, Pardi N, Weissman D and Parhiz H. Highly efficient CD4+ T cell targeting and genetic recombination using engineered CD4+ cell-homing mRNA-LNPs. Mol. Ther. J. Am. Soc. Gene Ther. 29, 3293-3304 (2021).
Trissel LA. ASHP Handbook on Injectable Drugs, 1986, 4th ed., pages 622- 630
Tsuji T.Yasukawa M.Matsuzaki J.Ohkuri T.Chamoto K.Wakita D.Azuma T.Niiya H.Miyoshi H.Kuzushima K.Oka Y.Sugiyama H.Ikeda H.Nishimura T.2005. Generation of tumor-specific, HLA class I-restricted human Th1 and Tc1 cells by cell engineering with tumor peptide-specific T-cell receptor genes. Blood 106470-476.
Vavassori V, Ferrari S, Beretta S, Asperti C, Albano L, Annoni A, Gaddoni, C, Varesi A, Soldi M, Cuomo A, Bonaldi T, Radrizzani M, Merelli, I and Naldini L. Lipid nanoparticles allow efficient and harmless ex vivo gene editing of human hematopoietic cells. 2023 Blood, Aug 31;142(9):812-826
Wada T. Downregulation of CD5 and dysregulated CD8+ T cell activation. Pediatr Int. (2018) 60 (9): 776-780
Weber EW, Maus MV, Mackall CL. The Emerging Landscape of Immune Cell Therapies. Cell (2020) 181:46-62
Zwaveling S, Mota SCF, Nouta J, Johnson M, Lipford GB, Offringa R, van der Burg SJ and Melief CJM. Established human papillomavirus type 16-expressing tumours are effectively eradicated following vaccination with long peptides. 2002. J. Immunol, 2002, 169:350-358

### Items I

1. A T-cell receptor (TCR), which is isolated and/or expressed in an engineered cell, specifically recognizing a melanoma-associated antigen A4 (MAGE-A4) peptide, wherein the peptide consists of GVYDGREHTV (SEQ ID NO: 1) presented in a HLA-A*02 complex, and wherein the TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.
2. The TCR according to item 1, wherein the TCR, when expressed in an engineered cell, does not cause specific cytotoxic activity towards cells not expressing a MAGE-A4 peptide.
3. The TCR according to item 1, wherein the TCR, when expressed in an engineered cell, does not cause specific cytotoxic activity towards cells not expressing a MAGE-A8 peptide.
4. The TCR according to item 1, wherein the TCR, when expressed in an engineered cell, does not cause specific cytotoxic activity towards cells not expressing a MAGE-A4 peptide and/or a MAGE-A8 peptide.
5. The TCR according to item 1, wherein the TCR, when expressed in an engineered cell, does not cause specific cytotoxic activity towards cells not expressing a MAGE-A4 peptide.
6. The TCR according to any of the preceding items, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02; HLA-A*02:03; HLA-A*02:04; HLA-A*02:05; HLA-A*02:06; HLA-A*02:07, HLA-B*57:01, HLA-A*68:02 and HLA-B*15:01, serotype.
7. The TCR according to any of the preceding items, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02; HLA-A*02:04; HLA-A*02:05; HLA-A*02:06; HLA-A*02:07, and HLA-B*57:01 serotype.
8. The TCR according to any of the preceding items, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02 and HLA-A*02:07 serotype.
9. The TCR according to any of the preceding items, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02; HLA-A*02:03; HLA-A*02:04; HLA-A*02:05; HLA-A*02:06; HLA-A*02:07; HLA-A*68:02 and HLA-B*15:01 serotype.
10. The TCR according to any one of the preceding items wherein the TCR when expressed in an engineered cell does not specifically recognise other allelic variants of the human leukocyte antigen (HLA) molecule other than an allelic variant of the human leukocyte antigen (HLA) molecule selected from the group consisting of HLA-B*57:01, HLA-A*02:02 and HLA-B*15:01 serotype.
11. The TCR according to any one of the preceding items wherein the TCR when expressed in an engineered cell does not specifically recognise other allelic variants of the human leukocyte antigen (HLA) molecule other than HLA-B*57:01 serotype.
12. The TCR according to any one of the preceding items wherein the TCR when expressed in an engineered cell does not specifically recognise other allelic variants of the human leukocyte antigen (HLA) molecule other than HLA-A*02:02 serotype.
13. The TCR according to any one of the preceding items wherein the TCR when expressed in an engineered cell does not specifically recognise other allelic variants of the human leukocyte antigen (HLA) molecule other than HLA-A*02:02 and HLA-B*15:01 serotype.
14. The TCR according to any of the preceding items, wherein the TCR, when expressed in an engineered cell, has at the most 4% cross-reactivity towards any somatically expressed HLA-presented peptide other than a MAGE-A4 peptide, which consists of GVYDGREHTV (SEQ ID NO: 1) and which is presented in a HLA-A*02:01 complex in a cross-reactivity assay.
15. The TCR according to any of the preceding items comprising:
a. a TCR alpha chain CDR3 sequence with at least 75% identity to a TCR alpha chain CDR3 sequence selected from the group consisting of SEQ ID NO: 46, SEQ ID NO: 48 and SEQ ID NO: 50 and
b. a TCR beta chain CDR3 sequence with at least 80% identity to a TCR beta chain CDR3 sequence selected from the group consisting of SEQ ID NO: 47, SEQ ID NO: 49 and SEQ ID NO: 51.
16. The TCR according to any of the preceding items comprising:
a. a TCR alpha chain CDR3 sequence with a TCR alpha chain CDR3 sequence selected from the group consisting of SEQ ID NO: 46, SEQ ID NO: 48 and SEQ ID NO: 50 and
b. a TCR beta chain CDR3 sequence with a TCR beta chain CDR3 sequence selected from the group consisting of SEQ ID SEQ ID NO: 47, SEQ ID NO: 49 and SEQ ID NO: 51.
17. The TCR according to any one of the preceding items, wherein the TCR comprises a paired TCR alpha- and TCR beta-chain comprising a complete assembly of combinations of the following sequences:

| | SEQ ID NO | SEQ ID NO | SEQ ID NO | SEQ ID NO |
|---|---|---|---|---|
| SC1alpha | 11 | 13 | 11 | 13 |
| CDR1alpha | 32 | 34 | 32 | 34 |
| SC2alpha | 18 | 20 | 18 | 20 |
| CDR2alpha | 39 | 41 | 39 | 41 |
| SC3alpha | 25 | 27 | 25 | 27 |
| CDR3alpha | 46 | 48 | 50 | 48 |
| SC4alpha | 5 | 5 | 8 | 5 |
| SC5alpha | 54 | 54 | 54 | 54 |
| SC1beta | 12 | 14 | 15 | 14 |
| CDR1beta | 33 | 35 | 36 | 35 |
| SC2beta | 19 | 21 | 22 | 21 |
| CDR2beta | 40 | 42 | 43 | 42 |
| SC3beta | 26 | 28 | 29 | 28 |
| CDR3beta | 47 | 49 | 51 | 49 |
| SC4beta | 6 | 7 | 9 | 7 |
| SC5beta | 55 | 56 | 55 | 82 |

or sequences having at least 90% such as at least 95%, at least 97% or at least 99%, or 100% identity to said respective sequences.
18. The TCR according to any of the preceding items, wherein the TCR comprises a paired TCR alpha- and TCR beta-chain comprising the following combination of sequences:

| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 11 | SEQ ID NO: 32 | SEQ ID NO: 18 | SEQ ID NO: 39 | SEQ ID NO: 25 | SEQ ID NO: 46 | SEQ ID NO: 5 | SEQ ID NO: 54 |
| TCR-2 alpha | SEQ ID NO: 13 | SEQ ID NO: 34 | SEQ ID NO: 20 | SEQ ID NO: 41 | SEQ ID NO: 27 | SEQ ID NO: 48 | SEQ ID NO: 5 | SEQ ID NO: 54 |
| TCR-3 alpha | SEQ ID NO: 11 | SEQ ID NO: 32 | SEQ ID NO: 18 | SEQ ID NO: 39 | SEQ ID NO: 25 | SEQ ID NO: 50 | SEQ ID NO: 8 | SEQ ID NO: 54 |
| TCR-1 beta | SEQ ID NO: 12 | SEQ ID NO: 33 | SEQ ID NO: 19 | SEQ ID NO: 40 | SEQ ID NO: 26 | SEQ ID NO: 47 | SEQ ID NO: 6 | SEQ ID NO: 55 |
| TCR-2 beta | SEQ ID NO: 14 | SEQ ID NO: 35 | SEQ ID NO: 21 | SEQ ID NO: 42 | SEQ ID NO: 28 | SEQ ID NO: 49 | SEQ ID NO: 7 | SEQ ID NO: 56 |
| TCR-2 beta | SEQ ID NO: 14 | SEQ ID NO: 35 | SEQ ID NO: 21 | SEQ ID NO: 42 | SEQ ID NO: 18 | SEQ ID NO: 49 | SEQ ID NO: 7 | SE ID NO: 82 |
| TCR-3 beta | SEQ ID | SEQ ID | SEQ ID | SEQ ID | SEQ ID | SEQ ID | SEQ ID NO: 9 | SEQ ID NO: 55 |

| | | | | | | | | |
|---|---|---|---|---|---|---|---|---|
| NO: 15 | NO: 36 | NO: 22 | NO: 43 | NO: 29 | NO: 51 | | | |

or sequences having at least 90% such as at least 95%, at least 97% or at least 99%, or 100% identity to said respective sequences.
19. The TCR according to any of the preceding items, wherein the alpha and beta chain CDRs have the following sequences:

| CDR1 | CDR2 | CDR3 | CDR1 | CDR2 | CDR3 |
|---|---|---|---|---|---|
| alpha | alpha | alpha | beta | beta | beta |
| SEQ ID NO: 32 | SEQ ID NO: 39 | SEQ ID NO: 46 | SEQ ID NO: 33 | SEQ ID NO: 40 | SEQ ID NO: 47 |
| SEQ ID NO: 34 | SEQ ID NO: 41 | SEQ ID NO: 48 | SEQ ID NO: 35 | SEQ ID NO: 42 | SEQ ID NO: 49 |
| SEQ ID NO: 32 | SEQ ID NO: 39 | SEQ ID NO: 50 | SEQ ID NO: 36 | SEQ ID NO: 43 | SEQ ID NO: 51 |

20. The TCR according to any of the preceding items, wherein the alpha and beta chain CDR3s have the following sequences:

| CDR3 alpha | CDR3 beta |
|---|---|
| SEQ ID NO: 46 | SEQ ID NO: 47 |
| SEQ ID NO: 48 | SEQ ID NO: 49 |
| SEQ ID NO: 50 | SEQ ID NO: 51 |

21. The TCR according to any of the preceding items, wherein the TCR alpha chain variable, domain scaffold regions, and/or or CDRs comprise and/or consist of one of the sequences selected from the group consisting of SEQ ID NO: 57, SEQ ID NO: 59 and SEQ ID NO: 61, and wherein the beta chain variable, domain scaffold regions, and/or or CDRs comprise and/or consist of one of the sequences selected from the group consisting of SEQ ID NO: 58, SEQ ID NO: 60, SEQ ID NO: 62 and SEQ ID NO: 83.
22. The TCR according to any of the preceding items, wherein the TCR comprises an alpha chain paired with a beta chain, wherein such pairs are selected from the group of alpha:beta chain pairs consisting of SEQ ID NO: 57 and SEQ ID NO 58; SEQ ID NO: 59 and SEQ ID NO 60; SEQ ID NO: 61 and SEQ ID NO: 62; SEQ ID NO: 61 and SEQ ID NO: 83.
23. The TCR according to any of the preceding items, wherein the TCR is selected from the group comprising chimeric, humanized and/or human TCRs.
24. The TCR according to any of the preceding items wherein the constant regions of the alpha and beta chains have been modified to include cysteine residues to allow covalent connection by di-sulphide bridges.
25. The TCR according to any of items 1-23, wherein there are no covalent connections between said paired alpha and beta chains.
26. The TCR according to any of items 1-25, wherein the alpha and/or beta chain comprise(s) a constant region of another mammal than human.
27. The TCR according to any one of items 23 to 26, wherein the alpha and/or beta chain comprise(s) a mouse chimeric constant region(s).
28. The TCR according to any of items 1-24 and 26-27, wherein the alpha and/or beta chain have been modified to include cysteine residues to allow di-sulphide bridges.
29. The TCR according to any of items 1-24 and 26-28, wherein the TCR alpha chain and the TCR beta chain are covalently linked.
30. The TCR according to any of items 1-24 and 26-29, wherein the TCR alpha chain and the TCR beta chain are covalently linked through a linker peptide.
31. The TCR according to any of the preceding items wherein the TCR alpha chain and/or the TCR beta chain are covalently linked to a moiety.
32. The TCR according to item 31, wherein the covalently linked moiety comprises an affinity tag or a label.
33. The TCR according to item 32, wherein the tag is selected from the group consisting of a truncated CD34 enrichment tag, glutathione-S-transferase (GST), calmodulin binding protein (CBP), protein C tag, Myc tag, Halo tag, HA tag, Flag tag, His tag, biotin tag, and V5 tag.
34. The TCR according to item 32, wherein the label is a fluorochrome or a fluorophore such as a fluorescent protein.
35. The TCR according to item 31, wherein the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.
36. A soluble TCR and/or a conjugated soluble TCR, wherein the TCR is a TCR as defined in any of items 1-35.
37. The TCR according to item 36, wherein the TCR is a bi-specific T-cell engager (BiTE), or is an immune mobilizing monoclonal T-cell receptor.
38. A pharmaceutical composition comprising a soluble protein consisting of all or a functional part of a TCR as defined in any of items 1-37 and at least one pharmaceutically acceptable excipient, carrier or stabilizer.
39. A pharmaceutical composition comprising a soluble TCR, a conjugated soluble TCR, a cell expressing a TCR or a composition comprising a TCR, wherein the TCR is a TCR as defined in any of items 1-38.
40. A pharmaceutical composition comprising a soluble protein consisting of all or a functional part of a TCR as defined in any of items 1-39, wherein said soluble protein has been conjugated to a radionuclide, a chemotherapeutic agent, a toxin or another therapeutically active component, and at least one pharmaceutically acceptable excipient, carrier or stabilizer.
41. A method of treating a disorder characterized by MAGE-A4 expression in a subject comprising administering to said subject a therapeutically effective amount of genetically engineered cells that express a TCR as defined in any of items 1-37, or a pharmaceutical composition as defined in any one of items 38-40, wherein said disorder is a solid malignancy expressing the peptide of GVYDGREHTV (SEQ ID NO: 1), wherein said engineered cell is a T-cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour or into the volume surrounding a solid tumour.
42. The method of treating a disorder in a subject according to item 41 wherein said subject carries a HLA-A*02 allele.
43. The method of treating a disorder in a subject according to any of items 41 or 42, wherein said subject carries an alternate subform of HLA-A*02 other than HLA-A*02:03, which alternate subform of HLA-A*02 is capable of specifically presenting the peptide of SEQ ID NO: 1 and which is recognized by the TCR according to any of items 1-30.
44. The method of treating a disorder in a subject according to any of items 41-43, wherein said subject carries an alternate subform of HLA-A*02 other than HLA-A*02:03, HLA-A*02:04, HLA-A*02:05, or HLA-A*02:06, which alternate subform of HLA-A*02 is capable of specifically presenting the peptide of SEQ ID NO: 1 and which is recognized by the TCR according to any of items 1-37
45. The method of treating a disorder in a subject according to any of items 41-43, wherein said subject carries a subform of HLA-A*02 which subform of HLA-A*02 is capable of specifically presenting the peptide of SEQ ID NO: 1 and which is recognized by the TCR according to any of items 1-37.
46. The method of treating a disorder in a subject according to any of items 41-43, wherein said subject carries a different HLA-A, HLA-B or HLA-C allele than HLA-A*02, that is capable of specifically presenting the peptide of SEQ ID NO: 1 and which is recognized by the TCR according to any of items 1-37.
47. The method of treating a disorder in a subject according to any of items 41-43, wherein said subject carries HLA-B*57:01, that is capable of specifically presenting the peptide of SEQ ID NO: 1 and which is recognized by the TCR according to any of items 1-37.
48. The method of treating a disorder in a subject according to any of items 41-43, wherein said subject carries HLA-A*68:02 or HLA-B*15:01, that is capable of specifically presenting the peptide of SEQ ID NO: 1 and which is recognized by the TCR according to any of items 1-37.
49. The TCR according to any of items 1-37 for use as a medicament.
50. The TCR according to any of items 1-37 for use in the treatment of a cancer in a patient carrying a HLA-A*02 subform selected from the group comprising HLA-A*02:01; HLA-A*02:02; HLA-A*02:03; HLA-A*02:04; HLA-A*02:05; HLA-A*02:06; HLA-A*02:07, comprising administering to said patient said TCR or a pharmaceutical composition comprising said TCR, wherein said cancer is a solid malignancy expressing the peptide of SEQ ID NO: 1 and wherein said TCR or a pharmaceutical composition comprising said TCR is infused systemically into said patient or injected or pumped directly into a solid tumour, into a blood vessel that feeds a tumour or into the volume surrounding a solid tumour in said patient.
51. The TCR for use according to item 49, for use in the treatment of a cancer in a patient carrying HLA-A*02:01, comprising administering to said patient an engineered cell, wherein said cancer is a solid malignancy expressing the peptide of SEQ ID NO: 1, wherein said engineered cell is a T cell, T cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour or into the volume surrounding a solid tumour.
52. The TCR for use according to item 49-51 for use in treating a disorder in a subject, wherein said subject carries an a HLA-A*02 subform selected from the group comprising HLA-A*02:01; HLA-A*02:02; HLA-A*02:03; HLA-A*02:04; HLA-A*02:05; HLA-A*02:06; HLA-A*02:07 is capable of specifically presenting the peptide of SEQ ID NO: 1 and which is recognized by the TCR according to any of items 1-37.
53. The TCR for use according to item 49-51, for use in treating a disorder in a subject, wherein said subject carries a different HLA-A, HLA-B or HLA-C allele than HLA-A*02, that is capable of specifically presenting the peptide of SEQ ID NO: 1 and which is recognized by the TCR according to any of items 1-37.
54. The TCR for use according to item 49-51, for use in treating a disorder in a subject, wherein said subject does not carry a different HLA-A, HLA-B or HLA-C allele than HLA-A*02, that is capable of specifically presenting the peptide of SEQ ID NO: 1 and which is recognized by the TCR according to any of items 1-37.
55. A nucleic acid encoding a TCR comprising a paired TCR alpha- and TCR beta-chain as defined in any one of items 1-37.
56. A vector comprising a nucleic acid encoding a TCR comprising a paired TCR alpha- and TCR beta-chain as defined in any one of the items 1-37.
57. A vector according to item 56 which is a vector selected from the group consisting of an expression vector, a coding vector, non-viral or viral vector.
58. The vector according to item 56 or 57 wherein the vector further comprises a nucleic acid sequence encoding CD8α and/or CD8β.
59. The vector according to item 58 wherein the nucleic acid sequence encoding CD8α or CD8β is operably linked to a nucleic acid encoding a tag.
60. The vector according to item 59 wherein the nucleic acid encoding a tag is at the 5' upstream of the nucleic acid sequence encoding CD8α or CD8β such that the tag is fused to the N-terminus of CD8α or CD8β when expressed.
61. The vector according to item 59 or 60 wherein the tag is a truncated CD34 enrichment tag.
62. The vector according to any of items 56-61, wherein the isolated nucleic acid of item 55 and the nucleic acid sequence encoding CD8α and/or CD8β according to any of items 58-60 are interconnected with an internal ribosome entry site or a nucleic acid sequence encoding a self-cleaving peptide.
63. The vector according to item 62, wherein the self-cleaving peptide is selected from the group consisting of P2A, E2A, F2A and T2A.
64. The vector according to any of items 56-63, wherein the vector is a nucleic acid vector selected from the group comprising an expression vector, a cloning vector, phagemid, cosmid, transposon, episome, artificial chromosome or RNA, a nanoparticle, or a viral vector.
65. The vector according to item 64 wherein the vector is a viral vector and is selected from the group comprising adenoviruses, adeno-associated viruses, retroviruses, lentiviruses, and herpes simplex viruses.
66. The viral vector according to item 65 wherein the viral vector is pseudotyped,
67. The vector according to item 64, wherein the vector is a nanoparticle vector selected from the group comprising lipid nanoparticles (LNPs), targeted LNP (tLNP), nanoparticles (NPs), cationic liposome complexes, cationic polymer complexes, cationic liposome-polymer complexes, exosomes, liposomes, and inorganic nanoparticles,
68. The vector according to any one of items 56-67 wherein the nucleic acid vector, the viral vector, or the nanoparticle vector comprises one or more cell surface receptors that bind to a ligand on a target cell, heterologous viral envelope glycoproteins, fusion glycoproteins, T-cell activation or co-stimulation molecules, cytokines or cytokine-based transduction enhancers, and/or transmembrane proteins comprising a mitogenic domain and/or cytokine-based domain exposed on the surface and/or conjugated to the surface of the viral vector.
69. The vector according to any of items 56-68, wherein the vector is pseudotyped with a Cocal or a Nipah virus envelope proteins.
70. The vector according item 69, wherein the Nipah envelope protein is engineered to bind EpCAM, CD4, CD8, CD3 or CD5 or other marker suitable for targeted cellular delivery of a TCR-encoding nucleic acid vector.
71. The vector according to any of items 56-68, wherein the vector comprises a lipid nanoparticle comprising ionizable cationic lipid, a sterol, a phospholipid, a non- functionalized PEG-lipid, a functionalized PED-lipid wherein the functionalized PEG-lipid has been conjugated with a binding moiety.
72. The vector according to any of items 56-68, wherein the vector comprises a lipid nanoparticle (LNP) or tLNP comprising a PEG lipid and further comprising one or more of a phospholipid, an ionizable cationic lipid, a sterol, a co-lipid and a further PEG-lipid or combination thereof and wherein LNP or tLNP comprises mRNA. The LNP or tLNP can be engineered to bind EpCAM, CD4, CD8, CD3 or CD5 or other marker suitable for targeted cellular delivery of a TCR-encoding nucleic acid vector
73. An engineered cell expressing the TCR as defined in any of items 1-37.
74. An engineered cell which comprises and/or expresses the isolated nucleic acid of item 55, or a vector according to any of items 56-72.
75. The engineered cell according to item 73 or 74, wherein the engineered cell comprises a chromosomal gene knock out of one or more TCR gene(s), one or more HLA gene(s), or both.
76. The engineered cell according to any of items 73-75, wherein the engineered cell comprises a knockout of an HLA gene selected from the group consisting of an alpha-1-macroglobulin gene, alpha-2-macroglobulin gene, alpha-3-macroglobulin gene, beta-1-microglobulin gene, beta-2-microglobulin gene, and combinations thereof.
77. The engineered cell according to any of items 73-76, wherein the engineered cell comprises a knockout of a TCR gene selected from a TCR alpha variable region gene, TCR beta variable region gene, TCR constant region gene, and combination thereof.
78. The engineered cell according to any of items 73-77, wherein the engineered cell expresses CD8a and/or CD8b, optionally the CD8 alpha and/or CD8 beta is fused to a truncated CD34 enrichment tag.
79. The engineered cell according to any of items 73-78, wherein the engineered cells are enriched using the truncated CD34 enrichment tag.
80. The engineered cell according to any of items 73-79, wherein the engineered cell is a hematopoietic progenitor cell, peripheral blood mononuclear cell (PBMC), cord blood cell, or immune cell.
81. The engineered cell according to item 80, wherein engineered cell is an immune cell and is selected from the group comprising a T cell, cytotoxic lymphocyte, cytotoxic lymphocyte precursor cell, cytotoxic lymphocyte progenitor cell, cytotoxic lymphocyte stem cell, CD4⁺ T cell, CD8⁺ T cell, CD4/CD8 double negative T cell, gamma delta (gd) T cell, natural killer (NK) cell, NK-T cell, dendritic cell, or a combination thereof.
82. The engineered cell according to item 80 or 81 wherein the cell is derived from a hematopoietic stem cell (HSC) or an induced pluripotent stem cell (iPSC).
83. The engineered cell according to item 81, wherein the cell is a T cell and is a T cell, central memory T cell, effector memory T cell, or a combination thereof.
84. The engineered cell according to any of items 81-83, wherein the T cell is a primary T cell or a cell of a T cell line.
85. The engineered cell according to any of items 81-84 wherein the T cell does not express or has a lower surface expression of an endogenous TCR.
86. The engineered cell according to any of items 81-85, wherein the engineered cell is capable of producing a cytokine or a cytotoxic molecule when contacted with a target cell that presents a MAGE-A4 peptide, wherein the peptide is a melanoma-associated antigen A4 (MAGE-A4) peptide, wherein the peptide consists of GVYDGREHTV (SEQ ID NO: 1) presented in a HLA-A*02:01 complex.
87. The engineered cell according to item 86, wherein the engineered cell is contacted with the target cell *in vitro* or ex *vivo.*
88. The engineered cell according to item 86 or 87, wherein the cytokine is TNF-alpha, IL-2, and/or IFN-gamma.
89. The engineered cell according to any of items 86 or 87, wherein the cytotoxic molecule is a perforin and/or a granzyme, such as granzyme B.
90. The engineered cell according to any of items 86-89, wherein the engineered cell is capable of producing a higher level of cytokine or a cytotoxic molecule when contacted with a target cell with a heterozygous expression of MAGE-A4.
91. The engineered cell according to item 90, wherein the engineered cell is capable of producing an at least 1.05-fold higher level of cytokine or a cytotoxic molecule.
92. The engineered cell according to any of items 86-89, wherein the engineered cell is capable of killing a target cell that presents a melanoma-associated antigen A4 (MAGE-A4) peptide, wherein the peptide consists of GVYDGREHTV (SEQ ID NO: 1) presented in a HLA-A*02:01 complex.
93. The engineered cell according to item 92, wherein the killing is determined by a killing assay.
94. The engineered cell according to item 92 or 93, wherein the ratio of the engineered cell and the target cell in the killing assay is from 1:16 to 1:1.
95. The engineered cell according to any of items 86-94, wherein the target cell is a target cell pulsed with a range of between 1x10³ nM and 0.1 nM of melanoma-associated antigen A4 (MAGE-A4) peptide, wherein the peptide consists of GVYDGREHTV (SEQ ID NO: 1) presented in a HLA-A*02:01 complex.
96. The engineered cell according to item 95, wherein the target cell is a cell monoallelic for HLA-A*02:01.
97. The engineered cell according to any of items 86-96, wherein the engineered cell is capable of killing a higher number of target cells when contacted with target cells with a heterozygous expression of a melanoma-associated antigen A4 (MAGE-A4) peptide, wherein the peptide consists of GVYDGREHTV (SEQ ID NO: 1) presented in a HLA-A*02:01 complex.
98. The engineered cell according to item 97, wherein the cell killing is at least 1.05-fold higher.
99. The engineered cell according to any of items 86-98, wherein the target cell is a cell line or a primary cell.
100. The engineered cell according to item 99, wherein the target cell is selected from the group consisting of a cancer cell line, a primary cancer cell line, a transformed cell line, and an immortalized cell line.
101. The engineered cell according to item 100, wherein the target cell is a HEK293-, A-375-, U266-, ARH -77-, NCI-H1755-, NCI-H1703-, or SAOS-2- derived cell line.
102.The engineered cell according to any of items 86-101, wherein the engineered cell does not induce T cell expansion, cytokine release, or cytotoxic killing when in contact with a target cell that does not present MAGE-A4 peptide in a HLA-A*02:01 complex.
103.A population of engineered cells according to any one of items 73-102.
104.A pharmaceutical composition comprising any one or more of the engineered cells according to any one of items 73-102 or vectors according to any of items 56-72.
105.The pharmaceutical composition according to item 104, wherein the vectors are selected from the group consisting of viral or non-viral vectors, LNPs or any other carrier comprising the nucleic acid sequence according to item 55.
106.A pharmaceutical composition comprising a soluble protein consisting of all or a functional part of one or more TCR(s) as defined in any of items 1-37, or one or more vector(s) according to any one of items 56-72, or one or more engineered cell(s) according to any one of items 73-102.
107.The nucleic acid according to item 55 or the vector according to any one of items 56-72, wherein the nucleic acid encoding a TCR comprising a paired TCR alpha- and TCR beta-chain is linear or circular double stranded DNA
108.The nucleic acid according to item 46 or the vector according to any one of items 56-72, wherein the nucleic acid encoding a TCR comprising a paired TCR alpha- and TCR beta-chain is linear or circular single stranded DNA.
109.The nucleic acid according to item 55 or 108, wherein the DNA is chemically modified.
110.The nucleic acid according to item 55 or 108-109 or the vector according to any one of items 56-72, wherein the nucleic acid encoding a TCR comprising a paired TCR alpha- and TCR beta-chain is linear or circular double stranded RNA
111.The nucleic acid according to item 55 or 108-109 or the vector according to any one of items 56-72, wherein the nucleic acid encoding a TCR comprising a paired TCR alpha- and TCR beta-chain is linear or circular single stranded RNA.
112.The nucleic acid according to item 110 or 111, wherein the RNA is chemically modified.
113.The vector according to any one of items 63-72, wherein the RNA contains a 5' CAP, a 5' UTR, a 3' UTR and 3' poly(A) tail, and an internal ribosome entry site (IRES).
114.The vector according to item 113 wherein the vector is circularized
115.The vector according to item 113 or 114 wherein the vector contains replication-directing elements for self-amplification.

## Claims

1. A T-cell receptor (TCR), which is isolated and/or expressed in an engineered cell, specifically recognizing a melanoma-associated antigen A4 (MAGE-A4) peptide, wherein the peptide consists of GVYDGREHTV (SEQ ID NO: 1) presented in a HLA-A*02 complex, and wherein the TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

2. The TCR according to claim 1, wherein the TCR, when expressed in an engineered cell, is capable of specifically recognising the peptide comprising SEQ ID NO: 1 when presented in a HLA-A*02 complex.

3. The TCR according to claim 1 to 4, wherein the TCR, when expressed in an engineered cell, does not cause specific cytotoxic activity towards cells not expressing a MAGE-A4 peptide.

4. The TCR according to any of the preceding claims wherein the TCR, when expressed in an engineered cell, does not specifically recognise other allelic variants of the human leukocyte antigen (HLA) molecule other than an allelic variant of the human leukocyte antigen (HLA) molecule selected from the group consisting of HLA-A*02:02, HLA-B*57:01, and HLA-B*15:01 serotype.

5. The TCR according to any of the preceding claims, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02; HLA-A*02:04; HLA-A*02:05; HLA-A*02:06; HLA-A*02:07, and HLA-B*57:01 serotype.

6. The TCR according to any of claims 1 to 4, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02 and HLA-A*02:07 serotype.

7. The TCR according to any of claims 1 to 4, wherein the TCR, when expressed in an engineered cell, does not specifically recognize cells expressing the MAGE-A4 peptide, wherein the peptide is GVYDGREHTV (SEQ ID NO: 1) presented in an allelic variant of the human leukocyte antigen (HLA) molecule other than an allelic variant of the human leukocyte antigen (HLA) molecule selected from the group consisting of HLA-A*02:01; HLA-A*02:02; HLA-A*02:03; HLA-A*02:04; HLA-A*02:05; HLA-A*02:06; HLA-A*02:07; HLA-A*68:02 and HLA-B*15:01 serotype.

8. The TCR according to any of the preceding claims, wherein the TCR, when expressed in an engineered cell, has at the most 4% cross-reactivity towards any somatically expressed HLA-presented peptide other than a MAGE-A4 peptide, which consists of GVYDGREHTV (SEQ ID NO: 1) and which is presented in a HLA-A*02:01 complex in a cross-reactivity assay.

9. The TCR according to any of the preceding claims comprising:
a. a TCR alpha chain CDR3 sequence with at least 75% identity to a TCR alpha chain CDR3 sequence selected from the group consisting of SEQ ID NO: 46, SEQ ID NO: 48 and SEQ ID NO: 50,
and
b. a TCR beta chain CDR3 sequence with at least 75% identity to a TCR beta chain CDR3 sequence selected from the group consisting of SEQ ID NO: 47, SEQ ID NO: 49 and SEQ ID NO: 51.

10. The TCR according to any of the preceding claims comprising:
a) a TCR alpha chain CDR3 sequence with a TCR alpha chain CDR3 sequence selected from the group consisting of SEQ ID NO: 46, SEQ ID NO: 48 and SEQ ID NO: 50
and
b) a TCR beta chain CDR3 sequence with a TCR beta chain CDR3 sequence selected from the group consisting of SEQ ID SEQ ID NO: 47, SEQ ID NO: 49 and SEQ ID NO: 51.

11. The TCR according to any one of the preceding claims, wherein the TCR comprises a paired TCR alpha- and TCR beta-chain comprising a complete assembly of combinations of the following sequences:
| | SEQ ID NO | SEQ ID NO | SEQ ID NO | SEQ ID NO |
|---|---|---|---|---|
| SC1alpha | 11 | 13 | 11 | 13 |
| CDR1alpha | 32 | 34 | 32 | 34 |
| SC2alpha | 18 | 20 | 18 | 20 |
| CDR2alpha | 39 | 41 | 39 | 41 |
| SC3alpha | 25 | 27 | 25 | 27 |
| CDR3alpha | 46 | 48 | 50 | 48 |
| SC4alpha | 5 | 5 | 8 | 5 |
| SC5alpha | 54 | 54 | 54 | 54 |
| SC1beta | 12 | 14 | 15 | 14 |
| CDR1beta | 33 | 35 | 36 | 35 |
| SC2beta | 19 | 21 | 22 | 21 |
| CDR2beta | 40 | 42 | 43 | 42 |
| SC3beta | 26 | 28 | 29 | 28 |
| CDR3beta | 47 | 49 | 51 | 49 |
| SC4beta | 6 | 7 | 9 | 7 |
| SC5beta | 55 | 56 | 55 | 82 |
or sequences having at least 90% such as at least 95%, at least 97% or at least 99%, or 100% identity to said respective sequences.

12. The TCR according to any of the preceding claims, wherein the TCR comprises a paired TCR alpha- and TCR beta-chain comprising the following combination of sequences:
| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 11 | SEQ ID NO: 32 | SEQ ID NO: 18 | SEQ ID NO: 39 | SEQ ID NO: 25 | SEQ ID NO: 46 | SEQ ID NO: 5 | SEQ ID NO: 54 |
| TCR-2 alpha | SEQ ID NO: 13 | SEQ ID NO: 34 | SEQ ID NO: 20 | SEQ ID | SEQ ID | SEQ ID | SEQ ID | SEQ ID NO: 54 |
| | | | | | | | | |
|---|---|---|---|---|---|---|---|---|
| | | | | NO: 41 | NO: 27 | NO: 48 | NO: 5 | |
| TCR-3 alpha | SEQ ID NO: 11 | SEQ ID NO: 32 | SEQ ID NO: 18 | SEQ ID NO: 39 | SEQ ID NO: 25 | SEQ ID NO: 50 | SEQ ID NO: 8 | SEQ ID NO: 54 |
| TCR-1 beta | SEQ ID NO: 12 | SEQ ID NO: 33 | SEQ ID NO: 19 | SEQ ID NO: 40 | SEQ ID NO: 26 | SEQ ID NO: 47 | SEQ ID NO: 7 | SEQ ID NO: 56 |
| TCR-2 beta | SEQ ID NO: 14 | SEQ ID NO: 35 | SEQ ID NO: 21 | SEQ ID NO: 42 | SEQ ID NO: 28 | SEQ ID NO: 49 | SEQ ID NO: 7 | SEQ ID NO: 56 |
| TCR-2 beta | SEQ ID NO: 14 | SEQ ID NO: 35 | SEQ ID NO: 21 | SEQ ID NO: 42 | SEQ ID NO: 28 | SEQ ID NO: 49 | SEQ ID NO: 7 | SEQ ID NO: 82 |
| TCR-3 beta | SEQ ID NO: 15 | SEQ ID NO: 36 | SEQ ID NO: 22 | SEQ ID NO: 43 | SEQ ID NO: 29 | SEQ ID NO: 51 | SEQ ID NO: 9 | SEQ ID NO: 55 |
or sequences having at least 90% such as at least 95%, at least 97% or at least 99%, or 100% identity to said respective sequences.

13. The TCR according to any of the preceding claims, wherein the alpha and beta chain CDRs have the following sequences:
| CDR1 | CDR2 | CDR3 | CDR1 | CDR2 | CDR3 |
|---|---|---|---|---|---|
| alpha | alpha | alpha | beta | beta | beta |
| SEQ ID NO: 32 | SEQ ID NO: 39 | SEQ ID NO: 46 | SEQ ID NO: 33 | SEQ ID NO: 40 | SEQ ID NO: 47 |
| SEQ ID NO: 34 | SEQ ID NO: 41 | SEQ ID NO: 48 | SEQ ID NO: 35 | SEQ ID NO: 42 | SEQ ID NO: 49 |
| | | | | | |
|---|---|---|---|---|---|
| SEQ ID NO: 32 | SEQ ID NO: 39 | SEQ ID NO: 50 | SEQ ID NO: 36 | SEQ ID NO: 43 | SEQ ID NO: 51 |

14. The TCR according to any of the preceding claims, wherein the alpha and beta chain CDR3s have the following sequences:
| CDR3 alpha | CDR3 beta |
|---|---|
| SEQ ID NO: 46 | SEQ ID NO: 47 |
| SEQ ID NO: 48 | SEQ ID NO: 49 |
| SEQ ID NO: 50 | SEQ ID NO: 51 |

15. The TCR according to any of the preceding claims, wherein the TCR alpha chain variable, domain scaffold regions, and/or or CDRs comprise and/or consist of one of the sequences selected from the group consisting of SEQ ID NO: 57, SEQ ID NO: 59 and SEQ ID NO: 61, and wherein the beta chain variable, domain scaffold regions, and/or or CDRs comprise and/or consist of one of the sequences selected from the group consisting of SEQ ID NO: 58, SEQ ID NO: 60, SEQ ID NO: 62 and SEQ ID NO: 83.

16. The TCR according to any of the preceding claims, wherein the TCR comprises an alpha chain paired with a beta chain, wherein such pairs are selected from the group of alpha:beta chain pairs consisting of SEQ ID NO:57 and SEQ ID NO 58; SEQ ID NO: 59 and SEQ ID NO 60; SEQ ID NO: 61 and SEQ ID NO: 62; SEQ ID NO: 61 and SEQ ID NO: 83.

17. An engineered cell expressing a TCR as defined in any one of claims 1-16, wherein the engineered cell is selected from the group consisting of an immune cell derived from, a peripheral blood mononuclear cell (PBMC), a cord blood cell, a hematopoietic progenitor cell, an induced pluripotent stem cell, and a primary immune cell collected from any bodily tissue and is either autologous or allogenic.

18. A nucleic acid encoding a TCR comprising a paired TCR alpha- and TCR beta-chain as defined in any one of claims 1-16

19. A vector comprising a nucleic acid encoding a TCR comprising a paired TCR alpha- and TCR beta-chain as defined in any one of the claims 1-16.

20. A vector according to claim 19, wherein the vector is a vector selected from the group consisting of an expression vector, a coding vector, non-viral or a viral vector, wherein said vector has the ability to carry said nucleic acid sequence into a suitable host cell where synthesis of the encoded polypeptide takes place.

21. A pharmaceutical composition comprising a soluble protein consisting of all or a functional part of one or more TCR(s) as defined in any of claims 1-16, or one or more engineered cell(s) according to claim 17 or one or more vector(s) according to claims 19 or 20.

22. The TCR according to any of claims 1-16, an engineered cell according to claim 17, a vector according to claims 19 or 20 or a pharmaceutical composition according to claim 21 for use in the treatment of a cancer in a patient carrying HLA-A*02, comprising administering to said patient said TCR, engineered cell, vector and/or a pharmaceutical composition comprising said TCR, wherein said cancer is a solid malignancy expressing the peptide of SEQ ID NO: 1, and wherein said TCR, engineered cell, vector and/or a pharmaceutical composition comprising said TCR is infused systemically into said patient or injected or pumped directly into a solid tumour, into a blood vessel that feeds a tumour or into the volume surrounding a solid tumour in said patient.
